# EUROPEAN PATENT APPLICATION

(11) **EP 4 470 570 A1**
(43) Date of publication of application: **04.12.2024**
(21) Application number: 23746246.0
(22) Date of filing: 19.01.2023
(51) Int. Cl.: A61K 47/68, C07K 16/28, A61K 31/4745, A61K 47/65, A61K 35/00

(54) **HER3 ANTIBODY-DRUG CONJUGATE AND USE THEREOF**

(30) Priority: 28.01.2022 CN 202210107800; 05.01.2023 CN 202310015113
(71) Applicant: Duality Biologics (Suzhou) Co., Ltd., Suzhou, Jiangsu 215000 (CN)
(72) Inventor: ZHU, Zhongyuan, Shanghai 201204 (CN); ZHANG, Yu, Shanghai 201204 (CN); LI, Xi, Shanghai 201204 (CN)
(74) Representative: Bianchetti & Minoja with Trevisan & Cuonzo IPS SRL
(86) International application number: PCT/CN2023/073130
(87) International publication number: WO 2023/143365

(57) **Abstract**

The present invention provides an anti-Her3 antibody-drug conjugate specifically binding to Her3 and a composition comprising the same. A method for using the antibody-drug conjugate of the present invention and use thereof are also provided.

## Description

### TECHNICAL FIELD

The present invention provides an antibody-drug conjugate specifically binding to Her3 and a composition comprising the same. A method for using the antibody-drug conjugate of the present invention and use thereof are also provided.

### BACKGROUND

Epidermal growth factor receptor (EGFR) is a large transmembrane glycoprotein with a molecular weight of about 170 kDa, and is a member of the ErbB receptor family. The EGFR receptor itself is a tyrosine kinase, which can form a dimer after binding to a ligand such as EGF and TNF-a, and activate a downstream signal (pathways such as MAPK, PI3K, and Stat) through transmission of phosphorylation, so that cell growth is maintained, and cell division and proliferation are promoted. As ErbB family receptors are conservative, EGFR can also form heterodimers with other proteins of the family (such as Her2, Her3, and Her4), thereby regulating cell growth more widely.

Her3 is a member of the ErbB family, and plays a key role in cell proliferation, tumor metastasis, and drug resistance. Although drugs targeting EGFR and Her2 show great clinical benefits in the alleviation of a variety of cancers, previous efforts to develop anti-Her3 antibodies for cancer therapy have repeatedly failed, suggesting that treatment of only Her3 and its pending approaches may not be sufficient to inhibit tumor growth.

An antibody-drug conjugate (ADC) consists of three portions, an antibody or an antigen-binding fragment thereof (targeting), a linker, and a small molecule drug. The antibody or the antigen-binding fragment thereof is conjugated with a bioactive small molecule drug such as cytotoxins with cytotoxicity, through a cleavable or non-cleavable linker. This effectively utilizes the specificity of the antibody or the antigen-binding fragment thereof for targeting cells of interest (target cells) or the specificity for binding to an antigen that is highly expressed as well as the high efficiency of small molecule drugs, and reduces or avoids the toxic side effects on non-targeted cells. This means that the antibody-drug conjugate for a tumor can precisely target a tumor cell and reduce the effect on non-tumor cells compared to conventional tumor chemotherapy drugs.

There remains a need in the art for an anti-Her3 antibody-drug conjugate that is superior in affinity, specificity, etc.

### SUMMARY

In one aspect, the present application provides an anti-Her3 antibody-drug conjugate, an isomer thereof, a pharmaceutically acceptable salt thereof, or a mixture thereof, wherein the anti-Her3 antibody-drug conjugate has a structure of formula (I):

Ab-(L-M-D)ₚ (I)

wherein,
L and M are linker units;
D is a cytotoxic drug;
p represents an average connection number, and p is selected from integers or decimals from 1 to 10, preferably integers or decimals from 3 to 8;
Ab is an anti-Her3 antibody or an antigen-binding fragment thereof, which comprises a heavy chain variable region and a light chain variable region, wherein the heavy chain variable region comprises HCDR1, HCDR2, and HCDR3 of the amino acid sequences set forth in SEQ ID NO: 1, SEQ ID NO: 2, and SEQ ID NO: 3, respectively, and the light chain variable region comprises LCDR1, LCDR2, and LCDR3 of the amino acid sequences set forth in SEQ ID NO: 4, SEQ ID NO: 5, and SEQ ID NO: 6, respectively.

In some embodiments, described herein is an anti-Her3 antibody-drug conjugate, the isomer thereof, the pharmaceutically acceptable salt thereof, or the mixture thereof, wherein the anti-Her3 antibody-drug conjugate has a structure of formula (I-1): 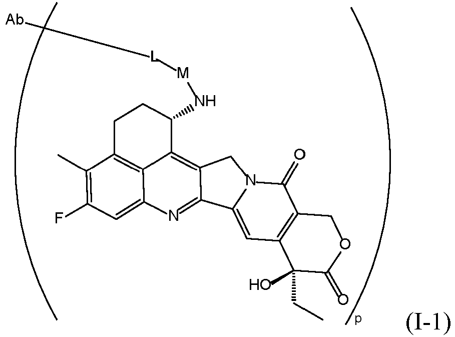
wherein,
L and M are linker units;
p represents an average connection number, and n is selected from integers or decimals from 1 to 10, preferably integers or decimals from 3 to 8;
Ab is an anti-Her3 antibody or an antigen-binding fragment thereof, which comprises a heavy chain variable region and a light chain variable region, wherein the heavy chain variable region comprises HCDR1, HCDR2, and HCDR3 of the amino acid sequences set forth in SEQ ID NO: 1, SEQ ID NO: 2, and SEQ ID NO: 3, respectively, and the light chain variable region comprises LCDR1, LCDR2, and LCDR3 of the amino acid sequences set forth in SEQ ID NO: 4, SEQ ID NO: 5, and SEQ ID NO: 6, respectively.

In some embodiments, the anti-Her3 antibody or the antigen-binding fragment thereof described herein comprises:
(I) a heavy chain variable region of the amino acid sequence set forth in SEQ ID NO: 7 or having at least 95%, 96%, 97%, 98%, or 99% identity thereto, and a light chain variable region of the amino acid sequence set forth in SEQ ID NO: 8 or having at least 95%, 96%, 97%, 98%, or 99% identity thereto; or
(II) a heavy chain variable region of the amino acid sequence set forth in SEQ ID NO: 9 or having at least 95%, 96%, 97%, 98%, or 99% identity thereto, and a light chain variable region of the amino acid sequence set forth in SEQ ID NO: 8 or having at least 95%, 96%, 97%, 98%, or 99% identity thereto; or
(III) a heavy chain variable region of the amino acid sequence set forth in SEQ ID NO: 10 or having at least 95%, 96%, 97%, 98%, or 99% identity thereto, and a light chain variable region of the amino acid sequence set forth in SEQ ID NO: 8 or having at least 95%, 96%, 97%, 98%, or 99% identity thereto.

In some embodiments, the anti-Her3 antibody or the antigen-binding fragment thereof described herein comprises:
(I) a heavy chain variable region of the amino acid sequence set forth in SEQ ID NO: 7 and a light chain variable region of the amino acid sequence set forth in SEQ ID NO: 8; or
(II) a heavy chain variable region of the amino acid sequence set forth in SEQ ID NO: 9 and a light chain variable region of the amino acid sequence set forth in SEQ ID NO: 8; or
(III) a heavy chain variable region of the amino acid sequence set forth in SEQ ID NO: 10 and a light chain variable region of the amino acid sequence set forth in SEQ ID NO: 8.

In some embodiments, the anti-Her3 antibody or the antigen-binding fragment described herein is a murine antibody or a fragment thereof, a chimeric antibody or an antigen-binding fragment, a humanized antibody or an antigen-binding fragment, or a fully human antibody or an antigen-binding fragment.

In some embodiments, the anti-Her3 antibody or the antigen-binding fragment thereof described herein is a humanized antibody or a fragment thereof.

In some embodiments, the anti-Her3 antibody or the antigen-binding fragment thereof described herein is selected from a Fab, a Fab', a Fab'-SH, an Fv, an scFv, a F(ab')₂, an sdAb, a bispecific antibody, or a linear antibody.

In some embodiments, the anti-Her3 antibody described herein is a monoclonal antibody.

In some embodiments, the antibody described herein is of the type of IgG1, IgG2, IgG3, or IgG4. In some embodiments, the antibody described herein is of the type of IgG1.

In some embodiments, the anti-Her3 antibody or the antigen-binding fragment thereof described herein comprises:
(1) a heavy chain of the amino acid sequence set forth in SEQ ID NO: 11 or having at least 95%, 96%, 97%, 98%, or 99% identity thereto, and a light chain of the amino acid sequence set forth in SEQ ID NO: 12 or having at least 95%, 96%, 97%, 98%, or 99% identity thereto; or
(2) a heavy chain of the amino acid sequence set forth in SEQ ID NO: 13 or having at least 95%, 96%, 97%, 98%, or 99% identity thereto, and a light chain of the amino acid sequence set forth in SEQ ID NO: 12 or having at least 95%, 96%, 97%, 98%, or 99% identity thereto; or
(3) a heavy chain of the amino acid sequence set forth in SEQ ID NO: 14 or having at least 95%, 96%, 97%, 98%, or 99% identity thereto, and a light chain of the amino acid sequence set forth in SEQ ID NO: 12 or having at least 95%, 96%, 97%, 98%, or 99% identity thereto.

In some embodiments, the anti-Her3 antibody or the antigen-binding fragment thereof described herein comprises:
(1) a heavy chain of the amino acid sequence set forth in SEQ ID NO: 11 and a light chain of the amino acid sequence set forth in SEQ ID NO: 12; or
(2) a heavy chain of the amino acid sequence set forth in SEQ ID NO: 13 and a light chain of the amino acid sequence set forth in SEQ ID NO: 12; or
(3) a heavy chain of the amino acid sequence set forth in SEQ ID NO: 14 and a light chain of the amino acid sequence set forth in SEQ ID NO: 12.

In some embodiments, described herein is an anti-Her3 antibody-drug conjugate, the isomer thereof, the pharmaceutically acceptable salt thereof, or the mixture thereof, wherein,
M is -L²-L³-X-L¹-;
L² is -O- or -S-;
L³ is -(C(R^{1a})(R^{1b}))ₘ-, and m is selected from 0, 1, 2, or 3, wherein when L³ comprises a methylene unit, 0 or 1 methylene unit of L³ may be replaced by -C(O)- or -C(S)-;
L¹ is -(C(R^{2a})(R^{2b}))ₙ-, and n is selected from 1, 2, or 3, wherein when L¹ may comprise a methylene unit, 0 or 1 methylene unit of L¹ may be replaced by -C(O)- or -C(S)-;
X is selected from 3- 6 membered saturated carbocyclyl, 3- 6 membered saturated heterocyclyl, or a single bond, wherein the 3-6 membered saturated carbocyclyl and the 3- 6 membered saturated heterocyclyl are optionally substituted with 0, 1, 2, or 3 R^{3a};
wherein each R^{1a}, each R^{1b}, each R^{2a}, each R^{2b}, and each R^{3a} may independently be hydrogen, halogen, or a C₁₋₆ aliphatic group which may be optionally substituted with R,
wherein each R may independently be hydrogen or halogen.

In some embodiments, the 3- 6 membered saturated heterocyclyl described herein comprises 1-3 heteroatoms selected from N, O, and S.

In some embodiments, L³ and X in the present invention are not single bonds at the same time.

In some embodiments, L² in the present invention is -O-. In some embodiments, L² in the present invention is -S-.

In some embodiments, described herein is an anti-Her3 antibody-drug conjugate, the isomer thereof, the pharmaceutically acceptable salt thereof, or the mixture thereof, wherein the linker unit M is linked to the linker unit L at the L² end and to the cytotoxic drug D at the L¹ end.

In some embodiments, described herein is an anti-Her3 antibody-drug conjugate, the isomer thereof, the pharmaceutically acceptable salt thereof, or the mixture thereof, wherein L³ is selected from a single bond, -C(R^{1a})(R^{1b})-, or -C(R^{1a})(R^{1b})C(R^{1a})(R^{1b})-,
wherein each R^{1a} and each R^{1b} may independently be hydrogen, halogen, or a C₁₋₆ aliphatic group which may be optionally substituted with R,
wherein each R may independently be hydrogen or halogen.

In some embodiments, described herein is an anti-Her3 antibody-drug conjugate, the isomer thereof, the pharmaceutically acceptable salt thereof, or the mixture thereof, wherein L³ is selected from a single bond, -C(R^{1a})(R^{1b})-, or -C(R^{1a})(R^{1b})C(R^{1a})(R^{1b})-,
wherein each R^{1a} and each R^{1b} may independently be hydrogen, halogen, CH₃, or CH₂CH₃.

In some embodiments, described herein is an anti-Her3 antibody-drug conjugate, the isomer thereof, the pharmaceutically acceptable salt thereof, or the mixture thereof, wherein L³ is selected from a single bond, -CH₂-, -CH(CH₃)-, -C(CH₃)₂-, -CH₂CH₂-, -CH(CH₃)CH₂-, or -C(CH₃)₂CH₂-.

In some embodiments, described herein is an anti-Her3 antibody-drug conjugate, the isomer thereof, the pharmaceutically acceptable salt thereof, or the mixture thereof, wherein L³ is selected from

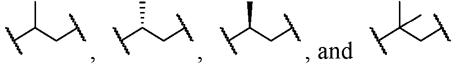

In some embodiments, described herein is an anti-Her3 antibody-drug conjugate, the isomer thereof, the pharmaceutically acceptable salt thereof, or the mixture thereof, wherein L³ is selected from a single bond.

In some embodiments, described herein is an anti-Her3 antibody-drug conjugate, the isomer thereof, the pharmaceutically acceptable salt thereof, or the mixture thereof, wherein L¹ is selected from - C(R^{2a})(R^{2b})-, -C(R^{2a})(R^{2b})C(O)-, or -C(O)-,
wherein each R^{2a} and each R^{2b} may independently be hydrogen, halogen, or a C₁₋₆ aliphatic group which may be optionally substituted with R,
wherein each R may independently be hydrogen or halogen.

In some embodiments, described herein is an anti-Her3 antibody-drug conjugate, the isomer thereof, the pharmaceutically acceptable salt thereof, or the mixture thereof, wherein L¹ is selected from - C(R^{2a})(R^{2b})-, -C(R^{2a})(R^{2b})C(O)-, or -C(O)-,
wherein each R^{2a} and each R^{2b} may independently be hydrogen, halogen, CH₃, or CH₂CH₃.

In some embodiments, described herein is an anti-Her3 antibody-drug conjugate, the isomer thereof, the pharmaceutically acceptable salt thereof, or the mixture thereof, wherein L¹ is selected from - CH₂-, -CH₂C(O)-, -CH(CH₃)C(O)-, or -C(O)-.

In some embodiments, described herein is an anti-Her3 antibody-drug conjugate, the isomer thereof, the pharmaceutically acceptable salt thereof, or the mixture thereof, wherein L¹ is selected from - C(O)-.

In some embodiments, described herein is an anti-Her3 antibody-drug conjugate, the isomer thereof, the pharmaceutically acceptable salt thereof, or the mixture thereof, wherein X is 3- 6 membered saturated carbocyclyl optionally substituted with 0, 1, 2, or 3 R^{3a} or a single bond,
wherein each R^{3a} may independently be hydrogen, halogen, or a C₁₋₆ aliphatic group which may be optionally substituted with R,
wherein each R may independently be hydrogen or halogen.

In some embodiments, described herein is an anti-Her3 antibody-drug conjugate, the isomer thereof, the pharmaceutically acceptable salt thereof, or the mixture thereof, wherein X is 3- 6 membered saturated carbocyclyl optionally substituted with 0, 1, 2, or 3 R^{3a} or a single bond,
wherein each R^{3a} may independently be hydrogen, halogen, CH₃, or CH₂CH₃.

In some embodiments, described herein is an anti-Her3 antibody-drug conjugate, the isomer thereof, the pharmaceutically acceptable salt thereof, or the mixture thereof, wherein X is cyclopropyl, cyclobutyl, cyclopentyl, cyclohexyl, or a single bond.

In some embodiments, described herein is an anti-Her3 antibody-drug conjugate, the isomer thereof, the pharmaceutically acceptable salt thereof, or the mixture thereof, wherein X is a single bond.

In some embodiments, described herein is an anti-Her3 antibody-drug conjugate, the isomer thereof, the pharmaceutically acceptable salt thereof, or the mixture thereof, wherein X is cyclopropyl, cyclobutyl, cyclopentyl, or cyclohexyl, preferably cyclobutyl.

In some embodiments, described herein is an anti-Her3 antibody-drug conjugate, the isomer thereof, the pharmaceutically acceptable salt thereof, or the mixture thereof, wherein X is 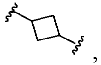 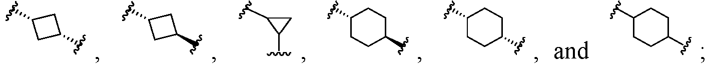 preferably, X is

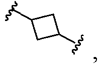

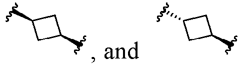

In some embodiments, described herein is an anti-Her3 antibody-drug conjugate, the isomer thereof, the pharmaceutically acceptable salt thereof, or the mixture thereof, wherein -M- is selected from: 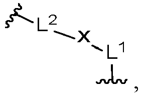 wherein
L² is -O- or -S-;
X is selected from 3- 6 membered saturated carbocyclyl optionally substituted with 0, 1, 2, or 3 R^{3a}; L¹ is selected from -C(R^{2a})(R^{2b})-, -C(R^{2a})(R^{2b})C(O)-, or -C(O)-,
wherein each R^{2a}, each R^{2b}, or each R^{3a} may independently be hydrogen, halogen, or a C₁₋₆ aliphatic group which may be optionally substituted with R,
wherein each R may independently be hydrogen or halogen.

In some embodiments, described herein is an anti-Her3 antibody-drug conjugate, the isomer thereof, the pharmaceutically acceptable salt thereof, or the mixture thereof, wherein -M- is selected from: 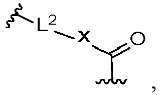 wherein
L² is -O- or -S-;
X is selected from 3- 6 membered saturated carbocyclyl optionally substituted with 0, 1, 2, or 3 R^{3a}, wherein each R^{3a} may independently be hydrogen, halogen, or a C₁₋₆ aliphatic group which may be optionally substituted with R,
wherein each R may independently be hydrogen or halogen.

In some embodiments, described herein is an anti-Her3 antibody-drug conjugate, the isomer thereof, the pharmaceutically acceptable salt thereof, or the mixture thereof, wherein -M- is selected from: 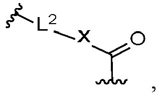 wherein
L² is -O- or -S-;
X is selected from cyclopropyl, cyclobutyl, cyclopentyl, or cyclohexyl.

In some embodiments, described herein is an anti-Her3 antibody-drug conjugate, the isomer thereof, the pharmaceutically acceptable salt thereof, or the mixture thereof, wherein -M- is selected from: 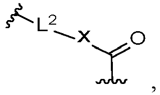 wherein
L² is -O- or -S-;
X is selected from

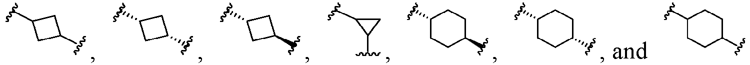

In some embodiments, described herein is an anti-Her3 antibody-drug conjugate, the isomer thereof, the pharmaceutically acceptable salt thereof, or the mixture thereof, wherein -M- is selected from: 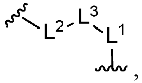 wherein
L² is -O- or -S-;
L³ is selected from -C(R^{1a})(R^{1b})- or -C(R^{1a})(R^{1b})C(R^{1a})(R^{1b})-;
L¹ is selected from -C(R^{2a})(R^{2b})-, -C(R^{2a})(R^{2b})C(O)-, or -C(O)-,
wherein each R^{1a}, each R^{1b}, each R^{2a}, or each ^{2b} may independently be hydrogen, halogen, or a C₁₋₆ aliphatic group which may be optionally substituted with R,
wherein each R may independently be hydrogen or halogen.

In some embodiments, described herein is an anti-Her3 antibody-drug conjugate, the isomer thereof, the pharmaceutically acceptable salt thereof, or the mixture thereof, wherein -M- is selected from: 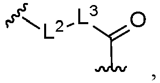 wherein
L² is -O- or -S-;
L³ is selected from -C(R^{1a})(R^{1b})- or -C(R^{1a})(R^{1b})C(R^{1a})(R^{1b})-,
wherein each R^{1a} and each R^{1b} may independently be hydrogen, halogen, or a C₁₋₆ aliphatic group which may be optionally substituted with R,
wherein each R may independently be hydrogen or halogen.

In some embodiments, described herein is an anti-Her3 antibody-drug conjugate, the isomer thereof, the pharmaceutically acceptable salt thereof, or the mixture thereof, wherein -M- is selected from: 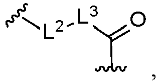 wherein
L² is -O- or -S-;
L³ is selected from -CH₂-, -CH(CH₃), -C(CH₃)₂, -CH₂CH₂-, -CH(CH₃)CH₂-, or -C(CH₃)₂CH₂-.

In some embodiments, described herein is an anti-Her3 antibody-drug conjugate, the isomer thereof, the pharmaceutically acceptable salt thereof, or the mixture thereof, wherein -M- is selected from: 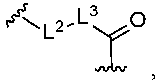 wherein
L² is -O- or -S-;
L³ is selected from

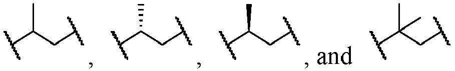

In some embodiments, described herein is an anti-Her3 antibody-drug conjugate, the isomer thereof, the pharmaceutically acceptable salt thereof, or the mixture thereof, wherein the linker unit M is linked to the linker unit L at the L² end.

In some embodiments, described herein is an anti-Her3 antibody-drug conjugate, the isomer thereof, the pharmaceutically acceptable salt thereof, or the mixture thereof, wherein -M- is selected from:

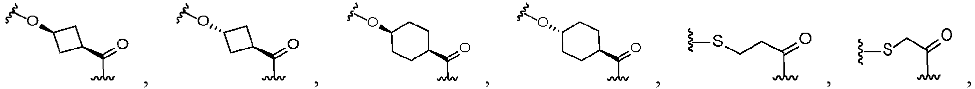

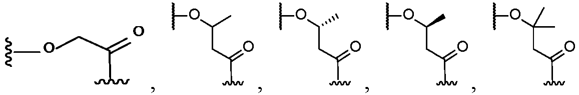

In some embodiments, described herein is an anti-Her3 antibody-drug conjugate, the isomer thereof, the pharmaceutically acceptable salt thereof, or the mixture thereof, wherein L is -Lₐ-L_{b}-L_{c}-, and -Lₐ- is 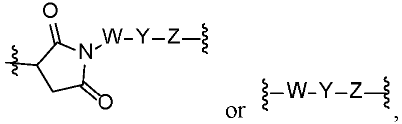
wherein W is -(C(R^{wa})(R^{wb}))_{wn}-, Y is -(OCH₂CH₂)_{yn}-O_{yp}-, and Z is -(C(R^{za})(R^{zb}))_{zn},
wherein wn is 1, 2, 3 or 6, and
1 methylene unit of W is each independently replaced by -Cyr-, -N(R^{wx})C(O)-, -C(O)N(R^{wx})- or - C(O)-;
wherein yn is 0, 4 or 8, and yp is 0 or 1;
wherein zn is 1, 2 or 3, and
1 methylene unit of Z is each independently replaced by -Cyr-, -N(R^{zx})C(O)-, -C(O)N(R^{zx})- or -C(O)-; -Cyr- is 3- 10 membered saturated carbocyclylene, wherein -Cyr- is unsubstituted or independently substituted with 1 to 3 substituent R^{cx};
wherein each R^{wa}, each R^{wb}, each R^{za}, each R^{zb}, each R^{wx}, each R^{zx} and each R^{cx} are independently hydrogen, halogen, -OR^{r}, or a C₁₋₆ aliphatic group optionally substituted with R^{r},
wherein each R^{r} is independently hydrogen, halogen or a C₁₋₆ aliphatic group;
-L_{b}- is a peptide residue consisting of 2 to 7 amino acids, and the peptide residue of -L_{b}- is a peptide residue formed of amino acids selected from the group consisting of: phenylalanine, glycine, alanine, valine, citrulline, lysine, serine, glutamic acid and aspartic acid;
-L_{c}- is 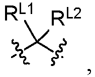
wherein R^{L1} and R^{L2} are each independently selected from the group consisting of: hydrogen, halogen, -OH and a C₁₋₆ aliphatic group.

In some embodiments, described herein is an anti-Her3 antibody-drug conjugate, the isomer thereof, the pharmaceutically acceptable salt thereof, or the mixture thereof, wherein L is -Lₐ-L_{b}-L_{c}-, and -Lₐ- is 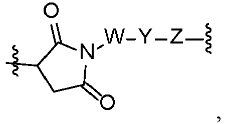
wherein W is -(C(R^{wa})(R^{wb}))_{wn}-, Y is -(OCH₂CH₂)_{yn}-O_{yp}-, and Z is -(C(R^{za})(R^{zb}))_{zn},
wherein wn is 1, 2, 3 or 6, and
1 methylene unit of W is each independently replaced by -Cyr-, -N(R^{wx})C(O)-, -C(O)N(R^{wx})- or - C(O)-;
wherein yn is 0, 4 or 8, and yp is 0 or 1;
wherein zn is 1, 2 or 3, and
1 methylene unit of Z is each independently replaced by -Cyr-, -N(R^{zx})C(O)-, -C(O)N(R^{zx})- or -C(O)-; -Cyr- is 3- 10 membered saturated carbocyclylene, wherein -Cyr- is unsubstituted or independently substituted with 1 to 3 substituent R^{cx};
wherein each R^{wa}, each R^{wb}, each R^{za}, each R^{zb}, each R^{wx}, each R^{zx} and each R^{cx} are independently hydrogen, halogen, -OR^{r}, or a C₁₋₆ aliphatic group optionally substituted with R^{r},
wherein each R^{r} is independently hydrogen, halogen or a C₁₋₆ aliphatic group;
-L_{b}- is selected from the group consisting of: 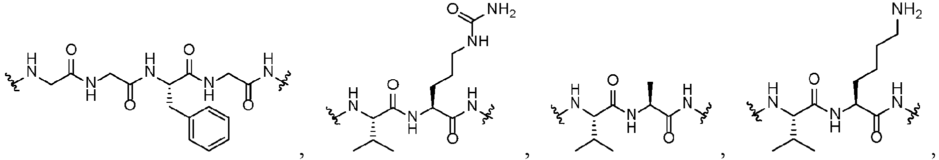 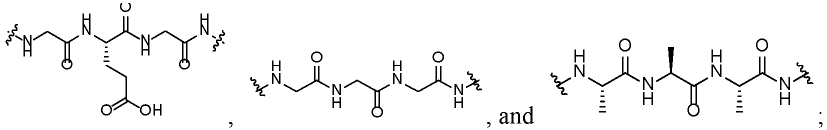
-L_{c}- is 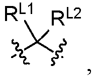
wherein R^{L1} and R^{L2} are each independently selected from the group consisting of: hydrogen, halogen, -OH and a C₁₋₆ aliphatic group.

In some embodiments, described herein is an anti-Her3 antibody-drug conjugate, the isomer thereof, the pharmaceutically acceptable salt thereof, or the mixture thereof, wherein -Lₐ- is 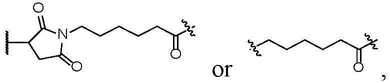 preferably

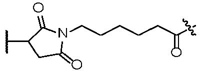

In some embodiments, described herein is an anti-Her3 antibody-drug conjugate, the isomer thereof, the pharmaceutically acceptable salt thereof, or the mixture thereof, wherein -Lₐ- is

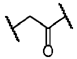

In some embodiments, described herein is an anti-Her3 antibody-drug conjugate, the isomer thereof, the pharmaceutically acceptable salt thereof, or the mixture thereof, wherein -L_{b}- is 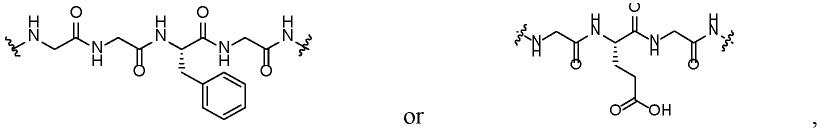 preferably

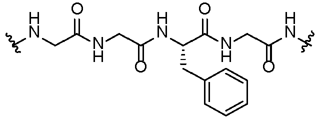

In some embodiments, described herein is an anti-Her3 antibody-drug conjugate, the isomer thereof, the pharmaceutically acceptable salt thereof, or the mixture thereof, wherein -L_{c}- is 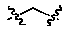 .

In some embodiments, described herein is an anti-Her3 antibody-drug conjugate, the isomer thereof, the pharmaceutically acceptable salt thereof, or the mixture thereof, wherein the linker unit L is linked to Ab at the Lₐ end and to the linker unit M at the L_{c} end .

In some embodiments, described herein is an anti-Her3 antibody-drug conjugate, the isomer thereof, the pharmaceutically acceptable salt thereof, or the mixture thereof, wherein L is

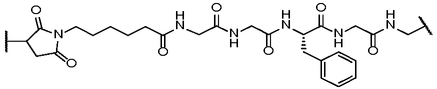

In some embodiments, described herein is an anti-Her3 antibody-drug conjugate, the isomer thereof, the pharmaceutically acceptable salt thereof, or the mixture thereof, wherein L is

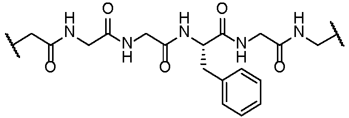

In some embodiments, described herein is an anti-Her3 antibody-drug conjugate, the isomer thereof, the pharmaceutically acceptable salt thereof, or the mixture thereof, wherein the anti-Her3 antibody-drug conjugate has a structure of formula (II-1) or (II-2): 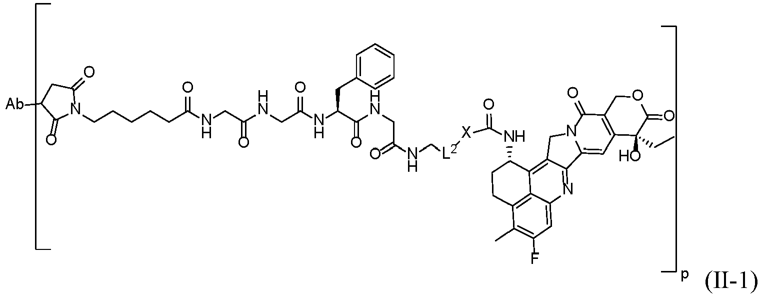 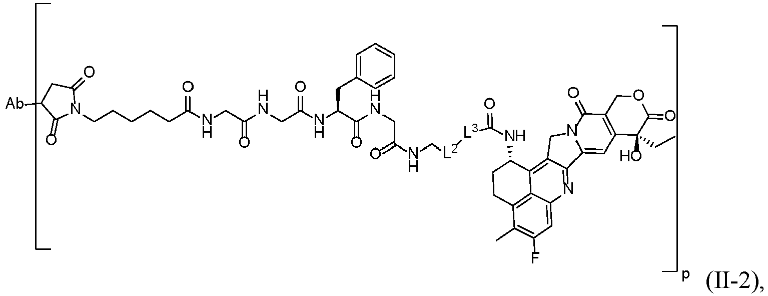 wherein,
Ab, L², X, p, and L³ are as defined in any one of the embodiments herein respectively.

In some embodiments, described herein is an anti-Her3 antibody-drug conjugate, the isomer thereof, the pharmaceutically acceptable salt thereof, or the mixture thereof, wherein the anti-Her3 antibody-drug conjugate has a structure of formula (II-1) or (II-2): 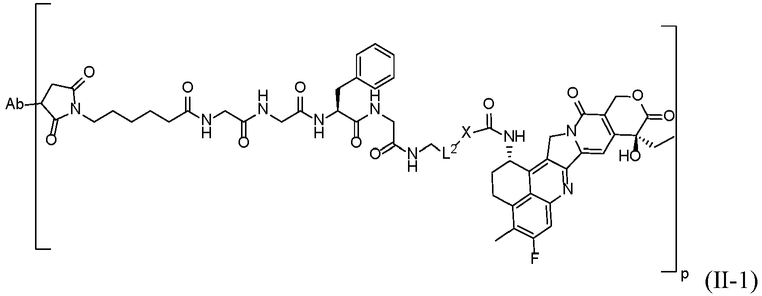 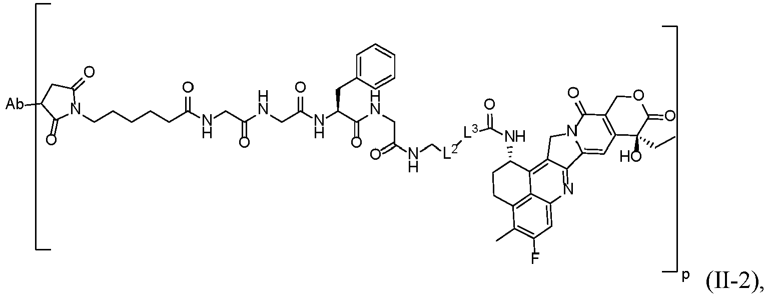 wherein,
p represents an average connection number, and p is selected from integers or decimals from 1 to 10, preferably integers or decimals from 3 to 8;
Ab is as defined in any one of the embodiments herein;
L² is -O- or -S-;
X is selected from 3- 6 membered saturated carbocyclyl optionally substituted with 0, 1, 2, or 3 R^{3a}; L³ is selected from -C(R^{1a})(R^{1b})- or -C(R^{1a})(R^{1b})C(R^{1a})(R^{1b})-,
wherein each R^{1a}, each R^{1b}, or each R^{3a} may independently be hydrogen, halogen, or a C₁₋₆ aliphatic group which may be optionally substituted with R,
wherein each R may independently be hydrogen or halogen.

In some embodiments, described herein is an anti-Her3 antibody-drug conjugate, the isomer thereof, the pharmaceutically acceptable salt thereof, or the mixture thereof, wherein the anti-Her3 antibody-drug conjugate has a structure of formula (II-1) or (II-2): 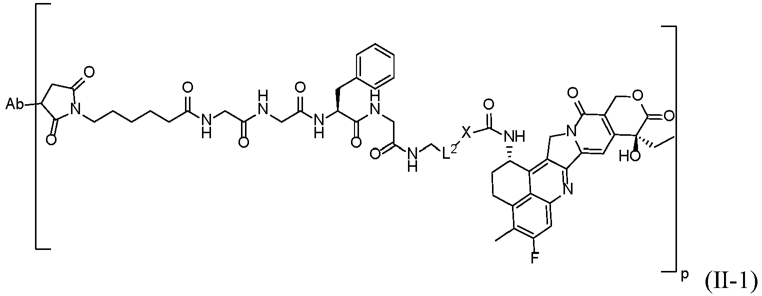 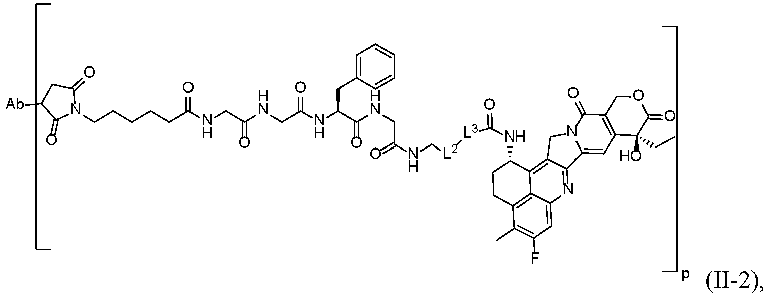 wherein,
p represents an average connection number, and p is selected from integers or decimals from 1 to 10, preferably integers or decimals from 3 to 8;
Ab is as defined in any one of the embodiments herein;
L² is -O- or -S-;
X is selected from cyclopropyl, cyclobutyl, cyclopentyl, or cyclohexyl;
L³ is selected from -CH₂-, -CH(CH₃)-, -C(CH₃)₂-, -CH₂CH₂-, -CH(CH₃)CH₂-, or -C(CH₃)₂CH₂-.

In some embodiments, described herein is an anti-Her3 antibody-drug conjugate, the isomer thereof, the pharmaceutically acceptable salt thereof, or the mixture thereof, wherein the anti-Her3 antibody-drug conjugate is selected from the following structural formulas: 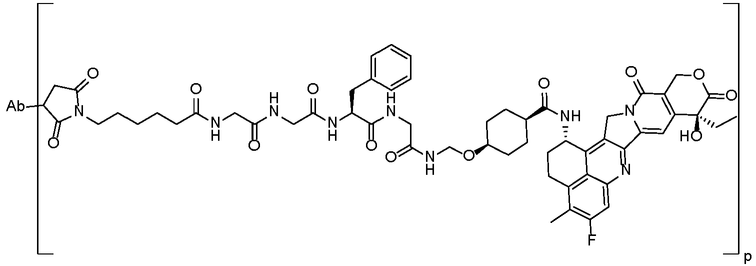 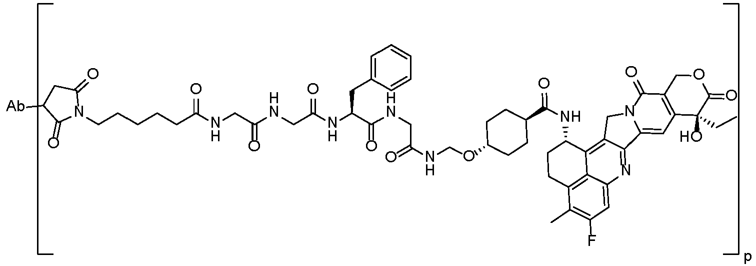 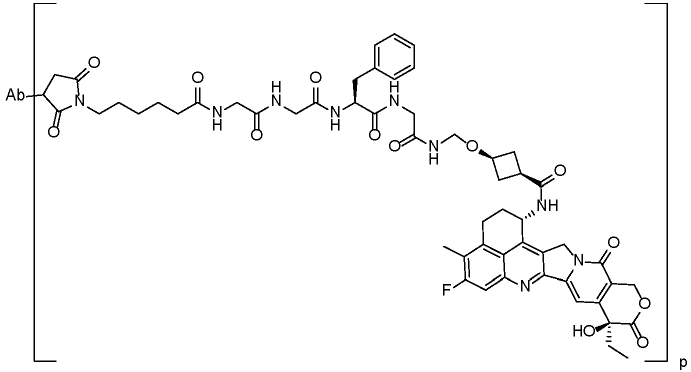 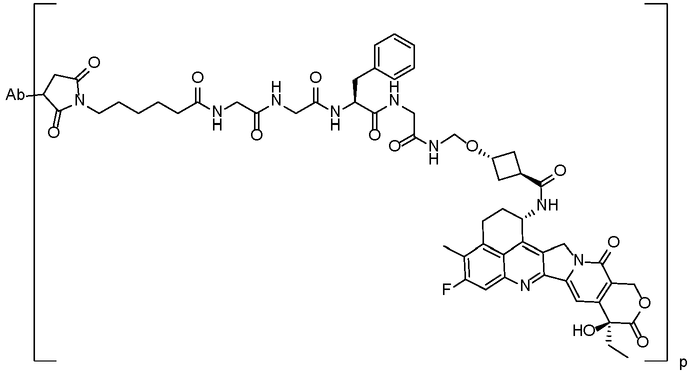 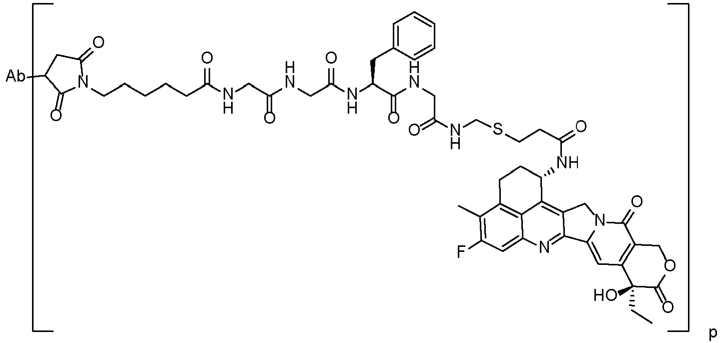 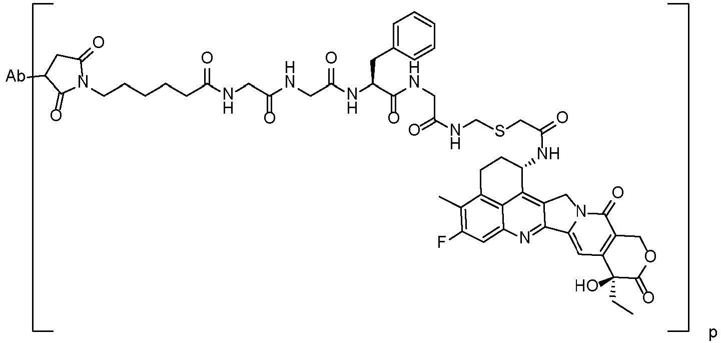 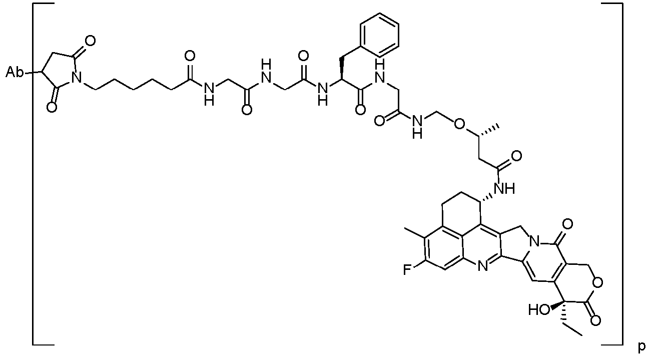 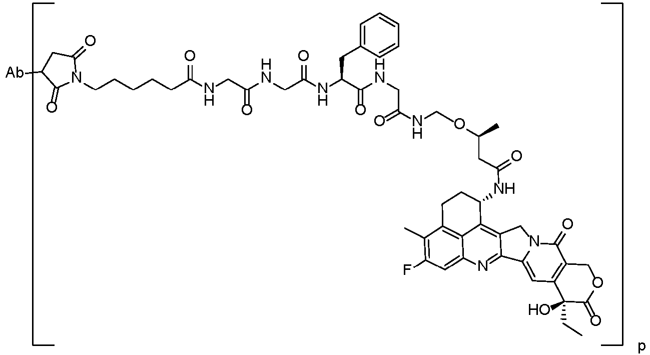 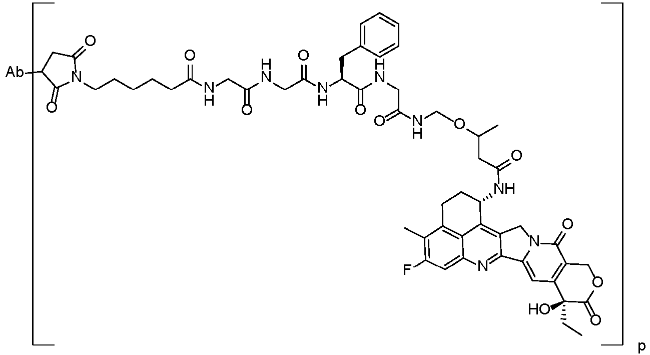 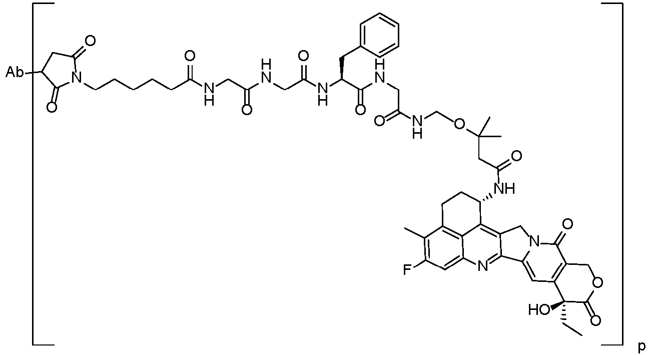 wherein,
p represents an average connection number, and p is selected from integers or decimals from 1 to 10, preferably integers or decimals from 3 to 8;
Ab is as defined in any one of the embodiments herein.

In yet another aspect, the present invention provides an anti-Her3 antibody-drug conjugate, an isomer thereof, a pharmaceutically acceptable salt thereof, or a mixture thereof, wherein the anti-Her3 antibody-drug conjugate is selected from: 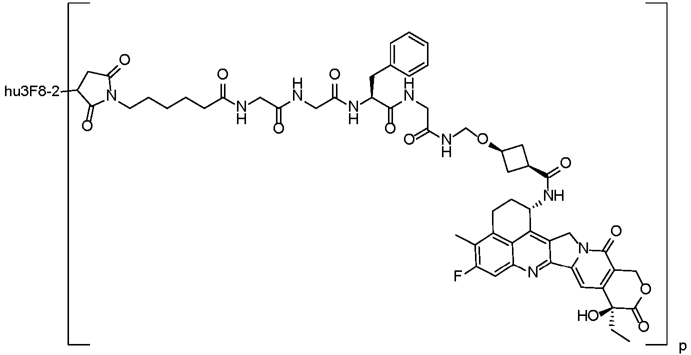 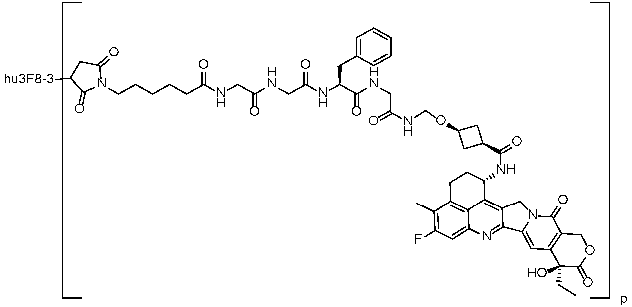 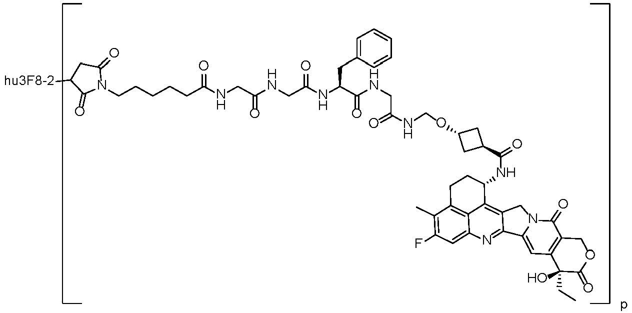 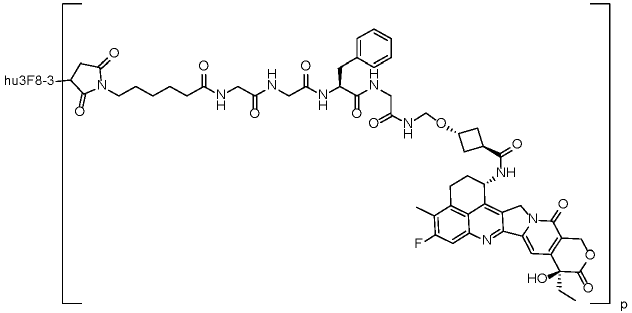 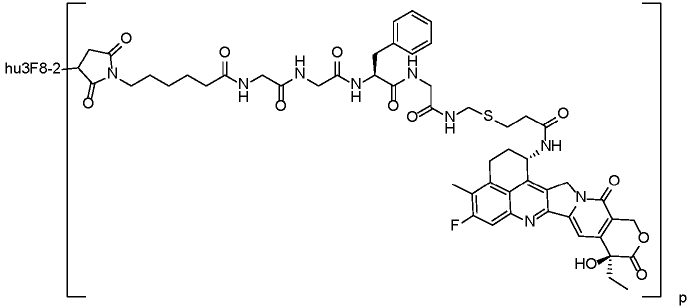 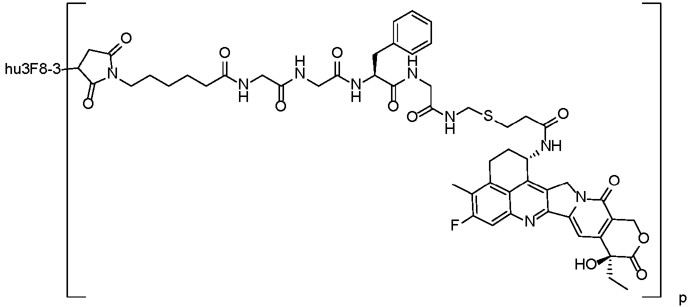 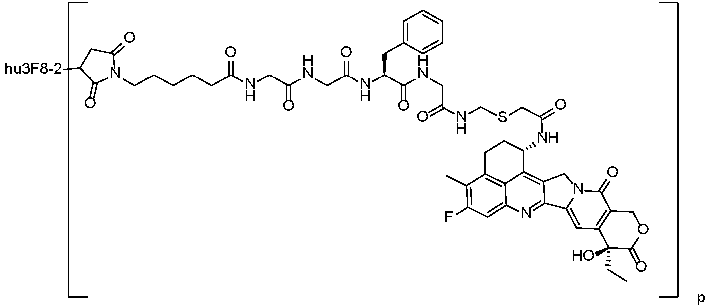 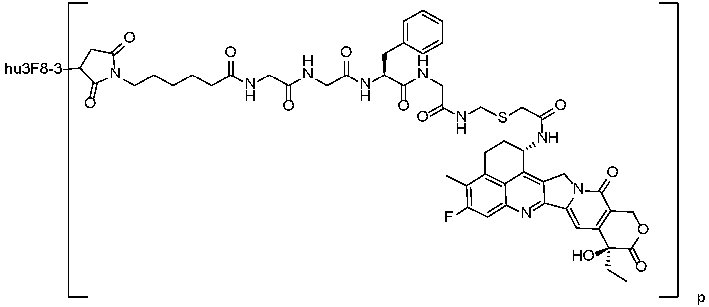 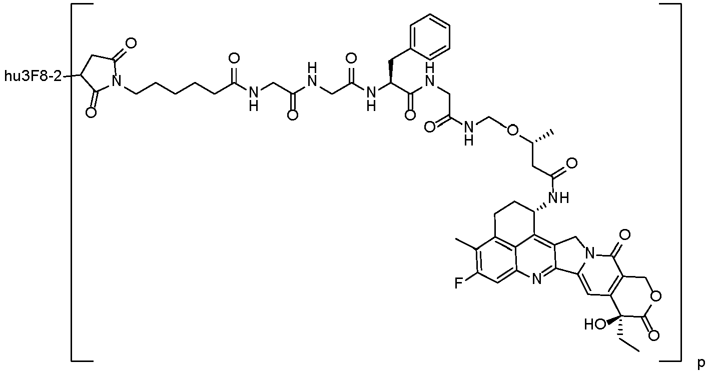 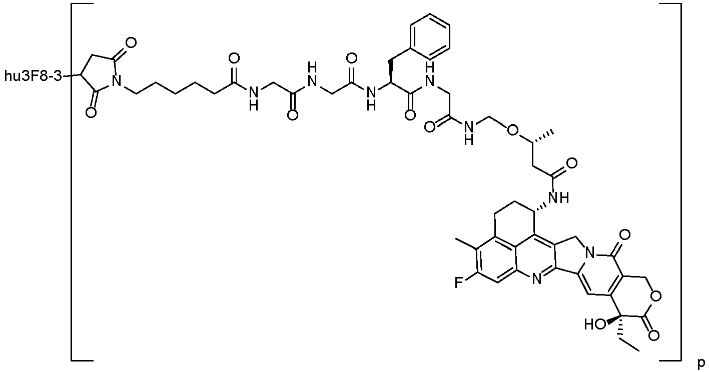 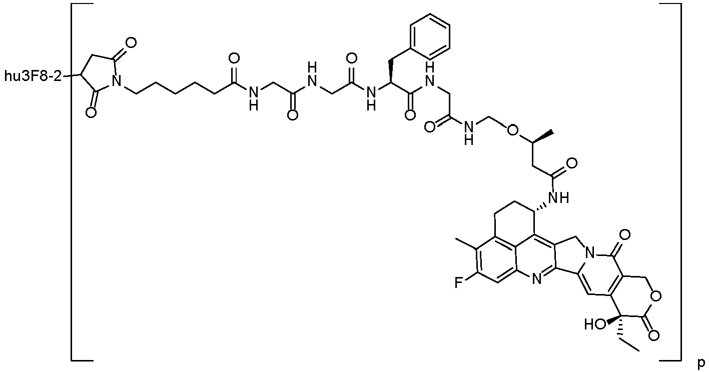 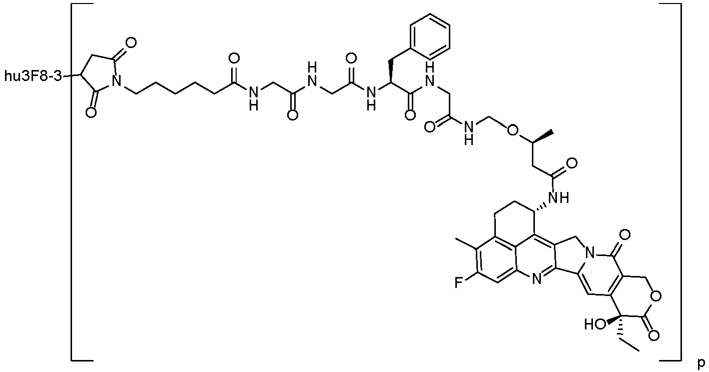 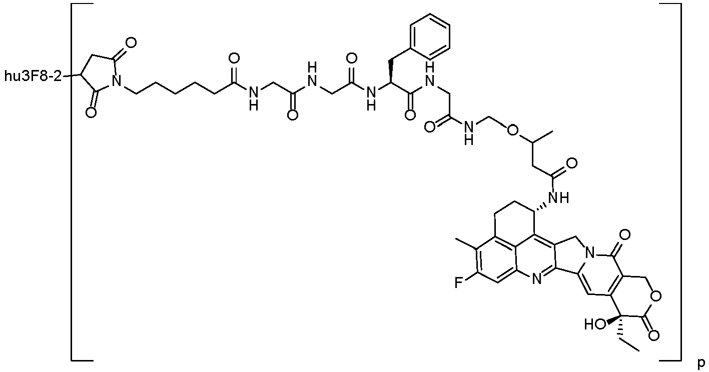 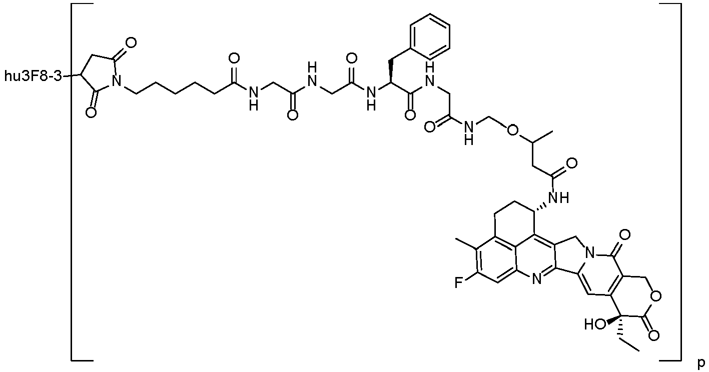 wherein,
p represents an average connection number, and p is selected from integers or decimals from 1 to 10, preferably integers or decimals from 3 to 8.

The hu3F8-2 and hu3F8-3 in the present invention are anti-Her2 antibodies. The anti-Her2 antibody hu3F8-2 comprises a heavy chain amino acid sequence set forth in SEQ ID NO: 13 and a light chain amino acid sequence set forth in SEQ ID NO: 12; the anti-Her2 antibody hu3F8-3 comprises a heavy chain amino acid sequence set forth in SEQ ID NO: 14 and a light chain amino acid sequence set forth in SEQ ID NO: 12.

In some embodiments, the average connection number p described herein may be an integer or a decimal from 2 to 8. For example, the average connection number n may be an integer or a decimal from 3 to 8. For example, the average connection number n may be an integer or a decimal from 1 to 2, 2 to 3, 3 to 4, 4 to 5, 5 to 6, 6 to 7, 7 to 8, 8 to 9, or 9 to 10.

In yet another aspect, the present invention provides a pharmaceutical composition, which comprises the anti-Her3 antibody-drug conjugate, the isomer thereof, the pharmaceutically acceptable salt thereof, or the mixture thereof described herein, and a pharmaceutically acceptable carrier or excipient.

In yet another aspect, the present invention provides use of the anti-Her3 antibody-drug conjugate, the isomer thereof, the pharmaceutically acceptable salt thereof, or the mixture thereof or the pharmaceutical composition described herein in preparing a medicament for treating and/or preventing a Her3-mediated disease or condition, wherein preferably, the disease or condition is cancer.

In yet another aspect, the present invention provides a method for treating and/or preventing a Her3-mediated disease or condition, which comprises administering to a subject in need the anti-Her3 antibody-drug conjugate, the isomer thereof, the pharmaceutically acceptable salt thereof, or the mixture thereof or the pharmaceutical composition described herein, wherein preferably, the disease or condition is cancer.

In yet another aspect, the present invention provides the anti-Her3 antibody-drug conjugate, the isomer thereof, the pharmaceutically acceptable salt thereof, or the mixture thereof or the pharmaceutical composition described herein for treating and/or preventing a Her3-mediated disease or condition, wherein preferably, the disease or condition is cancer.

In some embodiments, the cancer described herein is selected from lung cancer, kidney cancer, urinary tract carcinoma, colorectal cancer, prostate cancer, glioblastoma multiforme, ovarian cancer, pancreatic cancer, breast cancer, melanoma, liver cancer, bladder cancer, gastric cancer, and esophageal cancer.

In yet another aspect, the present invention provides a pharmaceutical combination, which comprises the antibody-drug conjugate or the pharmaceutically acceptable salt thereof described herein, or the pharmaceutical composition described herein, and one or more additional therapeutic agents.

In yet another aspect, the present invention provides a kit, which comprises the antibody-drug conjugate described herein or the pharmaceutical composition described herein.

### BRIEF DESCRIPTION OF THE DRAWINGS

FIG. 1 shows that murine 3F8 specifically bound to SP2/0-HER3 cells.
FIG. 2 shows binding affinities of murine 3F8 to human HER3, HER2 and EGFR measured by ELISA.
FIG. 3 shows the potency of murine 3F8 to recognize human and monkey HER3 measured by ELISA.
FIG. 4 shows that murine 3F8 blocked NRG1-induced HER3 phosphorylation.
FIG. 5 shows that murine 3F8 was rapidly taken up by cells with different surface HER3 levels.
FIG. 6 shows that the anti-HER3 antibody inhibited tumor growth in the BT474 subcutaneous xenograft model.
FIG. 7 is [⁸⁹Zr]Zr-ch3F8 imaging in gastric PDX model GAS078.
FIG. 8 shows representative [⁸⁹Zr]Zr-ch3F8 imaging in 6 PDX models.
FIGs. 9A-9C show that hu3F8 retains binding affinity after stress testing of heat, acid, and repeated freeze-thaw. FIG. 9A three clones of hu3F8 were incubated at pH 3.5 for 0, 2, 4 and 6 h, and then subjected to ELISA to measure the binding affinities. FIG. 9B three clones of hu3F8 were incubated at 40 °C for different days, and then subjected to ELISA to measure the binding affinities. FIG. 9C three clones of hu3F8 were repeatedly freeze-thawed for 3 or 5 cycles, and then subjected to ELISA to measure the binding affinities.
FIG. 10 shows the internalization activity of the antibody-drug conjugates.
FIGs. 11A-11C show the test for inhibition of in vitro proliferation of tumor cells of HER3 target by antibody-drug conjugates ADC-1-X1 and ADC-1-X2; FIG. 11A shows HCC1569 tumor cells with high expression of Her3; FIG. 11B shows MDA-MB-453 tumor cells with high expression of Her3;
FIG. 11C shows MDA-MB-231 tumor cells not expressing Her3.
FIGs. 12A-12B show in vivo anti-tumor efficacy of antibody-drug conjugates ADC-1-X1 and ADC-1-X2 in HCC1569 tumor-bearing mice. FIG. 12A shows changes in tumor volume. FIG. 12B shows tumor growth inhibition.
FIGs. 13A-13B show in vivo anti-tumor efficacy of antibody-drug conjugates ADC-1-X1 and ADC-1-X2 in Colo205 tumor-bearing mice. FIG. 13A shows changes in tumor volume. FIG. 13B shows tumor growth inhibition.
FIG. 14 shows in vivo anti-tumor efficacy of antibody-drug conjugate ADC-1-X2 in NCI-H441 lung cancer cell tumor-bearing mice.
FIG. 15 shows in vivo anti-tumor efficacy of antibody-drug conjugate ADC-1-X2 in human lung cancer LU1542 subcutaneous xenograft tumor-bearing mice.

### DETAILED DESCRIPTION

The embodiments of the present invention are described below with reference to specific examples, and other advantages and effects of the present invention will be readily apparent to those skilled in the art from the disclosure of the present specification.

### Definitions of terms

Unless otherwise stated, embodiments of the present invention will employ conventional technologies of molecular biology (including recombinant technology), microbiology, cytobiology, biochemistry and immunology, which are all within the skill of the art.

In order to facilitate the understanding of the present invention, some technical and scientific terms are specifically defined as follows. Unless otherwise specifically defined herein, all technical and scientific terms used herein have the same meaning as commonly understood by those of ordinary skill in the art to which the present invention belongs. For definitions and terminology in the art, the skilled person can refer specifically to Current Protocolsin Molecular Biology (Ausubel). Abbreviations for amino acid residues are standard 3-letter and/or 1-letter codes used in the art to denote one of the 20 commonly used L-amino acids. The singular forms used herein (including claims) include their plural forms, unless otherwise specified in the context explicitly.

The term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% above or below the stated value.

The term "human epidermal growth factor receptor 3 (Her3)", also known as receptor tyrosine-protein kinase ERBB-3 (ERBB3), is a member of the EGFR/erBB family. Unlike other erBB family members Her2 and EGFR, Her3 itself does not have kinase activity. Thus, Her3 must bind to its kinase active member EGFR or Her2 to trigger its downstream activity as a heterodimer. Upon binding to its natural ligand NRG1, Her3 undergoes conformational changes, heterodimerization and phosphorylation, and then activates MAPK, PI3K/Akt, and PLCy via signal transduction.

The term "antibody" refers to any form of antibody having a desired bioactivity. Thus, it is used in the broadest sense and specifically includes, but is not limited to, monoclonal antibodies (including full-length monoclonal antibodies), polyclonal antibodies, multispecific antibodies (e.g., bispecific antibodies), humanized antibodies, fully human antibodies, chimeric antibodies, and camelized single-domain antibodies.

A "monoclonal antibody" refers to an antibody obtained from a substantially homogeneous population of antibodies, i.e., the antibodies composing the population are identical except for possible naturally occurring mutations that may be present in minor amounts. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristic of an antibody obtained from a substantially homogeneous population of antibodies, and is not to be construed as producing the antibody by any particular method.

The term "full-length antibody" refers to an immunoglobulin molecule, when naturally occurring, comprising four peptide chains, in which two heavy (H) chains (about 50-70 kDa in total length) and two light (L) chains (about 25 kDa in total length) are linked to each other by disulfide bonds. Each heavy chain consists of a heavy chain variable region (abbreviated herein as VH) and a heavy chain constant region (abbreviated herein as CH). The heavy chain constant region consists of 3 domains (CH1, CH2 and CH3). Each light chain consists of a light chain variable region (abbreviated herein as VL) and a light chain constant region. The light chain constant region consists of one domain CL. The VH and VL regions can be further divided into complementarity determining regions (CDRs) with high variability and more conservative regions called framework regions (FRs) that are spaced apart by the CDRs. Each VH or VL region consists of 3 CDRs and 4 FRs arranged in the following order from the amino terminus to the carboxyl terminus: FR1, CDR1, FR2, CDR2, FR3, CDR3, FR4. The variable regions of the heavy and light chains comprise binding domains that interact with antigens. The constant regions of an antibody can mediate the binding of immunoglobulins to host tissues or factors, including the binding of various cells of the immune system (e.g., effector cells) to the first component (Clq) of a classical complement system.

The term "CDR" refers to a complementarity determining region within an antibody variable sequence. There are 3 CDRs in each of the heavy chain variable regions and light chain variable regions, which are named HCDR1, HCDR2 and HCDR3 for the heavy chain variable region, or LCDR1, LCDR2 and LCDR3 for the light chain variable region. The precise amino acid sequence boundaries of the variable region CDRs of the antibodies of the present invention can be determined using any of a number of well-known schemes, including Chothia based on the three-dimensional structure of antibodies and the topology of the CDR loops (Chothia et al., (1989) Nature 342: 877-883; Al-Lazikani et al., Standard conformations for the canonical structures of immunoglobulins, Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (1999 Nucleic Acids Research, 27, 209-212), and North CDR definition based on the affinity propagation clustering using a large number of crystal structures. The boundaries of the CDRs of the antibodies disclosed herein can be determined by one skilled in the art according to any scheme (e.g., different assignment systems or combinations) in the art.

It should be noted that boundaries of CDRs in variable regions of the same antibody determined by different assignment systems may differ. That is, CDR sequences of variable regions of the same antibody defined by different assignment systems differ. Thus, when it comes to defining an antibody with a particular CDR sequence defined in the present invention, the scope of the antibody also encompasses antibodies whose variable region sequences comprise the particular CDR sequence but whose claimed CDR boundaries differ from the particular CDR boundaries defined in the present invention due to the application of different schemes (e.g., different assignment systems or combinations).

The term "antigen-binding fragment" of an antibody ("parent antibody") includes a fragment or a derivative of the antibody, generally including at least one fragment of an antigen-binding region or variable region (e.g., one or more CDRs) of a parent antibody, which retains at least some of the binding specificity of the parent antibody. Examples of binding fragments of an antibody include, but are not limited to, Fab, Fab', F(ab')2 and Fv fragments; a bispecific antibody; a linear antibody; a single-chain antibody molecule, such as scFv; and a nanobody and a multispecific antibody formed by fragments of the antibody. A binding fragment or a derivative generally retains at least 10% of its antigen-binding activity when the antigen-binding activity is expressed on a molar concentration basis. Preferably, the binding fragment or the derivative retains at least 20%, 50%, 70%, 80%, 90%, 95% or 100% or more of the antigen-binding affinity of the parent antibody. It is also contemplated that an antigen-binding fragment of an antibody may include conservative or non-conservative amino acid substitutions that do not significantly alter its bioactivity (referred to as "conservative variants" or "function-conservative variants" of the antibody).

A "chimeric antibody" is an antibody having the variable domains of a first antibody and the constant domains of a second antibody, wherein the first and second antibodies are from different species. Typically, the variable domains are obtained from an antibody of an experimental animal such as a rodent ("parent antibody"), and the constant domain sequences are obtained from a human antibody, such that the resulting chimeric antibody is less likely to induce an adverse immune response in a human subject as compared to the parent rodent antibody.

A "humanized antibody" refers to an antibody form containing sequences from both human and non-human (such as mouse and rat) antibodies. In general, a humanized antibody comprises substantially all of at least one, and typically two, variable domains, in which all or substantially all of the hypervariable loops correspond to those of a non-human immunoglobulin and all or substantially all of the framework regions (FRs) are those of a human immunoglobulin sequence. The humanized antibody may optionally comprise at least a portion of a human immunoglobulin constant region (Fc). The term "fully human antibody" refers to an antibody that comprises only human immunoglobulin sequences. A fully human antibody may contain mouse glycochains if produced in mice, mouse cells or hybridomas derived from mouse cells. Likewise, a "mouse antibody" refers to an antibody that comprises only mouse immunoglobulin sequences. Alternatively, a fully human antibody may contain rat glycochains if produced in rats, rat cells or hybridomas derived from rat cells. Likewise, a "rat antibody" refers to an antibody that comprises only rat immunoglobulin sequences.

The term "isotypes" of antibodies refer to types of antibodies (e.g., IgM, IgE and IgG (such as IgG1, IgG2 or IgG4)) provided by heavy chain constant region genes. Isotype also includes modified forms of one of these types in which modifications have been made to alter Fc function, for example, to enhance or attenuate effector function or binding to Fc receptors.

The term "Fc region" is used to define the C-terminal region of an immunoglobulin heavy chain comprising at least a part of constant regions. The term includes a native sequence Fc region and a variant Fc region. In some embodiments, the Fc region of a human IgG heavy chain extends from Cys226 or Pro230 to the carboxyl-terminus of the heavy chain. However, the C-terminal lysine (Lys447) of the Fc region may or may not exist (numbering in this paragraph is according to the EU numbering system, also known as the EU index, Kabat et al., Sequences of Proteins of Immunological Interest, 5th Ed. Public Health Service, National Institutes of Health, Bethesda, MD, 1991).

The term "cross-reaction" refers to binding to antigenic fragments of the same target molecule of human, monkey and/or murine (mouse or rat) origin. Thus, "cross-reaction" should be understood as an interspecies reaction with the same molecule X expressed in different species. The cross-reaction specificity of monoclonal antibodies recognizing human Her3R, and monkey and/or murine (mouse or rat) Her3R can be determined by FACS analysis.

The term "affinity" or "binding affinity" refers to the inherent binding affinity that reflects the interaction between members of a binding pair. The affinity of molecule X for its partner Y can be generally represented by the equilibrium dissociation constant (KD), which is a ratio of the dissociation rate constant to the association rate constant (kdis and kon, respectively). Affinity can be measured by common methods known in the art. One specific method for measuring affinity is the ForteBio kinetic binding assay described herein.

The term "not bind to" a protein or cell means not binding to the protein or cell, or not binding to it with high affinity, that is, binding to the protein or cell with a K_{D} of 1.0 × 10⁻⁶ M or higher, more preferably 1.0 × 10⁻⁵ M or higher, more preferably 1.0 × 10⁻⁴ M or higher and 1.0 × 10⁻³ M or higher, and more preferably 1.0 × 10⁻² M or higher.

The term "high affinity" of IgG antibodies refers to a K_{D} for the antigen of 1.0 × 10⁻⁶ M or less, preferably 5.0 × 10⁻⁸ M or less, more preferably 1.0 × 10⁻⁸ M or less and 5.0 × 10⁻⁹ M or less, and more preferably 1.0 × 10⁻⁹ M or less. For other antibody subtypes, "high affinity" binding may vary. For example, "high affinity" binding of the IgM subtype refers to a K_{D} of 10⁻⁶ M or less, preferably 10⁻⁷ M or less, and more preferably 10⁻⁸ M or less.

The term "cytotoxic drug" generally refers to a toxic drug, and the cytotoxic drug may be a chemical molecule within the tumor cell that is strong enough to disrupt its normal growth. Cytotoxic drugs can kill tumor cells at a sufficiently high concentration. The "cytotoxic drug" may include toxins, such as small molecule toxins or enzymatically active toxins of bacterial, fungal, plant or animal origin, radioisotopes (e.g., At²¹¹, I¹³¹, I¹²⁵, Y⁹⁰, Re¹⁸⁶, Re¹⁸⁸, Sm¹⁵³, Bi²¹², P³² or radioactive isotopes of Lu), toxic drugs, chemotherapeutic drugs, antibiotics and nucleolytic enzymes; for example, the cytotoxic drug may be toxic drugs, including but not limited to camptothecin derivatives, which, for example, may be the camptothecin derivative exatecan (chemical name: (1*S*,9*S*)-1-amino-9-ethyl-5-fluoro-2,3-dihydro-9-hydroxy-4-methyl-1*H*,12*H*-benzo[*de*]pyrano[3',4':6,7]imidazo[1,2-*b*] quinoline-10,13(9*H*,15*H*)-dione).

The term "linker unit" or "linker structure" generally refers to a chemical structural fragment or bond that is linked to a ligand at one end and to a cytotoxic drug at the other end, or that is linked to other linkers before being linked to the cytotoxic drug. The direct or indirect linking of a ligand may mean that the group is directly linked to the ligand via a covalent bond, and may also be linked to the ligand via a linker structure. For example, the linker structure may be a structure shown as -Lax-Lb-Lc-and/or -La-Lb-Lc- described herein. For example, a chemical structure fragment or bond comprising an acid-labile linker structure (e.g., hydrazone), a protease-sensitive (e.g., peptidase-sensitive) linker structure, a photolabile linker structure, a dimethyl linker structure or a disulfide-containing linker structure may be used as a linker structure.

The term a structure being "optionally linked to other molecular moieties" generally means that the structure is not linked to any other chemical structure, or that the structure is linked (e.g., via a chemical bond or a linker structure) to one or more other chemical structures (e.g., ligands described herein) different from the structure.

The term "ligand-drug conjugate" generally means that a ligand is linked to a biologically active cytotoxic drug via a stable linking unit. In the present application, the "ligand-drug conjugate" may be an antibody-drug conjugate (ADC), which may mean that a monoclonal antibody or an antibody fragment is linked to a biologically active cytotoxic drug via a stable linking unit.

In the present application, the term "methylene" generally refers to a residue derived by removal of two hydrogen atoms from a group having 1 carbon atom. Methylene may be substituted or unsubstituted, or replaced or unreplaced. The term "alkylene" generally refers to a saturated linear or branched aliphatic hydrocarbon group having 2 residues derived from the parent alkane by removal of two hydrogen atoms from the same carbon atom or two different carbon atoms, and it may be a linear or branched group containing 1 to 20 carbon atoms, such as alkylene containing 1 to 12 carbon atoms (e.g., 1 to 6 carbon atoms). Non-limiting examples of alkylene include, but are not limited to, methylene(-CH₂-), 1,1-ethylidene(-CH(CH₃)-), 1,2-ethylidene(-CH₂CH₂)-, 1,1-propylidene(-CH(CH₂CH₃)-), 1,2-propylidene(-CH₂CH(CH₃)-), 1,3-propylidene(-CH₂CH₂CH₂-), 1,4-butylidene(-CH₂CH₂CH₂CH₂-), 1,5-butylidene(-CHzCHzCHzCHzCHz-), etc. Alkylene groups may be substituted or unsubstituted, replaced or unreplaced. For example, when it is substituted, substitution with a substituent may be performed at any available linking point, and the substituent is preferably independently optionally selected from one or more of alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio, and oxo, and it may, e.g., be hydrogen, protium, deuterium, tritium, halogen, -NO₂, -CN, -OH, -SH, -NH₂, -C(O)H, -CO₂H, - C(O)C(O)H, -C(O)CH₂C(O)H, -S(O)H, -S(O)₂H, -C(O)NH₂, -SO₂NH₂, -OC(O)H, -N(H)SO₂H or a C₁₋₆ aliphatic group.

The term "arylene" generally refers to a group having two residues derived by removal of two hydrogen atoms from the same carbon atom or two different carbon atoms of the aromatic ring. The term "aromatic ring" may refer to a 6- to 14-membered all-carbon monocyclic ring or fused polycyclic ring (i.e., rings that share adjacent pairs of carbon atoms) having a conjugated π-electron system, and it may be 6- to 10-membered, such as benzene and naphthalene. The aromatic ring can be fused to a heteroaryl, heterocyclyl or cycloalkyl ring, wherein the ring connected to the parent structure is the aryl ring. Aryl may be substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

In the present application, the term "heteroarylene" generally refers to a group having two residues derived by removal of two hydrogen atoms from the same carbon atom or two different carbon atoms of the heteroaromatic ring. The term "heteroaromatic ring" refers to a heteroaromatic system comprising 1 to 4 heteroatoms and 5 to 14 ring atoms, wherein the heteroatoms may be selected from the group consisting of: oxygen, sulfur and nitrogen. Heteroaryl may be 5- to 10-membered and may be 5- or 6-membered, such as furanyl, thienyl, pyridinyl, pyrrolyl, *N*-alkylpyrrolyl, pyrimidinyl, pyrazinyl, imidazolyl and tetrazolyl. The heteroaryl ring can be fused to an aryl, heterocyclyl or cycloalkyl ring, wherein the ring connected to the parent structure is the heteroaryl ring. Heteroarylene may be optionally substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "heterocyclylene" generally refers to a 3- to 7-membered monocyclic structure, a fused 7-to 10-membered bicyclic heterocyclic structure or a bridged 6- to 10-membered bicyclic heterocyclic structure that is stable and non-aromatic. These cyclic structures may be saturated or partially saturated, and contain one or more heteroatoms in addition to carbon atoms, wherein the heteroatoms may be selected from the group consisting of: oxygen, sulfur and nitrogen. For example, they contain 1 to 4 heteroatoms as defined above. When used to refer to atoms on a heterocyclic cyclic structure, the term "nitrogen" may include nitrogen that has undergone a substitution reaction. Heterocyclylene may be substituted or unsubstituted.

The term "carbocyclylene" generally refers to a group having two residues derived by removal of two hydrogen atoms from the same carbon atom or two different carbon atoms of the carbon ring. The term "carbon ring" generally refers to a saturated or partially unsaturated monocyclic or polycyclic cyclic hydrocarbon, and it contains 3 to 20 carbon atoms, may contain 3 to 12 carbon atoms, may contain 3 to 10 carbon atoms, and may contain 3 to 8 carbon atoms, for example, 3 to 6 carbon atoms. Non-limiting examples of a monocyclic carbon ring include cyclopropane, cyclobutane, cyclopentane, cyclopentene, cyclohexane, cyclohexene, cyclohexadiene, cycloheptane, cycloheptatriene, cyclooctane, etc.; polycyclic carbon rings may include spiro, fused and bridged carbon rings. For example, "3- 6 membered saturated carbocyclyl" includes cyclopropyl, cyclobutyl, cyclopentyl, cyclohexyl, cyclopropylene, cyclobutylene, cyclopentylene, or cyclohexylene. Carbocyclyl and carbocyclylene may be substituted or unsubstituted. For example, they may be substituted with one or more halogens, a C₁₋₆ aliphatic group, or a C₁₋₆ aliphatic group substituted with halogen.

In the present application, the term "partially unsaturated" generally means that the cyclic structure contains at least one double or triple bond between the ring molecules. The term "partially unsaturated" encompasses cyclic structures having multiple sites of unsaturation, but is not intended to include aromatic or heteroaromatic rings defined herein. The term "unsaturated" means that the moiety has one or more degrees of unsaturation.

The term "halogen" generally refers to fluorine, chlorine, bromine or iodine, and it may be, for example, fluorine or chlorine.

The term "aliphatic group" generally refers to a linear hydrocarbon, branched hydrocarbon or cyclic hydrocarbon having 1-12 carbon atoms, and the hydrocarbon may be either a fully saturated hydrocarbon or a hydrocarbon with one or more unsaturated units, but the unsaturated units are not aromatic groups. For example, suitable aliphatic groups may include substituted or unsubstituted linear, branched or cyclic alkyl, alkenyl, alkynyl and mixtures of the groups, such as (cycloalkyl)alkyl, (cycloalkenyl)alkyl or (cycloalkyl)alkenyl. For example, aliphatic groups have 1-12, 1-8, 1-6, 1-4 or 1-3 carbon atoms. For example, "C₁₋₆ aliphatic group" refers to an aliphatic group as described above having 1-6 carbon atoms, including but not limited to linear, branched or cyclic alkyl, alkenyl, or alkynyl having 1-6 carbon atoms, such as methyl, ethyl, propyl, isopropyl, n-butyl, isobutyl, *tert-*butyl, pentyl, and hexyl.

In the present application, the term "optional" or "optionally" generally means that the event or circumstance subsequently described may, but not necessarily occur, and that the description includes instances where the event or circumstance occurs or does not occur. For example, "heterocyclyl group optionally substituted with alkyl" means that alkyl may be, but not necessarily present, and that the description may include instances where the heterocyclyl group is or is not substituted with alkyl. The term "substituted" generally means that one or more hydrogen atoms in the group, for example, up to 5 (e.g., 1-3) hydrogen atoms, are each independently substituted with a corresponding number of substituents. A substituent is only in its possible chemical position, and those skilled in the art will be able to determine (by experiments or theories) possible or impossible substitution without undue efforts. For example, it may be unstable when amino or hydroxy having a free hydrogen is bound to a carbon atom having an unsaturated (such as olefin) bond.

In the present application, the term "0 or more (e.g., 0 or at least 1, 0 or 1, or 0) methylene units are replaced" generally means that when the structure comprises one or more methylene units, the one or more methylene units may be unreplaced or replaced by one or more groups that are not methylene (e.g., -NHC(O)-, -C(O)NH-, -C(O)-, -OC(O)-, -C(O)O-, -NH-, -O-, -S-, -SO-, -SO₂-, -PH-, -P(=O)H-, -NHSO₂-, -SO₂NH-, -C(=S)-, -C(=NH)-, -N=N-, -C=N-, -N=C- or -C(=N₂)-).

One or more hydrogen atoms in the group, for example, up to 5 (e.g., 1-3) hydrogen atoms, are each independently substituted with a corresponding number of substituents. A substituent is only in its possible chemical position, and those skilled in the art will be able to determine (by experiments or theories) possible or impossible substitution without undue efforts. For example, it may be unstable when amino or hydroxy having a free hydrogen is bound to a carbon atom having an unsaturated (such as olefin) bond.

The term "pharmaceutically acceptable salt" generally refers to a salt of the drug conjugate disclosed herein. Such salts may be safe and/or effective when used in mammals and may possess the required biological activity, and the antibody-drug conjugate disclosed herein may form a salt with an acid.

Non-limiting examples of pharmaceutically acceptable salts include: hydrochloride, hydrobromide, hydriodate, sulfate, bisulfate, citrate, acetate, succinate, ascorbate, oxalate, nitrate, sorbate, hydrophosphate, dihydrophosphate, salicylate, hydrocitrate, tartrate, maleate, fumarate, formate, benzoate, mesylate, ethanesulfonate, benzenesulfonate and*p*-toluenesulfonate.

The term "solvate" or "solvent compound" generally refers to a pharmaceutical acceptable solvate formed by the drug conjugate disclosed herein and one or more solvent molecules, and non-limiting examples of solvent molecules include water, ethanol, acetonitrile, isopropanol, DMSO and ethyl acetate.

The term "drug loading" generally refers to the average amount of cytotoxic drug loaded per ligand and may also be expressed as the ratio of cytotoxic drug to antibody, and the cytotoxic drug loading may range from 0 to 12 (e.g., 1-10) cytotoxic drugs per ligand (Ab). In the embodiments of the present application, the drug loading is expressed as Na, and may illustratively be an average of 1, 2, 3, 4, 5, 6, 7, 8, 9 and 10. The drug loading per ADC molecule after the coupling reaction can be characterized by conventional methods such as UV/visible spectroscopy, mass spectrometry, ELISA assays and HPLC.

"Pharmaceutically acceptable carrier" refers to a component in a pharmaceutical formulation or composition other than the active component that is non-toxic to the subject. A pharmaceutically acceptable carrier includes, but is not limited to, buffer, excipient, stabilizer or preservative.

The term "comprise" "comprising", "contain" or "containing" is generally intended to include the explicitly specified features without excluding other elements. The terms "no less than" and "no more than" generally refer to the situations where the number itself is included.

"Subject" includes any human or non-human animal. The term "non-human animal" includes all vertebrates, e.g., mammals and non-mammals, such as non-human primates, sheep, dog, cat, horse, cattle, chicken, amphibians, and reptiles. As used herein, the term "cyno" refers to a cynomolgus monkey.

Administration "in combination with" one or more other therapeutic agents includes simultaneous (co-) administration and sequential administration in any order.

"Therapeutically effective amount", "therapeutically effective dose" and "effective amount" refer to an amount of the Her3 antibody or the antigen-binding fragment thereof of the present invention that is effective in preventing or improving one or more symptoms of a disease or condition or the development of the disease or condition, when administered alone or in combination with other therapeutic agents to a cell, tissue or subject. The therapeutically effective dose also refers to an amount of the antibody or the antigen-binding fragment thereof sufficient to cause amelioration of symptoms, e.g., an amount for treating, curing, preventing or ameliorating a related condition or promoting the treatment, cure, prevention or amelioration of such condition. When an active ingredient is administered to an individual alone, a therapeutically effective dose only refers to the amount of the ingredient. In the case of administration in combination, a therapeutically effective dose refers to the combined amount of active ingredients that produces a therapeutic effect, regardless of whether these active ingredients are administered in combination, sequentially or simultaneously. An effective amount of a therapeutic agent will result in an increase in a diagnostic index or parameter by at least 10%, generally at least 20%, preferably at least about 30%, more preferably at least 40%, and most preferably at least 50%.

The term "cancer" is used herein to refer to a group of cells that exhibit abnormally high levels of proliferation and growth. Cancer may be benign (also referred to as benign tumor), premalignant or malignant. Cancer cells may be solid cancer cells or leukemia cancer cells. The term "tumor" as used herein refers to one or more cells comprising cancer. The term "tumor growth" is used herein to refer to the proliferation or growth of one or more cells comprising cancer, which results in a corresponding increase in the size or extent of the cancer.

### Anti-Her3 antibody

The present application discloses a novel anti-HER3 antibody, which can effectively inhibit the growth of tumor cells in vitro and in vivo and has good safety. The HER3 antibody 3F8 was identified in mouse hybridomas immunized with SP2/0 cells overexpressing human HER3. 3F8 recognizes human and monkey HER3 with a nanomolar binding affinity and has a high selectivity for other ERBB family members. In addition, cells with different HER3 levels can rapidly and effectively take up HER3. HER3 is considered an essential property for ideal ADC drugs. PET imaging studies have shown that [⁸⁹Zr]Zr-3F8 accumulates significantly in tumors of a PDX model, indicating that 3F8 may be an effective vector, bringing cytotoxicity into tumor cells. 3F8 and hu3F8 maintain the above binding affinity, selectivity, and tumor suppression characteristics in the PDX model. hu3F8 has good developability and shows little change in binding affinity, aggregation and post-translational modification in stress tests of repeated freeze-thaw treatments, acid incubation and storage at 40 °C. The term "anti-Her3 antibody", "anti-Her3", "Her3 antibody" or "antibody that binds to Her3" of the present invention refers to an antibody that is capable of binding to Her3 protein or a fragment thereof with sufficient affinity such that the antibody can be used as a diagnostic and/or therapeutic agent in targeting Her3.

The anti-Her3 antibody or the antigen-binding fragment thereof described herein includes any one of the anti-Her3 antibodies or the antigen-binding fragments thereof described in the Application No. PCT/CN2021/099998, the disclosure of which is incorporated herein by reference in its entirety. In some embodiments, the CDR sequences of the antibody used in the drug conjugate, composition, use or method of the present invention comprise the CDR sequences of the antibody hu3F8-2 described in PCT/CN2021/099998. In some embodiments, the variable region sequences of the antibody used in the drug conjugate, composition or use of the present invention comprise the variable region sequences of the antibody hu3F8-1 described in PCT/CN2021/099998. In some embodiments, the variable region sequences of the antibody used in the drug conjugate, composition, use or method of the present invention comprise the variable region sequences of the antibody hu3F8-2 described in PCT/CN2021/099998. In some embodiments, the variable region sequences of the antibody used in the drug conjugate, composition or use of the present invention comprise the variable region sequences of the antibody hu3F8-3 described in PCT/CN2021/099998.

The non-limiting and exemplary antibody used in the examples herein is selected from the humanized antibodies hu3F8-1, hu3F8-2 and hu3F8-3 described in PCT/CN2021/099998, the amino acid sequences of which are set forth in the table below.

As used herein, hu3F8-1, hu3F8-2 and hu3F8-3 correspond to clone 1 (clone-1), clone 2 (clone-2) and clone 3 (clone-3) in PCT/CN2021/099998, respectively.

Amino acid sequence of humanized anti-Her3 antibody (KABAT scheme)

| Humanized antibody | hu3F8-1 | hu3F8-2 | hu3F8-3 |
|---|---|---|---|
| HCDR1 | SEQ ID NO:1 | SEQ ID NO:1 | SEQ ID NO:1 |
| HCDR2 | SEQ ID NO:2 | SEQ ID NO:2 | SEQ ID NO:2 |
| HCDR3 | SEQ ID NO:3 | SEQ ID NO:3 | SEQ ID NO:3 |
| LCDR1 | SEQ ID NO:4 | SEQ ID NO:4 | SEQ ID NO:4 |
| LCDR2 | SEQ ID NO:5 | SEQ ID NO:5 | SEQ ID NO:5 |
| LCDR3 | SEQ ID NO:6 | SEQ ID NO:6 | SEQ ID NO:6 |
| VH | SEQ ID NO:7 | SEQ ID NO:9 | SEQ ID NO:10 |
| VL | SEQ ID NO:8 | SEQ ID NO:8 | SEQ ID NO:8 |
| HC | SEQ ID NO:11 | SEQ ID NO:13 | SEQ ID NO:14 |
| LC | SEQ ID NO:12 | SEQ ID NO:12 | SEQ ID NO:12 |

Any suitable method for producing antibodies may be employed to produce the antibody of the present invention. Her3 in any suitable form may be used as an immunogen (antigen) for antibody production. By way of example and not limitation, any Her3 variant or a fragment thereof may be used as an immunogen. In some embodiments, hybridoma cells that produce murine monoclonal anti-human Her3 antibodies can be produced by methods well known in the art. Antibodies derived from rodents (e.g., mouse) may induce unwanted immunogenicity of the antibodies when used as therapeutic agents in vivo. Repeated use of these antibodies induces an immune response in the human body to therapeutic antibodies. Such immune responses result in at least a loss of therapeutic efficacy and, for severe cases, a potentially lethal allergic reaction. One method for reducing the immunogenicity of rodent antibodies includes producing chimeric antibodies, in which the mouse variable region is fused to the human constant region (Liu et al., (1987) Proc. Natl. Acad. Sci. USA 84: 3439-43). However, the preservation of intact rodent variable region in a chimeric antibody may still induce deleterious immunogenicity in patients. Grafting of the complementarity determining region (CDR) loops of the rodent variable domain onto the human framework (i.e., humanization) has been used to further minimize rodent sequences (Jones et al., (1986) Nature 321: 522; Verhoeyen et al., (1988) Science 239: 1534).

In some embodiments, the chimeric or humanized antibodies of the present invention can be prepared based on the sequences of the prepared murine monoclonal hybridoma antibodies. DNA encoding the immunoglobulin heavy and light chains can be obtained from a murine hybridoma of interest and engineered to comprise non-murine (e.g., human) immunoglobulin sequences using standard molecular biology techniques.

In some embodiments, the chimeric Her3 antibodies described herein can be prepared by obtaining the chimeric heavy chains and the chimeric light chains by operably linking the immunoglobulin heavy chain and light chain variable regions of hybridoma origin to human IgG constant regions using methods known in the art (see, e.g., U.S. Pat. No.4,816,567 to Cabilly et al.). In some embodiments, the chimeric antibodies of the present invention comprise constant regions which may be selected from any human IgG subtype, such as IgG1, IgG2, IgG3 and IgG4, preferably IgG4.

In some embodiments, the chimeric Her3 antibodies of the present invention can be obtained by "mixing and matching" a chimeric light chain expression plasmid with a chimeric heavy chain expression plasmid to transfect expression cells. The Her3 binding of such "mixed and matched" antibodies can be tested using the binding assays and other conventional binding assays described above (e.g., ELISA).

For the humanized antibodies described herein, murine CDR regions can be inserted into human germline framework regions using methods known in the art. See U.S. Pat. No. 5,225,539 to Winter et al. and U.S. Pat. Nos. 5,530,101; 5,585,089; 5,693,762 and 6,180,370 to Queen et al.

In some embodiments, the amino acid change includes an amino acid deletion, insertion, or substitution. In some embodiments, the anti-Her3 antibodies or the antigen-binding fragments thereof of the present invention include those antibodies that have been mutated by amino acid deletion, insertion or substitution but still have at least about 90%, 91%, 92%, 93%, 94%, 95%, 96%, 97%, 98%, 99%, or 100% identity to amino acid sequences of the antibodies described above (particularly in the CDR regions set forth in the above sequences). In some embodiments, when compared to the CDR regions set forth in a particular sequence, the antibodies of the present invention have no more than 1, 2, 3, 4, or 5 amino acid mutations (deletions, insertions or substitutions) in the CDR regions. In some embodiments, one or more amino acid modifications may be introduced into an Fc region of the antibody provided herein, thus producing an Fc region variant. The Fc region variant may comprise a human Fc region sequence (such as the human IgG1, IgG2, IgG3, or IgG4 Fc region) comprising an amino acid modification (such as substitution) at one or more amino acid positions. In some embodiments, antibodies modified by cysteine engineering may need to be produced, such as "sulfo-MAb", wherein one or more residues of the antibodies are substituted by cysteine residues. In some embodiments, the antibodies provided herein can be further modified to contain other non-protein moieties known in the art and readily available. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone)polyethylene glycol, propylene glycol homopolymer, polypropylene oxide/ethylene oxide copolymer, polyoxyethylated polyol (such as glycerol), polyvinyl alcohol, and mixtures thereof.

### Drug conjugate

The present application provides an anti-Her3 antibody-drug conjugate, an isomer thereof, a pharmaceutically acceptable salt thereof, or a mixture thereof, which may have one or more effects selected from the group consisting of: (1) having inhibitory activity against in vitro proliferation of tumor cells; (2) having targeting inhibition; (3) having plasma stability; (4) having an in vivo tumor-inhibiting effect; (5) having a bystander effect; (6) having the capacity to inhibit transport by transporters; (7) having the in vivo tumor targeting capability; and (8) having good safety in vivo. The present application provides an anti-Her3 antibody-drug conjugate, an isomer thereof, a pharmaceutically acceptable salt thereof, or a mixture thereof, wherein the anti-Her3 antibody-drug conjugate has a structure of formula (I):

Ab-(L-M-D)ₚ (I)

wherein,
L and M are linker units;
D is a cytotoxic drug;
p represents an average connection number, and p is selected from integers or decimals from 1 to 10, preferably integers or decimals from 3 to 8;
Ab is an anti-Her3 antibody or an antigen-binding fragment thereof, which comprises a heavy chain variable region and a light chain variable region, wherein the heavy chain variable region comprises HCDR1, HCDR2, and HCDR3 of the amino acid sequences set forth in SEQ ID NO: 1, SEQ ID NO: 2, and SEQ ID NO: 3, respectively, and the light chain variable region comprises LCDR1, LCDR2, and LCDR3 of the amino acid sequences set forth in SEQ ID NO: 4, SEQ ID NO: 5, and SEQ ID NO: 6, respectively; preferably, the anti-Her3 antibody or the antigen-binding fragment thereof described herein comprises: (I) a heavy chain variable region of the amino acid sequence set forth in SEQ ID NO: 7 and a light chain variable region of the amino acid sequence set forth in SEQ ID NO: 8; or (II) a heavy chain variable region of the amino acid sequence set forth in SEQ ID NO: 9 and a light chain variable region of the amino acid sequence set forth in SEQ ID NO: 8; or (III) a heavy chain variable region of the amino acid sequence set forth in SEQ ID NO: 10 and a light chain variable region of the amino acid sequence set forth in SEQ ID NO: 8; more preferably, the anti-Her3 antibody or the antigen-binding fragment thereof described herein comprises: (1) a heavy chain of the amino acid sequence set forth in SEQ ID NO: 11 and a light chain of the amino acid sequence set forth in SEQ ID NO: 12; or (2) a heavy chain of the amino acid sequence set forth in SEQ ID NO: 13 and a light chain of the amino acid sequence set forth in SEQ ID NO: 12; or (3) a heavy chain of the amino acid sequence set forth in SEQ ID NO: 14 and a light chain of the amino acid sequence set forth in SEQ ID NO: 12.

In some embodiments, described herein is an anti-Her3 antibody-drug conjugate, the isomer thereof, the pharmaceutically acceptable salt thereof, or the mixture thereof, wherein the anti-Her3 antibody-drug conjugate has a structure of formula (I-1): 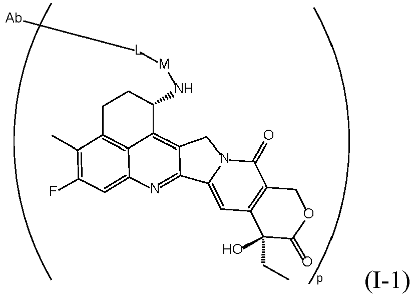 wherein,
L and M are linker units;
p represents an average connection number, and n is selected from integers or decimals from 1 to 10, preferably integers or decimals from 3 to 8;
Ab is an anti-Her3 antibody or an antigen-binding fragment thereof, which comprises a heavy chain variable region and a light chain variable region, wherein the heavy chain variable region comprises HCDR1, HCDR2, and HCDR3 of the amino acid sequences set forth in SEQ ID NO: 1, SEQ ID NO: 2, and SEQ ID NO: 3, respectively, and the light chain variable region comprises LCDR1, LCDR2, and LCDR3 of the amino acid sequences set forth in SEQ ID NO: 4, SEQ ID NO: 5, and SEQ ID NO: 6, respectively; preferably, the anti-Her3 antibody or the antigen-binding fragment thereof described herein comprises: (I) a heavy chain variable region of the amino acid sequence set forth in SEQ ID NO: 7 and a light chain variable region of the amino acid sequence set forth in SEQ ID NO: 8; or (II) a heavy chain variable region of the amino acid sequence set forth in SEQ ID NO: 9 and a light chain variable region of the amino acid sequence set forth in SEQ ID NO: 8; or (III) a heavy chain variable region of the amino acid sequence set forth in SEQ ID NO: 10 and a light chain variable region of the amino acid sequence set forth in SEQ ID NO: 8; more preferably, the anti-Her3 antibody or the antigen-binding fragment thereof described herein comprises: (1) a heavy chain of the amino acid sequence set forth in SEQ ID NO: 11 and a light chain of the amino acid sequence set forth in SEQ ID NO: 12; or (2) a heavy chain of the amino acid sequence set forth in SEQ ID NO: 13 and a light chain of the amino acid sequence set forth in SEQ ID NO: 12; or (3) a heavy chain of the amino acid sequence set forth in SEQ ID NO: 14 and a light chain of the amino acid sequence set forth in SEQ ID NO: 12.

In some embodiments, described herein is an anti-Her3 antibody-drug conjugate, the isomer thereof, the pharmaceutically acceptable salt thereof, or the mixture thereof, wherein
M is -L²-L³-X-L¹-;
L² is -O- or -S-;
L³ is -(C(R^{1a})(R^{1b}))ₘ-, and m is selected from 0, 1, 2, or 3, wherein when L³ comprises a methylene unit, 0 or 1 methylene unit of L³ may be replaced by -C(O)- or -C(=S)-; preferably, L³ is selected from a single bond, -C(R^{1a})(R^{1b})-, or -C(R^{1a})(R^{1b})C(R^{1a})(R^{1b})-; more preferably, L³ is selected from a single bond, -CH₂-, -CH(CH₃), -C(CH₃)₂, -CH₂CH₂-, -CH(CH₃)CH₂-, or -C(CH₃)₂CH₂-;
L¹ is -(C(R^{2a})(R^{2b}))ₙ-, and n is selected from 1, 2, or 3, wherein when L¹ may comprise a methylene unit, 0 or 1 methylene unit of L¹ may be replaced by -C(O)- or -C(=S)-; preferably, L¹ is selected from -C(R^{2a})(R^{2b})-, -C(R^{2a})(R^{2b})C(O)-, or -C(O)-; more preferably, L¹ is selected from -CH₂-, - CH₂C(O)-, -CH(CH₃)C(O)-, or -C(O)-;
X is selected from 3- 6 membered saturated carbocyclyl, 3- 6 membered saturated heterocyclyl, or a single bond, wherein the 3- 6 membered saturated carbocyclyl and the 3- 6 membered saturated heterocyclyl are optionally substituted with 0, 1, 2, or 3 R^{3a}; preferably, X is 3- 6 membered saturated carbocyclyl optionally substituted with 0, 1, 2, or 3 R^{3a} or a single bond; more preferably, X is 3- 6 membered saturated carbocyclyl or a single bond;
wherein each R^{1a}, each R^{1b}, each R^{2a}, each R^{2b}, and each R^{3a} may independently be hydrogen, halogen, or a C₁₋₆ aliphatic group which may be optionally substituted with R,
wherein each R may independently be hydrogen or halogen.

In some embodiments, described herein is an anti-Her3 antibody-drug conjugate, the isomer thereof, the pharmaceutically acceptable salt thereof, or the mixture thereof, wherein -M- is selected from: 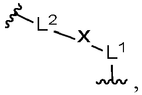 wherein
L² is -O- or -S-; preferably, L² is -O-;
X is selected from 3- 6 membered saturated carbocyclyl optionally substituted with 0, 1, 2, or 3 R^{3a}; preferably, X is selected from cyclopropyl, cyclobutyl, cyclopentyl, or cyclohexyl; more preferably, X is cyclopropyl; more preferably, X is cyclobutyl; more preferably, X is cyclohexyl;
L¹ is selected from -C(R^{2a})(R^{2b})-, -C(R^{2a})(R^{2b})C(O)-, or -C(O)-; preferably, L¹ is -C(O)-,
wherein each R^{2a}, each R^{2b}, or each R^{3a} may independently be hydrogen, halogen, or a C₁₋₆ aliphatic group which may be optionally substituted with R,
wherein each R may independently be hydrogen or halogen.

In some embodiments, described herein is an anti-Her3 antibody-drug conjugate, the isomer thereof, the pharmaceutically acceptable salt thereof, or the mixture thereof, wherein -M- is selected from: 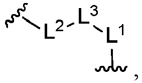 wherein
L² is -O- or -S-; preferably, L² is -O-; preferably, L² is -S-;
L³ is selected from -C(R^{1a})(R^{1b})- or -C(R^{1a})(R^{1b})C(R^{1a})(R^{1b})-; preferably, L³ is selected from -CH₂-, -CH(CH₃), -C(CH₃)₂, -CH₂CH₂-, -CH(CH₃)CH₂-, or -C(CH₃)₂CH₂-; preferably, L³ is selected from CH₂-, -CH(CH₃), and -CH₂CH₂-.
L¹ is selected from -C(R^{2a})(R^{2b})-, -C(R^{2a})(R^{2b})C(O)-, or -C(O)-; preferably, L¹ is -C(O)-;
wherein each R^{1a}, each R^{1b}, each R^{2a}, or each ^{2b} may independently be hydrogen, halogen, or a C₁₋₆ aliphatic group which may be optionally substituted with R,
wherein each R may independently be hydrogen or halogen.

In some preferred embodiments, described herein is an anti-Her3 antibody-drug conjugate, the isomer thereof, the pharmaceutically acceptable salt thereof, or the mixture thereof, wherein -M- is selected from:

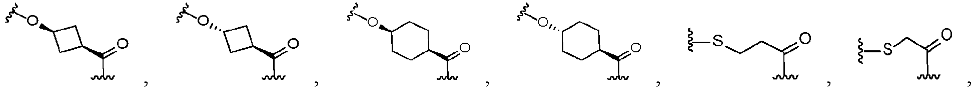

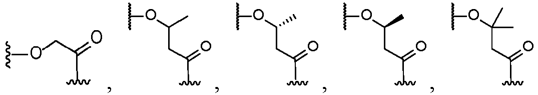

In some embodiments, described herein is an anti-Her3 antibody-drug conjugate, the isomer thereof, the pharmaceutically acceptable salt thereof, or the mixture thereof, wherein L is -Lₐ-L_{b}-L_{c}-, and -Lₐ- is 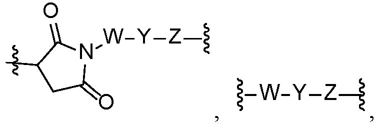
wherein W is -(C(R^{wa})(R^{wb}))_{wn}-, wherein wn is 1, 2, 3 or 6, and 1 methylene unit of W is each independently replaced by -Cyr-, -N(R^{wx})C(O)-, -C(O)N(R^{wx})-, or -C(O)-; preferably, W is - (C(R^{wa})(R^{wb}))₂- or -(C(R^{wa})(R^{wb}))₃-; preferably, W is -CH₂CH₂CH₂- or -CH₂CH₂-;
Y is -(OCH₂CH₂)_{yn}-O_{yp}-, wherein yn is 0, 4 or 8, and yp is 0 or 1; preferably, Y is a single bond;
Z is -(C(R^{za})(R^{zb}))_{zn}, wherein zn is 1, 2 or 3, and 1 methylene unit of Z is each independently replaced by -Cyr-, -N(R^{zx})C(O)-, -C(O)N(R^{zx})-, or -C(O)-, wherein -Cyr- is 3- 10 membered saturated carbocyclylene, wherein -Cyr- is unsubstituted or independently substituted with 1 to 3 substituent R^{cx}; preferably, Z is -(C(R^{wa})(R^{wb}))₂C(O)- or -(C(R^{wa})(R^{wb}))₃C(O)-; preferably, Z is - CH₂CH₂CH₂C(O)- or CH₂CH₂C(O)-;
wherein each R^{wa}, each R^{wb}, each R^{za}, each R^{zb}, each R^{wx}, each R^{zx} and each R^{cx} are independently hydrogen, halogen, -OR^{r}, or a C₁₋₆ aliphatic group optionally substituted with R^{r},
wherein each R^{r} is independently hydrogen, halogen or a C₁₋₆ aliphatic group;
preferably, -Lₐ- is 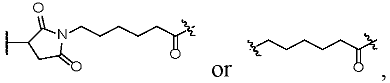 preferably 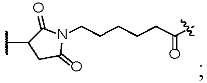
-L_{b}- is selected from the group consisting of: 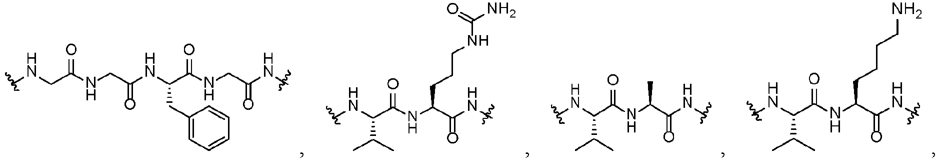 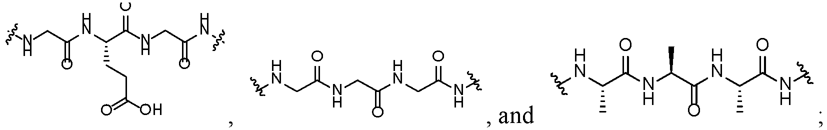 preferably, -L_{b}- is 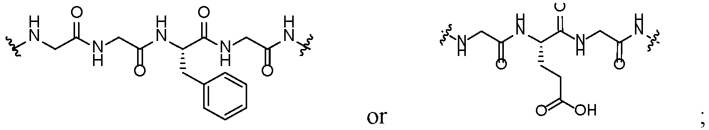 preferably, -L_{b}- is 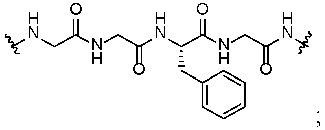
-L_{c}- is 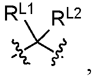 wherein R^{L1} and R^{L2} are each independently selected from the group consisting of: hydrogen, halogen, -OH, and a C₁₋₆ aliphatic group; preferably, -L_{c}- is

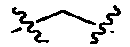

In some particularly preferred embodiments, described herein is an anti-Her3 antibody-drug conjugate, the isomer thereof, the pharmaceutically acceptable salt thereof, or the mixture thereof, wherein L is

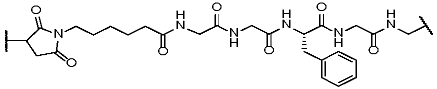

In some embodiments, described herein is an anti-Her3 antibody-drug conjugate, the isomer thereof, the pharmaceutically acceptable salt thereof, or the mixture thereof, wherein the anti-Her3 antibody-drug conjugate has a structure of formula (II-1) or (II-2): 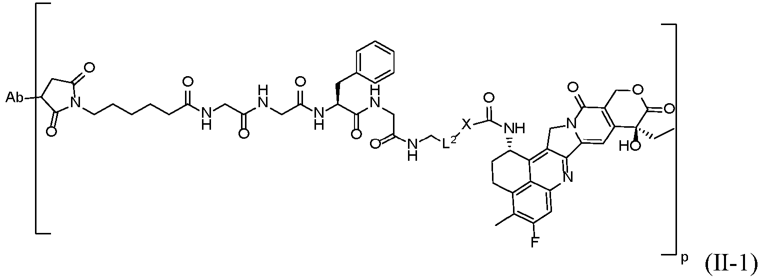 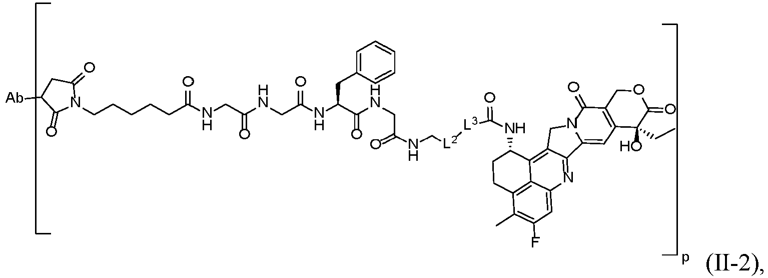 wherein,
L² is -O- or -S-; preferably, L² is -O-; preferably, L² is -S-;
X is selected from 3- 6 membered saturated carbocyclyl optionally substituted with 0, 1, 2, or 3 R^{3a}; preferably, X is selected from cyclopropyl, cyclobutyl, cyclopentyl, or cyclohexyl;
L³ is selected from -C(R^{1a})(R^{1b})- or -C(R^{1a})(R^{1b})C(R^{1a})(R^{1b})-, wherein each R^{1a}, each R^{1b}, or each R^{3a} may independently be hydrogen, halogen, or a C₁₋₆ aliphatic group which may be optionally substituted with R, wherein each R may independently be hydrogen or halogen; preferably, L³ is selected from -CH₂-, -CH(CH₃)-, -C(CH₃)₂-, -CH₂CH₂-, -CH(CH₃)CH₂-, or -C(CH₃)₂CH₂-;
p represents an average connection number, and n is selected from integers or decimals from 1 to 10, preferably integers or decimals from 3 to 8;
Ab is an anti-Her3 antibody or an antigen-binding fragment thereof, which comprises a heavy chain variable region and a light chain variable region, wherein the heavy chain variable region comprises HCDR1, HCDR2, and HCDR3 of the amino acid sequences set forth in SEQ ID NO: 1, SEQ ID NO: 2, and SEQ ID NO: 3, respectively, and the light chain variable region comprises LCDR1, LCDR2, and LCDR3 of the amino acid sequences set forth in SEQ ID NO: 4, SEQ ID NO: 5, and SEQ ID NO: 6, respectively; preferably, the anti-Her3 antibody or the antigen-binding fragment thereof described herein comprises: (I) a heavy chain variable region of the amino acid sequence set forth in SEQ ID NO: 7 and a light chain variable region of the amino acid sequence set forth in SEQ ID NO: 8; or (II) a heavy chain variable region of the amino acid sequence set forth in SEQ ID NO: 9 and a light chain variable region of the amino acid sequence set forth in SEQ ID NO: 8; or (III) a heavy chain variable region of the amino acid sequence set forth in SEQ ID NO: 10 and a light chain variable region of the amino acid sequence set forth in SEQ ID NO: 8; more preferably, the anti-Her3 antibody or the antigen-binding fragment thereof described herein comprises: (1) a heavy chain of the amino acid sequence set forth in SEQ ID NO: 11 and a light chain of the amino acid sequence set forth in SEQ ID NO: 12; or (2) a heavy chain of the amino acid sequence set forth in SEQ ID NO: 13 and a light chain of the amino acid sequence set forth in SEQ ID NO: 12; or (3) a heavy chain of the amino acid sequence set forth in SEQ ID NO: 14 and a light chain of the amino acid sequence set forth in SEQ ID NO: 12.

In some embodiments, the average connection number p described herein may be an integer or a decimal from 2 to 8. For example, the average connection number n may be an integer or a decimal from 3 to 8. For example, the average connection number n may be an integer or a decimal from 1 to 2, 2 to 3, 3 to 4, 4 to 5, 5 to 6, 6 to 7, 7 to 8, 8 to 9, or 9 to 10.

### Pharmaceutical composition and pharmaceutical formulation

In yet another aspect, the present invention provides a pharmaceutical composition, which comprises the anti-Her3 antibody-drug conjugate, the isomer thereof, the pharmaceutically acceptable salt thereof, or the mixture thereof described herein, and a pharmaceutically acceptable carrier or excipient.

It should be understood that the anti-Her3 antibody-drug conjugate or the isomer thereof, the pharmaceutically acceptable salt thereof or the mixture thereof or the pharmaceutical composition thereof provided herein can be integrated into a suitable carrier, an excipient and other reagents in a formulation for administration in combination, thus providing improved transfer, delivery, tolerance, etc.

The term "pharmaceutical composition" refers to a formulation which allows the biological activity of active ingredients contained therein to be present in an effective form and does not contain additional ingredients having toxicity unacceptable to a subject to which the formulation is administered.

The pharmaceutical formulation comprising the anti-Her3 antibody described herein, preferably in the form of an aqueous solution or a lyophilized formulation, may be prepared by mixing the anti-Her3 antibody-drug conjugate or the pharmaceutically acceptable salt thereof of the present invention having the desired purity with one or more optional pharmaceutical adjuvants (Remington's Pharmaceutical Sciences, 16th edition, Osol, A. Ed. (1980)).

The pharmaceutical composition or formulation of the present invention can further comprise one or more additional active ingredients which are required for a specific indication being treated, preferably active ingredients having complementary activities that do not adversely affect one another. In some embodiments, the additional active ingredients are chemotherapeutic agents, immune checkpoint inhibitors, growth inhibitors, antibiotics or various known anti-tumor or anticancer agents, which are suitably present in combination in amounts that are effective for purpose intended. In some embodiments, the pharmaceutical composition of the present invention also comprises a composition of a polynucleotide encoding the anti-Her3 antibody.

In yet another aspect, the present invention provides a pharmaceutical combination, which comprises the antibody-drug conjugate or the pharmaceutically acceptable salt thereof described herein, or the pharmaceutical composition described herein, and one or more additional therapeutic agents.

In yet another aspect, the present invention provides a kit, which comprises the antibody-drug conjugate or the isomer thereof, the pharmaceutically acceptable salt thereof, or the mixture thereof described herein, or the pharmaceutical composition described herein, and preferably further comprises a drug delivery device.

### Medical use

In yet another aspect, the present invention provides use of the antibody-drug conjugate, the isomer thereof, the pharmaceutically acceptable salt thereof, or the mixture thereof described herein, or the pharmaceutical composition described herein in preparing a medicament for treating and/or preventing a Her3-mediated disease or condition, wherein preferably, the disease or condition is cancer.

In yet another aspect, the present invention provides the antibody-drug conjugate, the isomer thereof, the pharmaceutically acceptable salt thereof, or the mixture thereof described herein, or the pharmaceutical composition described herein for treating and/or preventing a Her3-mediated disease or condition, wherein preferably, the disease or condition is cancer.

In yet another aspect, the present invention provides a method for treating and/or preventing a Her3-mediated disease or condition, which comprises administering to a subject in need the antibody-drug conjugate, the isomer thereof, the pharmaceutically acceptable salt thereof, or the mixture thereof described herein, or the pharmaceutical composition described herein, wherein preferably, the disease or condition is cancer.

In some embodiments, the cancer is selected from lung cancer, kidney cancer, urinary tract carcinoma, colorectal cancer, prostate cancer, glioblastoma multiforme, ovarian cancer, pancreatic cancer, breast cancer, melanoma, liver cancer, bladder cancer, gastric cancer and esophageal cancer.

In some embodiments, routes of administration for the present invention include, but are not limited to, oral administration, intravenous administration, subcutaneous administration, intramuscular administration, intra-arterial administration, intra-articular administration (e.g., in arthritic joints), inhalation, aerosol delivery, intratumoral administration, etc.

In some embodiments, the present invention provides one or more therapies (e.g., treatment modalities and/or other therapeutic agents) co-administrating to a subject a therapeutically effective amount. In some embodiments, the therapies include surgical treatment and/or radiation therapy.

In some embodiments, the method or use provided herein further comprises administering to the individual one or more therapies (e.g., treatment modalities and/or other therapeutic agents). The antibody-drug conjugate or the pharmaceutically acceptable salt thereof of the present invention may be used alone or in combination with other therapeutic agents in a therapy. For example, it may be co-administered with at least one additional therapeutic agent.

The drug conjugate described herein may have inhibitory activity against in vitro proliferation of tumor cells. The inhibitory activity may be that: adding the drug conjugate disclosed herein to a medium with tumor cells leads to a decrease of no less than 1%, no less than 2%, no less than 4%, no less than 5%, no less than 8%, no less than 10%, no less than 15%, no less than 18%, no less than 20%, no less than 25%, no less than 40%, no less than 50%, no less than 60%, no less than 70%, no less than 80%, no less than 90%, or no less than 95%, in the proliferation capacity of the tumor cells, compared with adding a negative control or a reference drug. For example, the inhibitory activity may be that: an IC50 value (nM) for tumor cells may be no more than 10000, no more than 5000, no more than 4000, no more than 3000, no more than 2000, no more than 1000, no more than 500, no more than 400, no more than 300, no more than 200, no more than 150, no more than 120, no more than 110, no more than 100, no more than 99, no more than 98, no more than 97, no more than 95, no more than 90, no more than 80, no more than 75, no more than 70, no more than 65, no more than 62, no more than 60, no more than 50, no more than 40, no more than 30, no more than 25, no more than 23, no more than 22, no more than 20, no more than 19, no more than 18, no more than 18.5, no more than 17, no more than 15, no more than 12, no more than 10, no more than 9, no more than 8.5, no more than 7, no more than 6.7, no more than 6, no more than 5.9, no more than 5.5, no more than 5.0, no more than 4.8, no more than 4.5, no more than 4.4, no more than 4, no more than 3.5, no more than 3, no more than 2.5, no more than 2, no more than 1.5, no more than 1.0, no more than 0.5, no more than 0.3, no more than 0.29, no more than 0.25, no more than 0.21, no more than 0.20, no more than 0.18, no more than 0.17, no more than 0.15, no more than 0.12, no more than 0.10, no more than 0.09, no more than 0.08, no more than 0.07, no more than 0.06, no more than 0.05, no more than 0.04, no more than 0.03, no more than 0.02, or no more than 0.01. For example, the tumor cells may include, but are not limited to, solid tumor cells; for example, the tumor cells include, but are not limited to, gastric cancer cells or breast cancer cells; for example, the tumor cells may include, but are not limited to, HCC1569 cells or MDA-MB-453 cells.

The drug conjugate described herein may have targeting inhibition. The targeting inhibition may be that: compared with in a medium of tumor cells with high expression of a specific target to which a negative control or a reference drug is added, the proliferation capacity of the tumor cells with high expression of a specific target is reduced by no less than 1%, no less than 2%, no less than 4%, no less than 5%, no less than 8%, no less than 10%, no less than 15%, no less than 18%, no less than 20%, no less than 25%, no less than 40%, no less than 50%, no less than 60%, no less than 70%, no less than 80%, no less than 90%, or no less than 95% in a medium to which the drug conjugate disclosed herein is added. For example, the targeting inhibition may be that: an IC50 value (nM) for tumor cells with high expression of a specific target may be no more than 10000, no more than 5000, no more than 4000, no more than 3000, no more than 2000, no more than 1000, no more than 500, no more than 400, no more than 300, no more than 200, no more than 185, no more than 150, no more than 120, no more than 110, no more than 100, no more than 99, no more than 98, no more than 97, no more than 95, no more than 91, no more than 80, no more than 74, no more than 70, no more than 65, no more than 62, no more than 60, no more than 50, no more than 40, no more than 30, no more than 25, no more than 23, no more than 22, no more than 20, no more than 19, no more than 18, no more than 18.5, no more than 17, no more than 15, no more than 12, no more than 10, no more than 9, no more than 8.5, no more than 7, no more than 6.7, no more than 6, no more than 5.9, no more than 5.5, no more than 5.0, no more than 4.8, no more than 4.5, no more than 4.4, no more than 4, no more than 3.5, no more than 3, no more than 2.5, no more than 2, no more than 1.5, no more than 1.0, no more than 0.5, no more than 0.3, no more than 0.29, no more than 0.25, no more than 0.21, no more than 0.20, no more than 0.18, no more than 0.17, no more than 0.15, no more than 0.12, no more than 0.10, no more than 0.09, no more than 0.08, no more than 0.07, no more than 0.06, no more than 0.05, no more than 0.04, no more than 0.03, no more than 0.02, or no more than 0.01. For example, the tumor cells with high expression of a specific target may include, but are not limited to, solid tumor cells; for example, the tumor cells with high expression of a specific target include, but are not limited to, gastric cancer cells or breast cancer cells; for example, the tumor cells with high expression of a specific target may include, but are not limited to, HCC1569 cells or MDA-MB-453 cells. The specific target may include, but is not limited to, Her3.

The drug conjugate described herein may have plasma stability. The plasma stability may be that: the drug conjugate disclosed herein releases no more than 50%, no more than 40%, no more than 30%, no more than 20%, no more than 10%, no more than 7%, no more than 5%, no more than 4%, no more than 3%, no more than 2%, no more than 1.9%, no more than 1.8%, no more than 1.7%, no more than 1.6%, no more than 1.5%, no more than 1.4%, no more than 1.3%, no more than 1.2%, no more than 1.1%, no more than 1.0%, no more than 0.9%, no more than 0.8%, no more than 0.7%, no more than 0.6%, no more than 0.5%, no more than 0.4%, no more than 0.3%, no more than 0.2% or no more than 0.1% of the cytotoxic drug 1 day, 3 days, 5 days, 7 days, 14 days, 20 days or 30 days after the drug conjugate is added to plasma.

The drug conjugate described herein may have an in vivo tumor-inhibiting effect. The tumor-inhibiting effect may be that: compared with the case where a negative control or a reference drug is administered to an animal, the tumor of the animal is reduced in volume by no less than 1%, no less than 2%, no less than 4%, no less than 5%, no less than 8%, no less than 10%, no less than 15%, no less than 18%, no less than 20%, no less than 25%, no less than 40%, no less than 50%, no less than 55%, no less than 60%, no less than 70%, no less than 73%, no less than 75%, no less than 80%, no less than 90% or no less than 95% 1 day, 3 days, 5 days, 7 days, 14 days, 20 days, 21 days or 30 days after the drug conjugate disclosed herein is administered, or the tumor of the animal is reduced in volume by no less than 1.1 fold, no less than 1.3 fold, no less than 1.5 fold, no less than 2 fold, no less than 3 fold, no less than 5 fold, no less than 10 fold, no less than 20 fold, no less than 22 fold, no less than 30 fold, no less than 50 fold, no less than 100 fold, no less than 500 fold, no less than 1000 fold or no less than 1500 fold 1 day, 3 days, 5 days, 7 days, 14 days, 20 days, 21 days or 30 days after the drug conjugate disclosed herein is administered. The animal may include, but is not limited to, a mammal. For example, the animal may include, but is not limited to, a cat, a dog, a horse, a pig, a cow, a sheep, a rabbit, a mouse, a rat, a monkey or a human. The administration may include, but is not limited to, oral administration, intravenous injection, intravenous drip, intraperitoneal injection or topical administration.

The drug conjugate described herein may have a bystander effect. The bystander effect may be that: the drug conjugate disclosed herein has no obvious inhibiting effect against cell proliferation of the tumor cells with low expression of a specific target, but in the co-culturing of the tumor cells with low expression of the specific target and the tumor cells with high expression of the specific target, the drug conjugate disclosed herein can simultaneously inhibit the cell proliferation of the tumor cells with low expression of the specific target and the tumor cells with high expression of the specific target. For example, in the co-culturing of the tumor cells with low expression of the specific target and the tumor cells with high expression of the specific target, the inhibitory activity may be that: an IC50 value (nM) for the tumor cells with low expression of the specific target may be no more than 10000, no more than 5000, no more than 4000, no more than 3000, no more than 2000, no more than 1000, no more than 500, no more than 400, no more than 300, no more than 200, no more than 185, no more than 150, no more than 120, no more than 110, no more than 100, no more than 99, no more than 98, no more than 97, no more than 95, no more than 91, no more than 80, no more than 74, no more than 70, no more than 65, no more than 62, no more than 60, no more than 50, no more than 40, no more than 30, no more than 25, no more than 23, no more than 22, no more than 20, no more than 19, no more than 18, no more than 18.5, no more than 17, no more than 15, no more than 12, no more than 10, no more than 9, no more than 8.5, no more than 7, no more than 6.7, no more than 6, no more than 5.9, no more than 5.5, no more than 5.0, no more than 4.8, no more than 4.5, no more than 4.4, no more than 4, no more than 3.5, no more than 3, no more than 2.5, no more than 2, no more than 1.5, no more than 1.0, no more than 0.5, no more than 0.3, no more than 0.29, no more than 0.25, no more than 0.21, no more than 0.20, no more than 0.18, no more than 0.17, no more than 0.15, no more than 0.12, no more than 0.10, no more than 0.09, no more than 0.08, no more than 0.07, no more than 0.06, no more than 0.05, no more than 0.04, no more than 0.03, no more than 0.02, or no more than 0.01. Compared with in tumor cells with high expression of the specific target, the expression of the specific target in tumor cells with low expression of the specific target may be reduced by no less than 1%, no less than 2%, no less than 4%, no less than 5%, no less than 8%, no less than 10%, no less than 15%, no less than 18%, no less than 20%, no less than 25%, no less than 40%, no less than 50%, no less than 60%, no less than 70%, no less than 80%, no less than 90% or no less than 95%. For example, the tumor cells with high expression of a specific target may include, but are not limited to, solid tumor cells; for example, the tumor cells with high expression of a specific target include, but are not limited to, gastric cancer cells or breast cancer cells; for example, the tumor cells with high expression of a specific target may include, but are not limited to, HCC1569 cells or MDA-MB-453 cells. For example, the tumor cells with low expression of a specific target may include, but are not limited to, solid tumor cells; for example, the tumor cells with low expression of a specific target include, but are not limited to, breast cancer cells; for example, the tumor cells with low expression of a specific target may include, but are not limited to, HCC1187 cells.

The drug conjugate described herein may have the capacity to inhibit transport by transporters. The capacity in inhibiting transport may be a reduction in the efflux ratio of the drug conjugate described herein by no less than 1%, no less than 2%, no less than 4%, no less than 5%, no less than 8%, no less than 10%, no less than 15%, no less than 18%, no less than 20%, no less than 25%, no less than 40%, no less than 50%, no less than 60%, no less than 70%, no less than 80%, no less than 90% or no less than 95% compared with a standard of a transport substrate. For example, the testing of the efflux ratio may be a method commonly used by those skilled in the art, or may be described in the examples of the present application.

The drug conjugate described herein may have in vivo tumor targeting capability. The in vivo targeting ability may be that: when the drug conjugate labeled with a signal substance is administered to an animal, compared with in other tissues and organs of the animal, the distribution of the labeled drug conjugate in a tumor tissue may be increased by no less than 1%, no less than 2%, no less than 4%, no less than 5%, no less than 8%, no less than 10%, no less than 15%, no less than 18%, no less than 20%, no less than 25%, no less than 40%, no less than 50%, no less than 60%, no less than 70%, no less than 80%, no less than 90% or no less than 95%, or may be increased by no less than 1.1 fold, no less than 1.3 fold, no less than 1.5 fold, no less than 2 fold, no less than 3 fold, no less than 5 fold, no less than 10 fold, no less than 20 fold, no less than 22 fold, no less than 30 fold, no less than 50 fold, no less than 100 fold, no less than 500 fold, no less than 1000 fold or no less than 1500 fold. The signal substance may be a radioactive material; for example, the signal substance includes, but is not limited to, 125I. The animal may include, but is not limited to, a mammal. For example, the animal may include, but is not limited to, a cat, a dog, a horse, a pig, a cow, a sheep, a rabbit, a mouse, a rat, a monkey or a human. The administration may include, but is not limited to, oral administration, intravenous injection, intravenous drip, intraperitoneal injection or topical administration. The tissues or organs may include, but are not limited to, heart, liver, spleen, lung, kidney, brain or bone marrow. The drug conjugate described herein may have good in vivo safety. The in vivo safety may be that: after the drug conjugate disclosed herein is administered to an animal, the release rate of in vivo free toxin in the animal is no more than 50%, no more than 40%, no more than 30%, no more than 20%, no more than 10%, no more than 7%, no more than 5%, no more than 4%, no more than 3%, no more than 2%, no more than 1.9%, no more than 1.8%, no more than 1.7%, no more than 1.6%, no more than 1.5%, no more than 1.4%, no more than 1.3%, no more than 1.2%, no more than 1.1%, no more than 1.0%, no more than 0.9%, no more than 0.8%, no more than 0.7%, no more than 0.6%, no more than 0.5%, no more than 0.4%, no more than 0.3%, no more than 0.2%, or no more than 0.1%. For example, the in vivo safety may be that: the drug conjugate described herein may be administered at a concentration of no less than 0.5 mg/kg, no less than 1 mg/kg, no less than 2 mg/kg, no less than 3 mg/kg, no less than 4 mg/kg, no less than 5 mg/kg, no less than 10 mg/kg, no less than 20 mg/kg, no less than 30 mg/kg, no less than 50 mg/kg, no less than 70 mg/kg, no less than 100 mg/kg, no less than 200 mg/kg, no less than 500 mg/kg or no less than 1000 mg/kg without causing toxic manifestation in the animal. For example, the animal may include, but is not limited to, a cat, a dog, a horse, a pig, a cow, a sheep, a rabbit, a mouse, a rat, a monkey or a human. The administration may include, but is not limited to, oral administration, intravenous injection, intravenous drip, intraperitoneal injection or topical administration.

### Sample assay

### 1. ADC DAR value analysis method - Hydrophobic Interaction Chromatography (HIC-HPLC)

High performance liquid chromatograph: Waters E2965 high performance liquid chromatography system.

Chromatography column: MabPac^{™} HIC-Butyl 5 µm 4.6 × 100 mm (manufacturer: Thermo).

Mobile phase A: 1.5 M (NH4)₂SO₄ + 50 mM potassium phosphate (pH 7.0).

Mobile phase B: 50 mM sodium phosphate (pH 7.0)/isopropanol (75:25 V/V).

Elution was carried out according to the following elution procedure.

| Time | Mobile phase B |
|---|---|
| 0-2 min | 0%-10% |
| 2-22 min | 10%-65% |
| 22-24 min | 65%-100% |
| 24-26 min | 100%-0% |
| 26-30 min | 0% |

Detection conditions: the flow rate of the mobile phase is set at 1 mL/min, the detection wavelength at 280 nm, and the column temperature at 30 °C.

### 2. SEC purity analysis - Size Exclusion Chromatography (SEC-HPLC)

High performance liquid chromatograph: Agilent 1260 liquid chromatograph.

Chromatography column: Waters Xbridge BEH200 SEC (7.8 × 300 mm, 3.5 µm)

Mobile phase: 50 mM potassium phosphate + 200 mM arginine (pH 6.80) + 10% isopropanol

| Time | Mobile phase |
|---|---|
| 0-30 min | 100% |

Detection conditions: the flow rate of the mobile phase is set at 0.5 mL/min, the detection wavelength at 280 nm, and the column temperature at 30 °C.

The present invention includes any combinations of the specific embodiments described. Further embodiments of the present invention and the full scope of applicability will become apparent from the detailed description provided below. However, it should be understood that the detailed description and the specific examples, while indicating preferred embodiments of the present invention, are provided by way of illustration only, as various changes and modifications within the spirit and scope of the present invention will become apparent to those skilled in the art from the detailed description. All publications, patents and patent applications cited herein, including the citations, are hereby incorporated by reference in their entirety for all purposes.

### Examples

The following examples are provided to demonstrate and further illustrate some preferred embodiments and aspects of the present invention and should not be construed as limiting the scope of the present invention.

### Example 1. Anti-Her3 Antibody

**Materials and methods:** the following reagents were purchased from Southern Biotech and used at the specified dilution: goat anti-mouse IgG-HRP (1030-05, 1:5000 dilution), goat anti-human IgG-PE (2040-09, 1:1000 dilution), goat anti-human Kappa IgG-HRP, (2061-05, 1:20000 dilution), goat anti-rabbit IgG-HRP (4030-05, 1:5000 dilution), FITC-labeled streptavidin (7100-02, 1:500 dilution), mouse anti-human Kappa-APC (9230-11, 1:500 dilution), and mouse IgG APC (0107-11, 0.1 mg/mL). NRG1 was from Origene (TP723155). Cell culture medium Roswell Park Memorial Institute (RPMI) 1640, Dulbecco's modified medium (DMEM) and fetal bovine serum were from Hyclone. The recombinant patritumab was prepared in house. The HER3 antibody 3F8 was produced by hybridoma or recombinantly. m3F8 represents murine 3F8 antibody, while ch3F8 and hu3F8 represent chimeric and humanized 3F8 antibodies, respectively.

**Cell culture:** SP2/0, SP2/0-HER3, SP2/0-HER2, SP2/O-EGFR, NCI-N87, MDA-MB-468, MDA-MB-453, 7901, HT29, MCF-7, and SK-BR-3 used in the study were purchased from ATCC and cultured using the appropriate media recommended by ATCC. SP2/0-EGFR, SP2/0-HER2, and SP2/0HER3 cells stably expressing human EGFR, HER2 and HER3 were generated in house.

**Preparation of anti-HER3 hybridoma:** on the first day, BALB/c mice (female, 8-10 weeks old) were intraperitoneally injected with 1-2 × 10⁶ SP2/0-HER3 cells expressing human HER3, together with Freund's complete adjuvant. On day 8, an enhanced immunization was performed using the same number of the cells with Freund's incomplete adjuvant. From day 14, the mice were immunized with the above amount of the cells every three days, and the procedure was repeated three times. Three days after the last immunization, B lymphocytes were isolated from the spleens and fused with the immortal myeloma cells NS-1 to generate hybridoma cells.

The hybridoma cells were cultured in a 96-well plate at gradient dilutions. The supernatant was collected and screened for antibodies recognizing HER3 expressed on the surfaces of SP2/0 cells by flow cytometry or screened for recombinant HER3 antibodies by ELISA.

**DNA cloning and sequencing of antibody variable region:** after reverse transcription of the total RNA extracted from the hybridoma using Trizol (ThermoFisher) to the first cDNA strand, 5' complementary DNA (5' RACE) was rapidly amplified using PCR according to the instructions of the 5' RACE kit (Invitrogen, 18374-058) to amplify the DNA sequence encoding the variable region. The PCR product was cloned into a pGM-T vector. The positive clones were subjected to DNA sequencing, from which the protein sequence was inferred. The amino acids of the variable region were analyzed in the Kabat numbering scheme.

**Antibody expression:** the DNA encoding the antibody heavy and light chains was cloned into the expression vector pCDNA3.1(+) (Invitrogen) and expressed in 293T cells. The antibodies were purified using a protein A or G column (GE).

**Humanization:** humanization was done by GenScript. First, a murine-human chimeric antibody (ch3F8) was produced by replacing the constant region of the heavy chain of a murine antibody with the constant region sequence of human IgG1 and replacing the constant region of the light chain of the murine antibody with the constant region sequence of human Igκ. Humanization was then performed on the chimeric antibody according to the reference (Kuramochi et al.). The mouse antibody framework residue necessary for affinity and specificity was retained and maintained while replacing the mouse framework with a human germline framework to produce a humanized antibody. A codon-optimized DNA sequence encoding the humanized antibody was synthesized by GenScript. **Antibody expression and purification:** after plasmids encoding the above antibody DNA sequence were transfected into ExpiCHO-S (Catl.# A2910001, Gibco) cells, and the cells were grown in a medium at 32 °C with 5% CO₂ and maintained for 12 days. The supernatant was collected after centrifugation at 4000 g for 30 min and filtered through a 0.22 µm membrane. Antibodies binding to protein A (Catl.# 17508001, GE) were washed with 20 mM sodium phosphate (pH 7.0) and eluted with 0.1 M glycine (pH 3.0) as recommended by the manufacturer's manual. The eluted fraction was neutralized with 0.1 M Tris buffer (pH 9.0) and then switched to PBS buffer by ultrafiltration centrifugation. The protein concentration was determined by BCA.

**Affinity assay by surface plasmon resonance (SPR):** antibody kinetics and affinity were determined using Biacore T200. Briefly, the recombinant human HER3 antibody was immobilized on a protein A chip (GE, Cat. 29-1275-5). The antigen concentration, from 50 nM to a final concentration of 0.78125 nM, was generated by 2-fold serial dilution, and affinity and kinetics were determined through the chip.

**FACS assay:** the cultured cells were digested with 0.25% trypsin-EDTA and then centrifuged at 1500 rpm for 5 min. The cell pellets were regenerated into 5 × 10⁶ cells/mL using a PBS FACS solution containing 5% FBS and 0.2% ProClin300. 50 µL of the cell suspension was incubated with 100 µL of the primary antibody at a concentration of 1 µg/mL on ice for 1 h. The cells were washed twice with the FACS solution. The pellets were regenerated using 100 µL of a FACS solution containing goat anti-mouse IgG-PE (1:1000 dilution) and incubated on ice in the dark for 1 h. The cells were then washed twice and resuspended in 200 µL of the FACS solution.

**Western blot assay:** the protein separated by SDS-PAGE was transferred to a nitrocellulose membrane for Western blot. The primary antibodies used were as follows: anti-HER2 (cell signaling, Catl#: 2165S), anti-HER3 (cell signaling, Catl#: 12708), anti-p-HER3 (cell signaling, Catl#: 4791), and anti-β-actin (cell signal transduction, Catl#: 4967).

**ELISA assay:** human HER2-ex-huFc, human HER3-huFc, and human EGFR-his were diluted to 2 µg/mL and 50 µL/well in a 96-well plate and incubated at 4 °C overnight. The plate was washed with 0.5× PBST, then incubated with 100 µL of a blocking buffer (PBS + 3% BSA) at 37 °C for 2 h, and washed with 0.5× PBST. The 3F8 antibody was diluted with the blocking buffer in a 1:3 serial gradient, added at 50 µL/well, incubated at 37 °C for 40-50 min, and then washed with 0.5× PBST. Goat anti-mouse IgG-HRP (Southern Biotech, 1030-05) was diluted at 1:20000 with the blocking buffer, added at 50 µL/well, incubated in the dark for 30 min, and then washed with 0.5× PBST. Prior to assay, 50 µL of luminol buffer A + B was added to each well in a 1:1 mix.

**HER3 phosphorylation induced by NRG1:** the cells were cultured in a 6-well plate and used for an experiment when the confluency reached 80%. On the day of the experiment, the cells were washed twice with PBS, incubated for 6 h in a serum-free medium, and then induced overnight with 10 µg/mL of the antibody. To induce HER3 phosphorylation, NRG1 was added to a working concentration of 100 ng/mL 30 min before the cells were collected for Western blot.

**Antibody stability test:** the purified antibody at 5 mg/mL was stored at 4 °C as regular practice, incubated at 40 °C for 7 days and 14 days for heat stability evaluation, transferred to a glycine solution at pH 3.5 for regeneration, and stored for 2, 4 and 6 h for acid stability evaluation, or subjected to repeated freeze-thaw treatments for 4 or 6 cycles for freeze-thaw stability evaluation. Aggregation was measured by SEC-HPLC, and binding affinity was determined by ELISA.

**Antibody [⁸⁹Zr]Zr labeling:** DFO-NCS was conjugated to the antibody as shown in the reference (Zeglis and Lewis, 2015). DFO and the antibody were mixed at a molar ratio of 5:1 and incubated at 37 °C for 1 h. The DFO-conjugated antibody was purified by SEC-HPLC.

A ⁸⁹Zr oxalate solution (0.8mCi) was mixed with the DFO-conjugated antibody (0.2 mg/mL) in HEPES/Na₂CO₂ buffer (pH 7.0-7.5) and incubated at room temperature for 30 min. Radiochemical purity was assessed by TLC. The Rf of the [⁸⁹Zr]Zr antibody was 0-0.3, while the Rf of free ⁸⁹Zr was 0.6-1.0.

**PET imaging:** each animal was injected intravenously with approximately 100 µCi of the [⁸⁹Zr]Zr antibody. At the indicated time after injection, images were collected and analyzed using a small-animal PET scanner.

**In vivo efficacy study:** the animals were maintained and used according to the IACUC guidelines. BALC/b nude mice and NPG mice, purchased from Charles River and SPF Biotech, respectively, were housed at 25 °C in a 12-h dark/light cycle with free access to food and drink. A patient-derived xenograft (PDX) model was generated by subcutaneous implantation of cryopreserved tissue fragments. Animals were initiated with drug treat when the tumor size reached 100-200 mm³ and were euthanized when the tumor size was 1000 mm³. The health condition was monitored daily. Tumor size and body weight were monitored and recorded every 3 days.

Software: data were analyzed using Olinda, GraphPad Prism 6.0, or EXCEL.

### Example 1.1

Murine 3F8 was co-incubated with SP2/0 wild-type cells or cells overexpressing HER3, HER2 or EGFR. The binding strength was measured in a FACS instrument with a PE-anti-mouse secondary antibody.

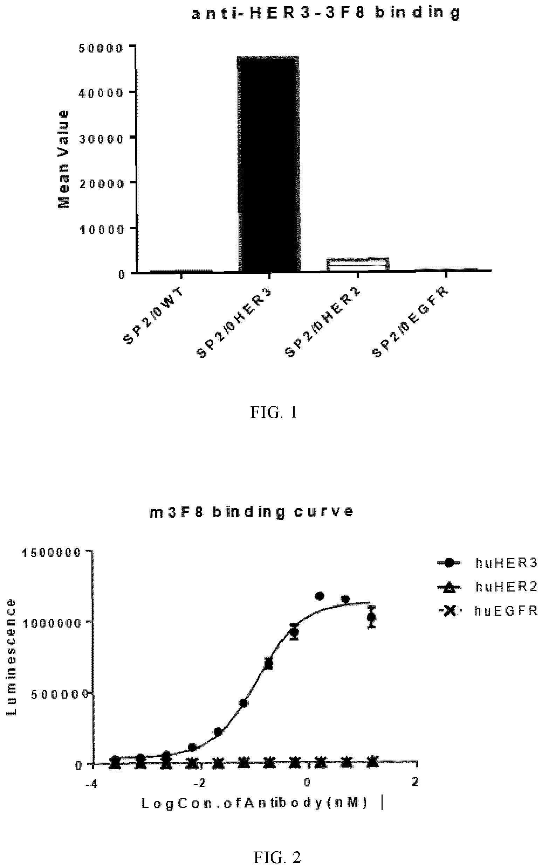

The results are shown in FIG. 1. 3F8 specifically bound to SP2/0-HER3 cells, but did not bind to other cells.

### Example 1.2

The binding affinities of murine 3F8 to human HER3, HER2 and EGFR were determined by ELISA. The results are shown in FIG. 2. Murine 3F8 only recognized HER3 and did not recognize HER2 or EGFR.

### Example 1.3

EC50 of murine 3F8 binding to human HER3, HER2, and EGFR was analyzed using GraphPad Prism 6.0. The results are shown in Table 1. Murine 3F8 showed strong binding affinity in the sub-nanomolar range.

**Table 1. EC50 of murine 3F8 binding to human HER3, HER2, or EGFR**

| 3F8 | huHER3 | huHER2 | huEGFR |
|---|---|---|---|
| EC50 (nM) | 0.11 | NA | NA |
| EC50 (ng/mL) | 16.50 | NA | NA |
| Hill Slope | 1.04 | NA | NA |
| R square | 0.99 | NA | NA |

### Example 1.4

The species selectivity of murine 3F8 to human, monkey, rat and mouse HER3 was determined by ELISA.

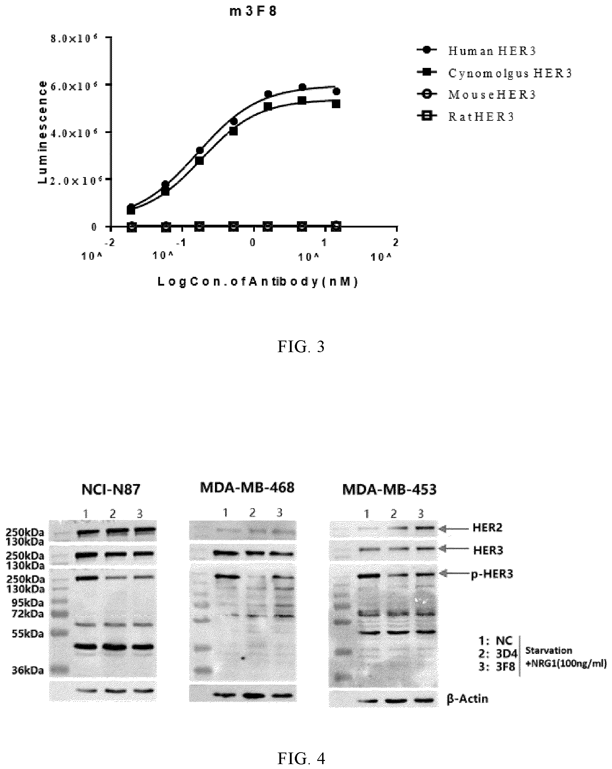

The results are shown in FIG. 3. Murine 3F8 recognized human and monkey HER3 with similar potency, but did not recognize mouse and rat HER3.

### Example 1.5

EC50 of murine 3F8 binding to human, monkey, rat and mouse HER3 was analyzed using GraphPad Prism 6.0. The results are shown in Table 2. Murine 3F8 showed the same binding affinity to human and cynomolgus monkey HER3 in the sub-nanomolar range.

**Table 2. EC50 of murine 3F8 binding to human, cynomolgus monkey, rat or mouse HER3**

| | Human HER3 | Cynomolgus monkey HER3 | Mouse HER3 | Rat HER3 |
|---|---|---|---|---|
| Hill Slope | 1.015 | 1.102 | NA | NA |
| EC₅₀ (nM) | 0.1635 | 0.1855 | NA | NA |
| EC₅₀ (ng/mL) | 24.525 | 27.825 | NA | NA |
| R square | 0.9957 | 0.9969 | NA | NA |

### Example 1.6

Murine 3F8 blocked NRG1-induced HER3 phosphorylation. NCI-N87, MDA-MB-468 and MDA-MB-453 were treated with NRG1, the HER3 ligand, to induce downstream phosphorylation of HER3. The effect of murine 3F8 in inhibiting NRG1-induced p-HER3 was determined by western blot. 3D4 is a previously demonstrated anti-HER3 antibody that competes with NRG1 for binding to HER3, and applied herein as a positive control.

The results are shown in FIG. 4. The data shows that murine 3F8 reduced the phosphorylation level of HER3 protein, but had no effect on the total HER3 protein level. Western blot of HER2 also shows that 3F8 had no effect on the HER2 protein level.

### Example 1.7

Murine 3F8 was rapidly taken up by cells at different HER3 surface levels. The cells at different HER3 surface levels were incubated with murine 3F8 on ice (control) or incubated at 37 °C for 1 or 4 h. The internalization fraction was determined by subtracting the 37 °C incubated cell surface signal from the ice incubated control.

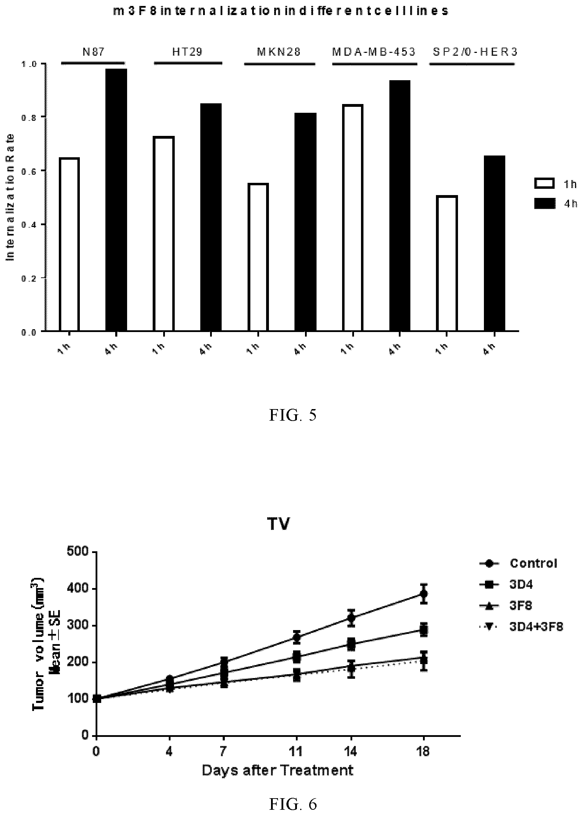

The results are shown in FIG. 5. The data indicates that murine 3F8 was rapidly internalized into the cell. Most were taken up within 1 h, and with the incubation time extended to 4 h, the number of intracellular fragments increased slightly, indicating that 3F8 internalization was a rapid and continuous process.

### Example 1.8

The anti-HER3 antibody was effective in inhibiting tumor growth in the BT474 subcutaneous xenograft model. The anti-HER3 antibody 3F8, 3D4, or 3F8 + 3D4 combination was injected intravenously at 25 mg/kg once every two weeks for three weeks. Tumor size was monitored every 3-4 days. Both 3F8 and 3D4 are anti-HER3 antibodies. 3F8 is a murine antibody.

The results are shown in FIG. 6. Both 3F8 and 3D4 significantly inhibited the tumor growth (one-way analysis of variance, p < 0.05), and 3F8 showed better efficacy. The combination of 3F8 and 3D4 was comparable to 3F8 alone in efficacy.

### Example 1.9

[⁸⁹Zr]Zr-ch3F8 was used to image the gastric PDX model GAS078. [⁸⁹Zr]Zr-ch3F8 was injected intravenously into the gastric model GAS078. Images were collected at 4, 24, 48, 72, 96 and 168 h after injection. The radioactive uptake of each organ was analyzed by Olinda and expressed as % ID/g (percent injection dose/gram of tissue).

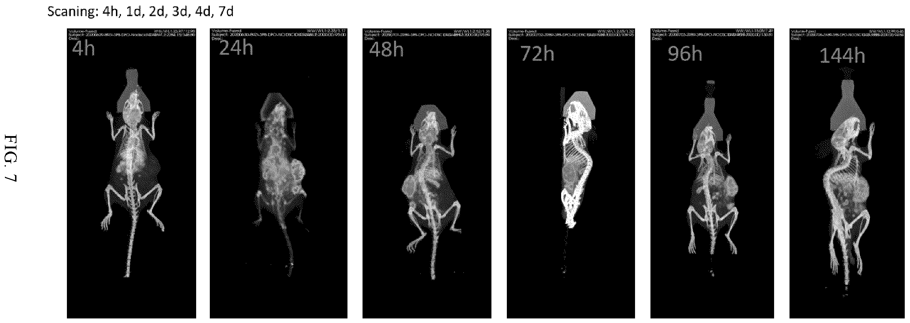

FIG. 7 shows representative images of [⁸⁹Zr]Zr-ch3F8 in the GAS078 model. Ch3F8 represents the chimeric 3F8 antibody. The data shows a gradual increase in [⁸⁹Zr]Zr-ch3F8 uptake in the tumor over time. Tumor uptake remained stable at 96 h after injection, followed by a slight decrease at 168 h after injection.

The data are also shown in Table 3. The uptake amounts of [⁸⁹Zr]Zr-ch3F8 in the tumors and the major organs of heart, liver, kidney and spleen were evaluated as % ID/g (% injection dose/g of tissue).

**Table 3. Radioactive uptake by tumor and major organ**

| %ID/g | | Heart | Liver | Kidney | Spleen | Tumor |
|---|---|---|---|---|---|---|
| 4h | Mouse 1 | 23.03 | 20.37 | 11.64 | 15.28 | 5.26 |
| | Mouse 2 | 14.75 | 14.97 | 8.77 | | 5.38 |
| 24h | Mouse 1 | 12.39 | 10.59 | 7.94 | 7.84 | 10.33 |
| | Mouse 2 | 8.66 | 8.41 | 6.41 | 9.74 | 10.31 |
| 48h | Mouse 1 | 6.02 | 8.36 | 6.72 | 10.69 | 12.15 |
| | Mouse 2 | 3.44 | 6.77 | 6.31 | 5.15 | 11.33 |
| 72h | Mouse 1 | 3 | 8.28 | 3.51 | 12.13 | 12.67 |
| | Mouse 2 | 2.19 | 7.09 | 4.19 | 12.64 | 10.86 |
| 96h | Mouse 1 | 1.61 | 6.94 | 3.42 | 11.49 | 12.07 |
| | Mouse 2 | 1.44 | 6.93 | 2.81 | 10.89 | 8.2 |

### Example 1.10

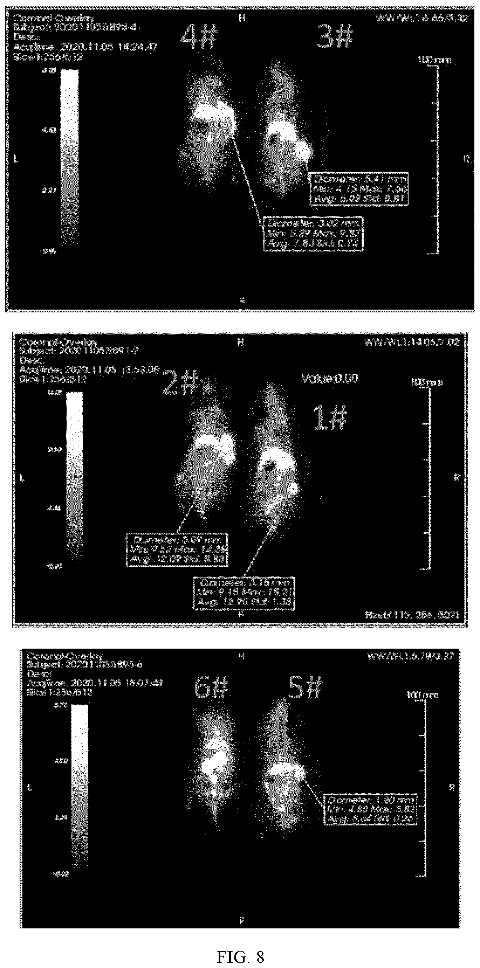

[⁸⁹Zr]Zr-ch3F8 was used to image a plurality of PDX models. [⁸⁹Zr]Zr-ch3F8 was injected intravenously. Images were collected 72 h after injection. FIG. 8 shows representative images of [⁸⁹Zr]Zr-ch3F8 imaging in 6 animal models. Table 4 lists HER3 expression levels in tumor tissues as determined by ELISA and the radioactive uptake in major organs and tumor tissues 72 h after injection. There was a considerable amount of tumor uptake in all 6 tested PDX models.

**Table 4. HER3 expression levels (ng/mg) in tumor tissues and radioactive uptake (% ID/g) in major organs and tumor tissues**

| No. | Model | HER3 (ng/mg, Elisa) | Heart | Kidney | Liver | Spleen | Tumor |
|---|---|---|---|---|---|---|---|
| 1 | 175P6 | 9.78 | 4.80 | 5.52 | 15.12 | 19.24 | 14.10 |
| 2 | 078P7 | 11.61 | 3.09 | 4.29 | 12.78 | 14.19 | 12.29 |
| 3 | 194P5 | 4.38 (P3) | 7.37 | 5.56 | 5.48 | 15.42 | 14.92 |
| 4 | 142P7 | 0.05 (P6) | 4.15 | 4.13 | 4.93 | 13.09 | 18.27 |
| 5 | 143P6 | 6.07 | 7.90 | 8.30 | 13.64 | 18.01 | 15.48 |
| 6 | 176R | 13.15 | 5.12 | 31.61 | 11.89 | 9.03 | 5.37 |

### Example 1.11

The binding affinity of humanized 3F8 (hu3F8) and chimeric 3F8. Binding kinetics of 3 humanized 3F8 clones and chimeric 3F8 were determined by Biacore. Three humanized 3F8 clones (clone 1, clone 2 and clone 3) had different heavy chains and one identical light chain. Clone 1, clone 2, and clone 3 corresponded to hu3F8-1, hu3F8-2, and hu3F8-3, respectively.

All data listed in Table 6 were processed using Biacore T200 evaluation software version 3.1.

**Table 6. Binding affinity of humanized 3F8 (hu3F8) and chimeric 3F8**

| **Ligand** | **Analyte** | **ka(1/Ms)** | **kd(1/s)** | **KD(M)** | **Rmax(RU)** | **Chi²(RU²)** |
|---|---|---|---|---|---|---|
| Chimeric | His-HER3 | 6.28E+05 | 3.91E-04 | 6.24E-10 | 103.1 | 8.35E-02 |
| Clone 1 | His-HER3 | 5.60E+05 | 6.12E-04 | 1.09E-09 | 110.1 | 9.52E-02 |
| Clone 2 | His-HER3 | 6.66E+05 | 3.28E-04 | 4.92E-10 | 113.2 | 4.63E-02 |
| Clone 3 | His-HER3 | 6.44E+05 | 3.48E-04 | 5.40E-10 | 124 | 3.60E-02 |

### Example 1.12

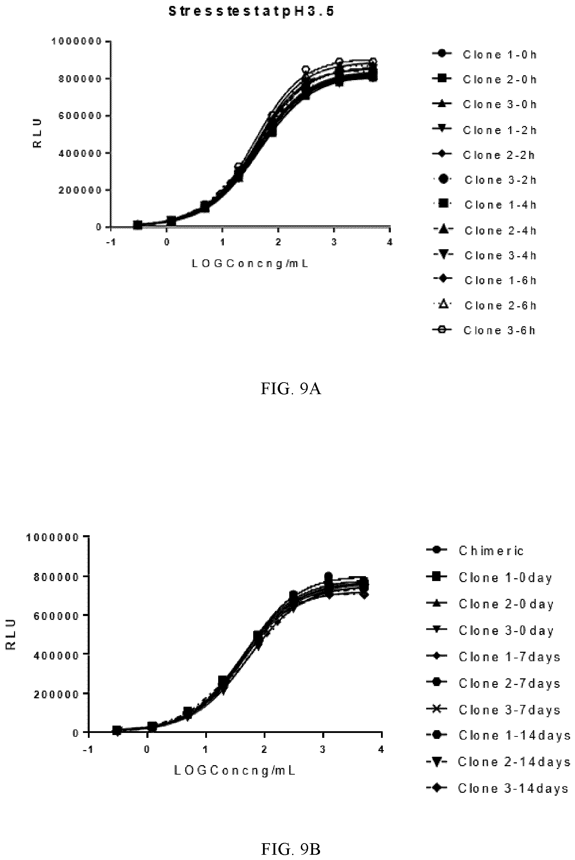

Three clones of hu3F8 were incubated at pH 3.5 for 0, 2, 4 and 6 h, and then subjected to ELISA to measure the binding affinities. The results (FIG. 9A) show that acid treatment had little effect on binding affinity.

EC50 assay of the three clones of hu3F8 after 0, 2, 4, and 6 h of incubation at pH 3.5. EC50 was determined by ELISA. The results (Table 7) show that acid treatment had little effect on binding affinity.

**Table 7. EC50 of three clones of hu3F8 after 0, 2, 4, and 6 h of incubation at pH3.5**

| Incubation time (hr) | EC₅₀ ng/mL | | |
|---|---|---|---|
| | Clone 1 | Clone 2 | Clone 3 |
| 0 | 38.74 | 45.04 | 38.71 |
| 2 | 38.11 | 48.31 | 41.71 |
| 4 | 40.89 | 44.54 | 39.45 |
| 6 | 38.96 | 41.18 | 38.06 |

The three clones of hu3F8 were incubated at 40 °C for different times and then subjected to ELISA to determine the binding affinities. The chimeric antibody was subjected to parallel assay. The results are shown in FIG. 9B.

EC50 assay of the three clones of hu3F8 incubated at 40 °C for different times. The three humanized 3F8 antibody clones were incubated in normal saline at 40 °C for 7 or 14 days, and the binding affinities after heat stress test were determined by ELISA. The chimeric antibody 3F8 was subjected to parallel assay. The results (Table 8) show that heat stress had little effect on binding affinity.

**Table 8. EC50 of three clones of hu3F8 incubated at 40 °C for different days**

| Incubation time at 40 °C (day) | EC₅₀ ng/mL | | | |
|---|---|---|---|---|
| | Chimeric antibody | Clone 1 | Clone 2 | Clone 3 |
| 0 | 47.37 | 39.16 | 45.40 | 42.44 |
| 7 | / | 42.35 | 48.09 | 46.96 |
| 14 | / | 41.00 | 52.03 | 41.25 |

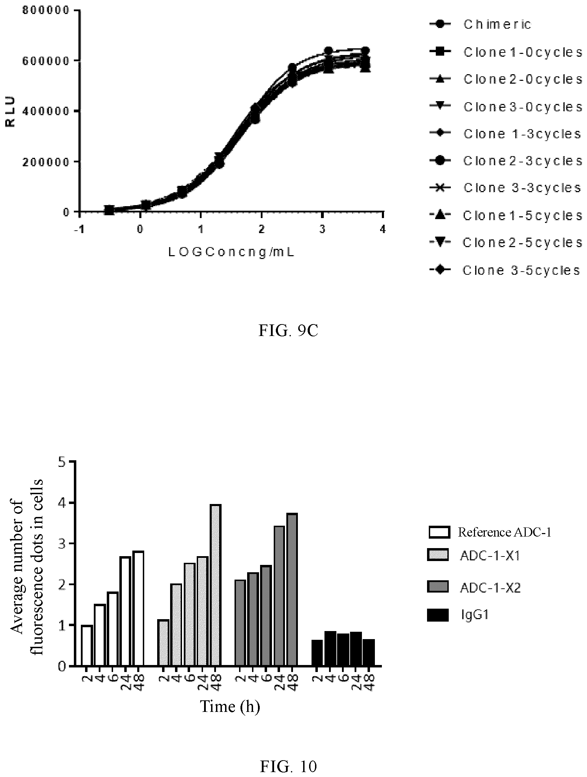

Three clones of hu3F8 were freeze-thawed for 3 or 5 cycles, and then subjected to ELISA to measure the binding affinities. The chimeric antibody was subjected to parallel assay. The results (FIG. 9C) show that repeated freeze-thaw had little effect on binding affinity.

Table 9 also shows EC50 of the three clones of hu3F8 that underwent a plurality of freeze-thaw stress tests.

**Table 9. EC50s of three clones of hu3F8 that underwent a plurality of freeze-thaw stress tests**

| Freeze-thaw cycle | EC₅₀ ng/mL | | | |
|---|---|---|---|---|
| | Chimeric antibody | Clone 1 | Clone 2 | Clone 3 |
| 0 | 47.06 | 45.41 | 42.46 | 42.25 |
| 3 | / | 44.29 | 46.66 | 37.42 |
| 5 | / | 38.58 | 43.79 | 33.63 |

In summary, the data indicates that hu3F8 retains binding affinity after testing of heat, acid, and repeated freeze-thaw, indicating that hu3F8 has good stability.

### Example 1.13

Aggregations of 3 clones of hu3F8 after stability test and their aggregation evaluations on storage at 4 °C. Aggregations of the three hu3F8 antibodies after acid treatment, repeated freeze-thaw and incubation at 40 °C were determined by SEC-HPLC and compared to each of the antibodies stored at 4 °C. The results are listed in Table 10. All the clones retained more than 95% of monomer after stress test, indicating little tendency to aggregate.

**Table 10. Results of aggregation evaluation of three clones of hu3F8 after stress test**

| Test item | Clone 1 | | | Clone 2 | | | Clone 3 | | |
|---|---|---|---|---|---|---|---|---|---|
| | Monomer | Dimer | Tetramer | Monomer | Dimer | Tetramer | Monomer | Dimer | Tetramer |
| Retention time | 8.84-8.85 | 7.33-7.35 | 6.66-6.68 | 8.86-8.87 | 7.36-7.37 | 6.70-6.73 | 8.86-8.87 | 7.36-7.37 | 6.70-6.73 |
| Antibody stored at 4 °C | 94.68% | 4.36% | 0.96% | 96.67% | 3.16% | 0.17% | 97.10% | 2.90% | - |
| Day 7 at 40 °C | 95.02% | 4.22% | 0.76% | 96.94% | 3.03% | 0.03% | 97.09% | 2.91% | - |
| Day 14 at 40 °C | 95.03% | 4.22% | 0.75% | 96.44% | 3.39% | 0.17% | 97.04% | 2.96% | - |
| pH 3.5 for 2 h | 94.63% | 4.41% | 0.96% | 96.98% | 2.99% | 0.04% | 97.04% | 2.96% | - |
| pH 3.5 for 4 h | 95.06% | 4.22% | 0.73% | 96.52% | 3.27% | 0.22% | 97.13% | 2.87% | - |
| pH 3.5 for 6 h | 94.80% | 4.29% | 0.91% | 96.57% | 3.27% | 0.16% | 97.06% | 2.94% | - |
| Freeze-thaw for 3 cycles | 94.63% | 4.41% | 0.96% | 96.65% | 3.22% | 0.13% | 96.97% | 2.90% | 0.13% |
| Freeze-thaw for 5 cycles | 94.69% | 4.43% | 0.88% | 96.31% | 3.44% | 0.25% | 97.01% | 2.87% | 0.12% |

### Example 1.14

Post-translational modification (PTM) analysis of hu3F8. P1: hu3F8 was not stressed; P2: hu3F8 was stressed at 40 °C for 2 weeks. hu3F8, which was not stressed or stressed at 40 °C for 2 weeks, was digested with trypsin, and then post-translational modification was analyzed by mass spectrometry.

The results show a slight increase in deamidation at HC: 372-393. In general, PTM did not differ much before and after stress, indicating good developability.

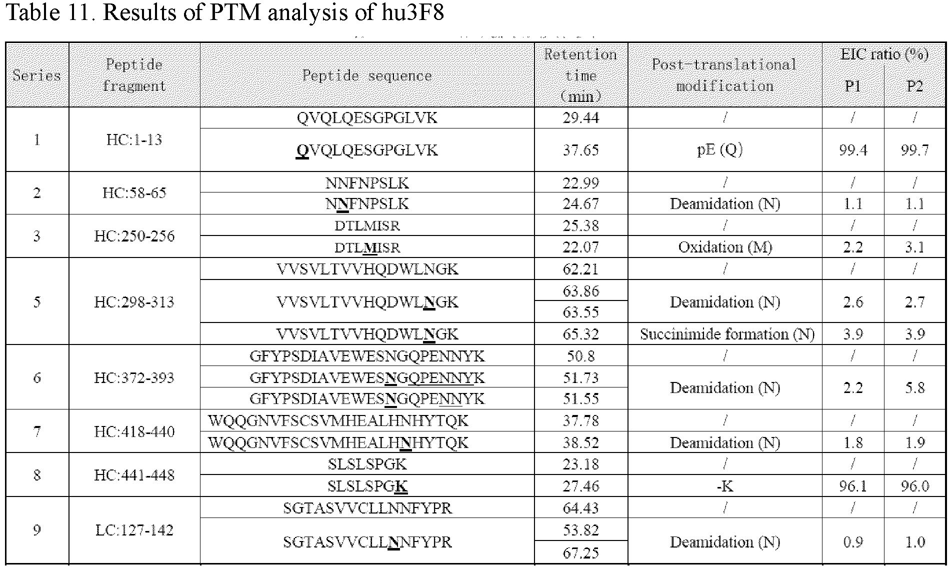

The amino acid sequences of the antibodies disclosed herein are listed below.
Amino acid sequence of light chain variable region of murine 3F8: 
DIQMTQTTSSLSASLGDRVTISCRASQDISNYLNWYQQKPDGTFKLLIYYTSILHSGVPSRFS
GSGSGTDYSLTISNLEQEDIATYFCQQGDTLPPTFG AGTKLELK (SEQ ID NO: 15)
Amino acid sequence of heavy chain variable region of murine 3F8: 
DVQLQESGPGLVKPSQSLSLTCSVTGYSITSAYYWNWIRQFPGDKLEWMGYISYDGRNNFN
PSLKNRISITRDTSKNQFFLKLNSVTSGDTATYYCARDGDYDYFDYWGQGTTLTVSS (SEQ
ID NO: 16)
Amino acid sequence of heavy chain of humanized 3F8 (Clone 1/hu3F8-1) 
QVQLQESGPGLVKPSETLSLTCTVSGYSITSAYYWNWIRQPPGKGLEWIGYISYDGRNNFNP
SLKNRVTISVDTSKNQFSLKLSSVTAADTAVYYCARDGDYDYFDYWGQGTTVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVM
HEALHNHYTQKSLS LSPGK (SEQ ID NO: 11)
Amino acid sequence of heavy chain of humanized 3F8 (Clone 2/hu3F8-2) 
QVQLQESGPGLVKPSETLSLTCTVSGYSITSAYYWNWIRQPFGKGLEWMGYISYDGRNNFN
PSLKNRVTISRDTSKNQFSLKLSSVTAADTAVYYCARDGDYDYFDYWGQGTTVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVM
HEALHNHYTQKSL SLSPGK (SEQ ID NO: 13)
Amino acid sequence of heavy chain of humanized 3F8 (Clone 3/hu3F8-3) 
QVQLQESGPGLVKPSETLSLTCTVSGYSITSAYYWNWIRQPFGKGLEWMGYISYDGRNNFN
PSLKNRVSISRDTSKNQFSLKLSSVTAADTATYYCARDGDYDYFDYWGQGTTVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVM
HEALHNHYTQKSLS LSPGK (SEQ ID NO: 14)
Amino acid sequence of light chain of humanized 3F8 (Clones 1-3/hu3F8-1-3)

DIQMTQSPSSLSASVGDRVTITCRASQDISNYLNWYQQKPGKAPKLLIYYTSILHSGVPSRFS
GSGSGTDYTFTISSLQPEDIATYFCQQGDTLPPTFGGGTKLEIKRTVAAPSVFIFPPSDEQLKS
GTASVVCLLNNFYPREAKVQWKVDNALQSGNSQESVTEQDSKDSTYSLSSTLTLSKADYE
KHKVYACEVTHQGLSSPVTKSFNRGEC (SEQ ID NO: 12)

### Example 2. Preparation of Anti-Her3 Antibody-Drug Conjugate (ADC)

### 1.1. Preparation of linker-cytotoxin (linker-payload) linker-payload X1

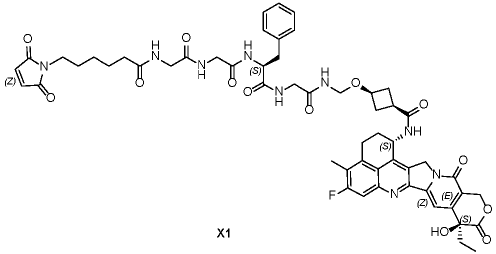

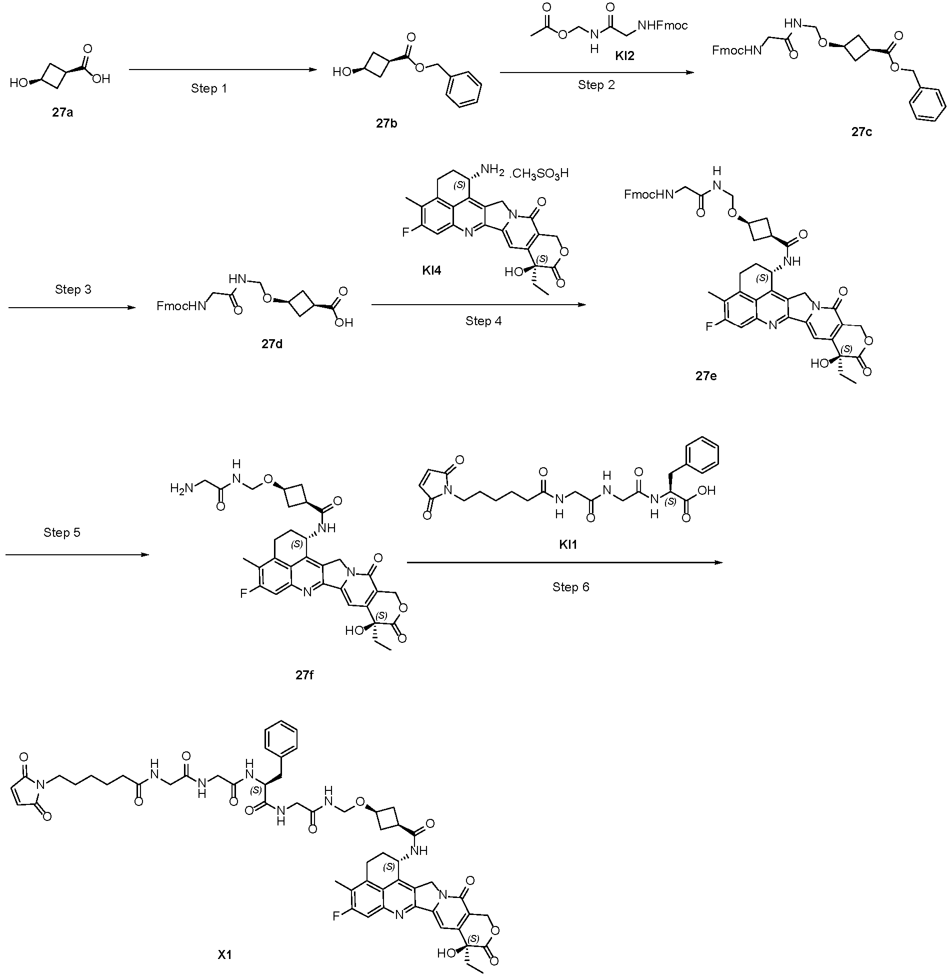

### Step 1

Benzyl bromide (11.0 g, 64.6 mmol) was added dropwise to a solution of 27a (5.00 g, 43.0 mmol) and NaHCO₃ (10.9 g, 129 mmol) in DMF (50 mL) under nitrogen atmosphere, and the mixture was reacted at 25 °C for 17 h. After the reaction was completed as detected by TLC (PE/EA = 2/1), the reaction solution was added to water (500 mL) and extracted twice with EA (250 mL). The organic phase was separated, washed with a saturated aqueous sodium chloride solution (500 mL), dried over anhydrous Na₂SO₄, concentrated, and purified by column chromatography (PE:EA = 3:2) to give a colorless liquid (5.1 g, yield 57.1%).

### Step 2

A solution of 27b (4.50 g, 21.8 mmol) in THF (10 mL) was added dropwise to a solution of KI2 (4.00 g, 10.9 mmol) and TsOH (800 mg, 4.65 mmol) in THF (30 mL) at 0 °C under nitrogen atmosphere, and the mixture was reacted at 25 °C for 2 h. After the reaction was completed as detected by TLC (PE/EA = 1/2), the reaction solution was added to water (200 mL) and extracted twice with EA (200 mL). The organic phase was separated, dried over anhydrous Na₂SO₄, concentrated, and purified by column chromatography (PE/EA = 3/2) to give a white solid (1.56 g, yield 26%).

### Step 3

Pd/C (80 mg) was added to a mixed solution of 27c (800 mg, 1.55 mmol) in EtOH (8 mL) and EA (8 mL) at 0 °C under hydrogen atmosphere, and the mixture was stirred at 0 °C for 2.5 h. After the reaction was completed as detected by LCMS, the reaction solution was filtered through celite, and the filter cake was washed with EA (200 mL). The filtrate was concentrated and dissolved in THF (20 mL), and dried by rotary evaporation to give a white solid (600 mg, 91% yield).

### Step 4

DIEA (152 mg, 1.18 mmol) was added to a solution of 27d (220 mg, 0.515 mmol), HY-13631A (250 mg, 0.47 mmol) and HATU (214 mg, 0.56 mmol) in DMF (6 mL) at 0 °C under nitrogen atmosphere, and the mixture was reacted at 0 °C for 2 h. After the reaction was completed as detected by LCMS, the reaction solution was added to an aqueous citric acid solution (pH = 4) (150 mL), and filtered. The filter cake was washed with water (175 mL), dried by filtration, and dried with an oil pump to give a brown solid (260 mg, yield 66%).

### Step 5

Diethylamine (8 mL) was added dropwise to a solution of 27e (260 mg, 0.309 mmol) in DCM (30 mL) at 0 °C under nitrogen atmosphere, and the mixture was reacted at 0 °C for 3 h. After the reaction was completed as detected by LCMS, the reaction solution was added to a petroleum ether solution (600 mL) at 0 °C, and a solid was precipitated. The resulting mixture was left to stand until the solid was adsorbed on the bottom of the flask, and the solution was poured out and dried with an oil pump to give a brown solid (90 mg, yield 47.1%).

### Step 6

HATU (74 mg, 0.19 mmol) was added to a solution of 27f (90 mg, 0.13 mmol), KI-1 (92 mg, 0.19 mmol) and DIEA (50 mg, 0.39 mmol) in DMF (2.5 mL) at 0 °C under nitrogen atmosphere, and the mixture was reacted at 0 °C for 2 h. After the reaction was substantially completed as detected by LCMS, the reaction solution was added to an aqueous citric acid solution (30 mL) at pH 4 at 0 °C, and a flocculent solid was precipitated. The resulting mixture was filtered, and purified on a preparation plate (DCM/MecOH = 10/1) to give X1 as a pale yellow solid (9.2 mg, yield 6%).
MS m/z (ESI): 1074 [M+1]

H-NMR (400 MHz, MeOD): 7.65 (d, 1H), 7.62 (s, 1H), 7.30-7.21(m, 5H), 6.79 (s, 2H), 5.69-5.65 (m, 1 H), 5.57 (d, 1H), 5.43-5.10 (m, 3H), 4.70 (d, 2H), 4.48-4.39 (m, 2H), 4.10-4.05 (m, 1H), 4.01-3.75 (m, 5H),3.46 (t, 2H), 3.22-3.15 (m, 2H), 3.07-3.00 (m, 1H), 2.75 (m, 1H), 2.62 (m, 1H), 2.45 (s, 3H), 2.37-2.20 (m, 6H), 2.10-2.02 (m, 2H), 2.00-1.92 (m, 2H) 1.68-1.57 (m, 6H), 1.01 (t, 3H)

### linker-payload X2

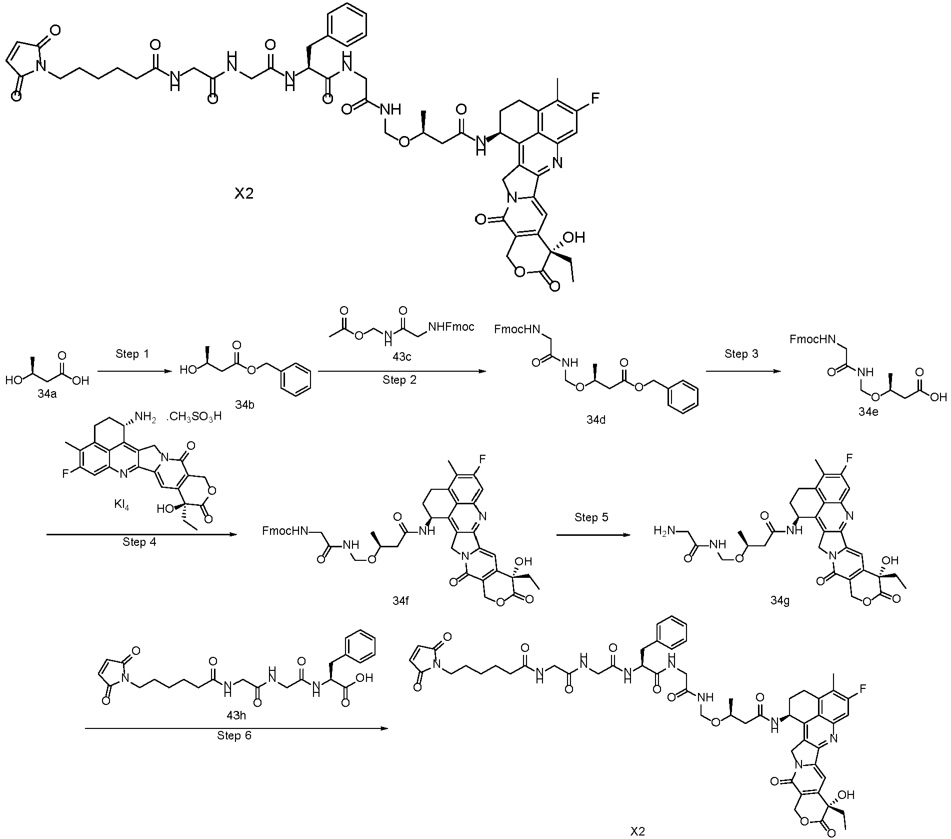

### Step 1

34a (5 g, 48.0 mmol) and K₂CO₃ (19.9 g, 144.0 mmol) were dissolved in DMF (20 mL), followed by benzyl bromide (12.3 g, 72.0 mmol) added dropwise. The mixture was reacted at 25 °C for 17 h. After the starting materials were completely consumed as detected by TLC (PE/EA = 3/1), the reaction solution was added to water (200 mL) and extracted with EA (250 mL). The organic phase was separated, washed with saturated NaCl, dried over anhydrous Na₂SO₄, concentrated, and purified by column chromatography (PE:EA = 2:1) to give 34b (8.7 g, yield 93%) as a colorless liquid. MS-ESI: m/z 195.1 [M+H]+.

### Step 2

34c (7.3 g, 19.8 mmol) and TsOH (1.46 g, 8.5 mmol) were dissolved in THF (20 mL), and the mixture was cooled to 0 °C under nitrogen atmosphere. A solution of 43b (7.7 g, 39.6 mmol) in THF (10 mL) was added dropwise, and after the addition, the mixture was reacted at 0 °C for 2 h. After most of the starting materials were consumed as detected by TLC (PE/EA= 2/1), the reaction solution was poured into water (100 mL) and extracted with DCM (100 mL). The organic phase was separated, washed with saturated NaCl, dried over anhydrous Na₂SO₄, and purified by column chromatography (PE/EA = 1/1) to give 34d as a colorless sticky substance (3.9 g, 39% yield). MS-ESI: m/z 503.3 [M+H]+.

### Step 3

Pd/C (1 g, 10 wt.%) was added to a mixed solution of 34d (1.9 g, 3.78 mmol) in EtOH (100 mL) and EA (100 mL) at 0 °C under hydrogen atmosphere, and the mixture was reacted at 0 °C for 3 h. After the reaction was completed as detected by TLC (PE/EA = 2/1), the reaction solution was filtered through celite, and the filter cake was washed with EA/EtOH (1:1, 100 mL × 3). The filtrate was concentrated and dissolved in THF (50 mL × 3), and dried by rotary evaporation; the procedure was repeated three times to give 34e as a gray solid (1 g, 64% yield). MS-ESI: m/z 435.2 [M+Na]+.

### Step 4

DIEA (303 mg, 2.35 mmol) was added dropwise to a solution of 34e (426 mg, 1.03 mmol), KI4 (500 mg, 0.94 mmol) and HATU (429 mg, 1.13 mmol) in DMF (20 mL) at 0 °C under nitrogen atmosphere, and the mixture was reacted at 0 °C for 2 h after the addition was completed. After the reaction was completed as detected by LCMS, the reaction solution was added dropwise to water (300 mL). The resulting mixture was stirred, then left to stand for 5 min, and filtered, and the filter cake was dissolved in DCM/MeOH (10:1, 100 mL) solution. The resulting solution was dried and concentrated to dryness by rotary evaporation, and the residue was mixed with silica gel and purified by column chromatography (EA:MeOH = 30:1) to give 34f as a yellow solid (600 mg, 77% yield). MS-ESI: m/z 830.3 [M+H]+.

### Step 5

Diethylamine (5 mL) was added dropwise to a solution of 34f (150 mg, 0.18 mmol) in DCM (5 mL) at 0 °C under nitrogen atmosphere, and the mixture was reacted at 0 °C for 2 h. After the reaction was completed as detected by LCMS, a petroleum ether solution (100 mL × 6) was added to the reaction solution, and a solid precipitated. The resulting mixture was let stand until the solid settled, and the solution was poured out. The residue was dried with an oil pump to give 34g as a white powder (120 mg, 76% yield), with a product content of 70% as determined by LCMS. MS-ESI: m/z 608.3 [M+H]+.

### Step 6

A solution of HATU (45 mg, 0.118 mmol) in DMF (1 mL) was added to a solution of 34g (60 mg, 0.099 mmol), 43h (51 mg, 0.108 mmol) and DIEA (32 mg, 0.25 mmol) in DMF (1 mL) at 0 °C under nitrogen atmosphere, and the mixture was reacted at 0 °C for 2 h. After the starting materials were completely consumed as detected by LCMS, the reaction solution was directly purified by reversed-phase column chromatography (eluent: (MeCN/MeOH = 1/1):H2O = 60%:40%) to give X2 as a yellow solid (14.8 mg, 14% yield).
MS-ESI: m/z 1062.4 [M+H]+.

1H NMR (400 MHz, Methanol-d4) δ 7.69 - 7.61 (m, 2H), 7.22 - 7.16 (m, 2H), 7.16 - 7.09 (m, 3H), 6.76 (s, 2H), 5.70 - 5.64 (m, 1H), 5.60 (d, J = 16.4 Hz, 1H), 5.40 - 5.31 (m, 2H), 5.26 (d, J = 19.0 Hz, 1H), 4.65 - 4.50 (m, 7H), 4.25 - 4.16 (m, 1H), 3.87 (d, J = 16.7 Hz, 1H), 3.83 - 3.76 (m, 3H), 3.72 (d, J = 17.0 Hz, 2H), 3.44 (t, J = 7.1 Hz, 2H), 3.25 - 3.17 (m, 2H), 3.10 - 3.02 (m, 1H), 2.92 - 2.83 (m, 1H), 2.45 - 2.39 (m, 5H), 2.32 - 2.20 (m, 5H), 1.97 - 1.89 (m, 2H), 1.63 - 1.50 (m, 4H), 1.34 - 1.20 (m, 6H), 0.99 (t, J = 7.3 Hz, 3H).

### linker-payload X3

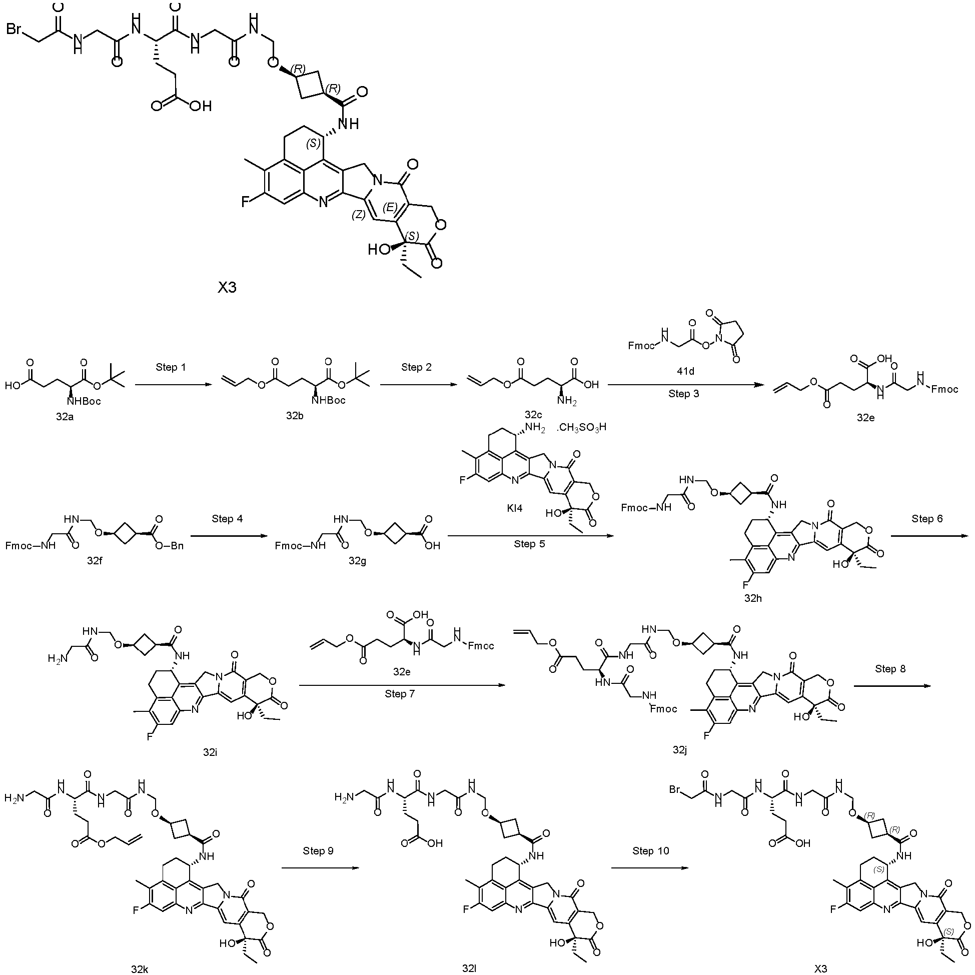

### Step 1

Bromopropene (960 mg, 7.92 mmol) was added to 32a (2.00 g, 6.6 mmol) and K2CO3 (1.82 g, 13.2 mmol) in MeCN (20 mL), and the mixture was stirred at 20 °C for 5 h. After the reaction was completed as detected by TLC (PE/EA= 1/2), the reaction solution was poured into water (100 mL), adjusted to pH 5, and extracted three times with EA (100 mL). The organic phase was dried over anhydrous sodium sulfate and concentrated to dryness by rotary evaporation, and the residue was purified by column chromatography (PE/EA = 2/1) to give 32b as a white solid (1.83 g, 81% yield).

### Step 2

TFA (10 mL) was added to 32b (1.38 g, 4.02 mmol) in DCM (10 mL), and the mixture was stirred at 25 °C for 17 h. After the reaction was completed as detected by TLC (PE/EA = 1/3), the reaction solution was concentrated to dryness by rotary evaporation to give 32c as a yellow sticky substance (0.91 g, yield not calculated).

### Step 3

41d (1.92 g, 4.87 mmol) was added to 32c (910 mg, 4.87 mmol) and NaHCO3 (613 mg, 7.3 mmol) in DME/H2O (20 mL/10 mL), and the mixture was stirred at 25 °C for 3 h. After the reaction was completed as detected by TLC (DCM/MeOH = 1/1), the reaction solution was poured into water (100 mL), adjusted to pH 5 with aq. HCl (1 N), and extracted twice with EA (150 mL). The organic phase was dried over anhydrous sodium sulfate and concentrated to dryness by rotary evaporation, and the residue was purified by column chromatography (DCM/MeOH = 20/1) to give 32e as a white solid (1.53 g, 67% yield). MS-ESI: m/z 467.4 [M+H]+.

### Step 4

Pd/C (600 mg) was added to 32f (3 g, 5.83 mmol) in MeOH (50 mL), and the mixture was stirred under hydrogen at 25 °C for 5 h. After the reaction was completed as detected by TLC (EA), the reaction solution was filtered, and the filtrate was concentrated to dryness by rotary evaporation to give 32g as a white solid (1.9 g, 77% yield).

### Step 5

HATU (707 mg, 1.86 mmol) was added to 32g (789 mg, 1.86 mmol), KI4 (900 mg, 1.69 mmol) and triethylamine (342 mg, 3.38 mmol) in DMF (10 mL), and the mixture was stirred at 0 °C for 3.5 h. After the reaction was completed as detected by TLC (EA), the reaction solution was poured into H2O (80 mL) and extracted twice with EA (100 mL). The organic phase was dried over anhydrous sodium sulfate and concentrated to dryness by rotary evaporation, and the residue was purified by column chromatography (EA) to give 32h as a white solid (1.186 g, 83% yield). MS-ESI: m/z 842.3 [M+H]+.

### Step 6

32h (1.186 g, 1.41 mmol) in DCM/diethylamine (20 mL, 20/1) was stirred at 25 °C for 17 h. After the reaction was completed as detected by TLC (DCM/MeOH = 10/1), the reaction solution was poured into petroleum ether (200 mL), and the resulting mixture was filtered to give 32i as a white solid (768 mg, 88% yield). MS-ESI: m/z 620.3 [M+H]+.

### Step 7

HATU (414 mg, 1.09 mmol) was added to 32i (676 mg, 1.09 mmol), 32e (508 mg, 1.09 mmol) and DIEA (423 mg, 3.27 mmol) in DMF (10 mL), and the mixture was stirred at 20 °C for 17 h. After the reaction was completed as detected by TLC (PE/EA = 1/5), the reaction solution was poured into water (30 mL). The resulting mixture was filtered, and the filter cake was purified by column chromatography (DCM/MeOH = 50/1) to give 32j as a white solid (511 mg, 44% yield). MS-ESI: m/z 1068.3 [M+H]+.

### Step 8

A solution of 32j (482 mg, 0.451 mmol) in diethylamine/DCM (10 mL, 1/5) was stirred at 10 °C for 17 h. After the reaction was completed as detected by TLC (EA), the reaction solution was poured into PE (300 mL), and the resulting mixture was filtered to give 32k as a white solid (301 mg, yield not calculated).

### Step 9

Morpholine (93 mg, 1.07 mmol) was added to 32k (301 mg, 0.356 mmol) and Pd(PPh3)4 (82 mg, 0.071 mmol) in THF (5 mL), and the mixture was stirred at 25 °C for 5 h. After the reaction was completed as detected by LCMS, the reaction solution was purified by preparative chromatography to give 32l as a white solid (108 mg, 38% yield). MS-ESI: m/z 806.3 [M+H]+.

### Step 10

Bromoacetyl bromide (27 mg, 0.134 mmol) was added to 32l (108 mg, 0.134 mmol) and triethylamine (41 mg, 0.402 mmol) in THF (2 mL) and DMF (2 mL), and the mixture was stirred at 0 °C for 1 h. After the reaction was completed as detected by TLC (DCM/MeOH = 10/1), the reaction solution was directly purified by preparative chromatography to give X3 as a white solid (15 mg, 12% yield). MS-ESI: m/z 926.3 [M+H]+.

1H NMR (400 MHz, DMSO-d6) δ 12.11 (s, 1H), 8.54 - 8.42 (m, 3H), 8.27 - 8.16 (m, 2H), 7.78 (d, J = 11.0 Hz, 1H), 7.30 (s, 1H), 6.53 (s, 1H), 5.61 - 5.51 (m, 1H), 5.42 (s, 2H), 5.20 - 5.05 (m, 2H), 4.56 - 4.42 (m, 2H), 4.32 - 4.22 (m, 1H), 3.96 - 3.87 (m, 3H), 3.79 (d, J = 5.6 Hz, 2H), 3.70 (d, J = 5.9 Hz, 2H), 3.25 - 3.08 (m, 2H), 2.61 - 2.53 (m, 2H), 2.45 - 2.36 (m, 4H), 2.36 - 2.22 (m, 3H), 2.20 - 2.03 (m, 4H), 1.99 - 1.68 (m, 4H), 0.87 (t, J = 7.3 Hz, 3H).

### linker-payload X4

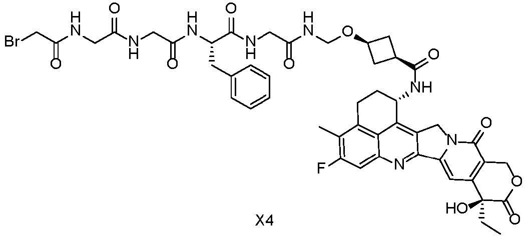

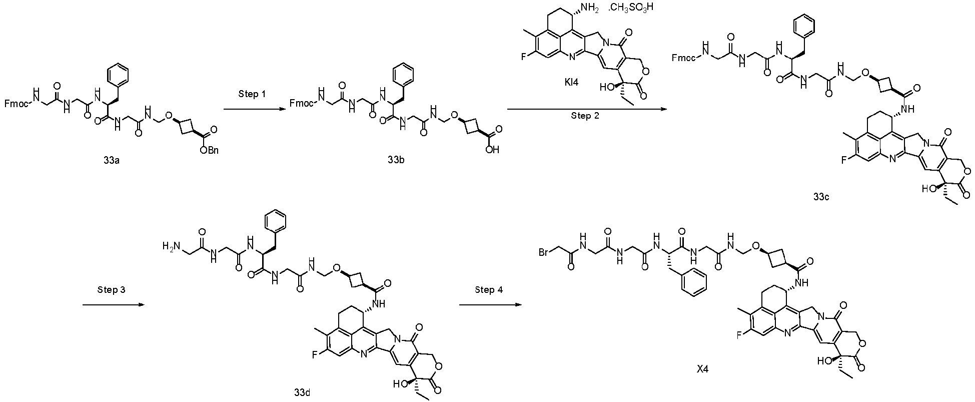

### Step 1

Pd/C (400 mg, 10 wt.%) was added to 33a (2.00 g, 2.58 mmol) in MeOH (20 mL), and the mixture was stirred at 20 °C for 5 h. After the reaction was completed as detected by TLC (EA), the reaction solution was filtered, and the filtrate was concentrated to dryness by rotary evaporation to give 33b as a white solid (1.3 g, 74% yield).

### Step 2

HATU (305 mg, 0.802 mmol) was added to 33b (0.55 g, 0.802 mmol), KI4 (427 mg, 0.802 mmol) and DIPEA (310 mg, 2.40 mmol) in DMF (5 mL), and the mixture was stirred at 0 °C for 2 h. After the reaction was completed as detected by TLC (DCM/MeOH = 1/10), the reaction solution was poured into water (40 mL). The resulting mixture was filtered to give a crude product, which was purified by column chromatography (DCM/MeOH = 20/1) to give 33c as a yellow solid (360 mg, 41% yield).

### Step 3

Diethylamine (2 mL) was added to 33c (360 mg, 0.326 mmol) in DCM (10 mL), and the mixture was stirred at 25 °C for 17 h. After the reaction was completed as detected by TLC (DCM/MeOH = 5/1), the reaction solution was poured into PE (100 mL), and the resulting mixture was filtered to give 33d as a white solid (205 mg, 71% yield). MS-ESI: m/z 881.3 [M+H]+.

### Step 4

A solution of bromoacetyl bromide (94 mg, 0.446 mmol) in THF (2 mL) was added to 33d (205 mg, 0.233 mmol) and triethylamine (118 mg, 1.17 mmol) in DMF (1 mL) and water (1 mL), and the mixture was stirred at 0 °C for 1 h. The reaction solution was directly purified by preparative chromatography to give X4 as a white solid (15 mg, 6% yield).

MS-ESI: m/z 1001.2 [M+H]+.

1H NMR (400 MHz, DMSO-d6) δ 8.57 - 8.50 (m, 1H), 8.50 - 8.43 (m, 2H), 8.35 - 8.29 (m, 1H), 8.19 - 8.12 (m, 2H), 7.80 (d, J = 10.8 Hz, 1H), 7.27 - 7.14 (m, 7H), 6.53 (s, 1H), 5.59 - 5.51 (m, 1H), 5.44 - 5.39 (m, 2H), 5.20 - 5.07 (m, 2H), 4.56 - 4.44 (m, 3H), 3.92 (s, 3H), 3.80 - 3.68 (m, 5H), 3.41 (s, 1H), 3.21 - 3.12 (m, 2H), 2.83 - 2.74 (m, 1H), 2.58 - 2.55 (m, 3H), 2.39 (s, 4H), 2.18 - 2.03 (m, 4H), 1.93 - 1.78 (m, 2H), 0.87 (t, J = 7.3 Hz, 3H).

### 2.2. Preparation of anti-Her3 antibody-drug conjugate

### Preparation of antibody-drug conjugate ADC-1-X1 (hu3F8-2-X1)

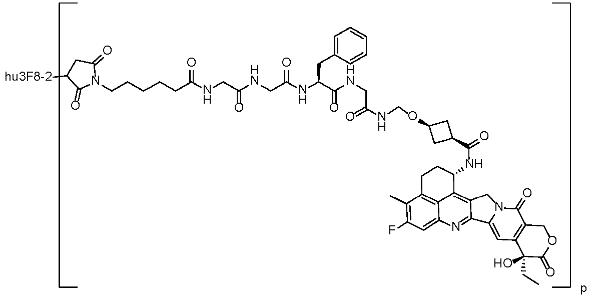

A reducing agent and a protective agent were formulated using ultrapure water: a 2 mg/mL TCEP (tris-2-carboxyethyl-phosphine, manufacturer: Thermo) and a 100 mmol/L sodium ethylenediaminetetraacetate salt (manufacturer: Sigma). 150 mg of 7.4 mg/mL anti-Her3 antibody (hu3F8-2) was placed into a 50 mL centrifuge tube and diluted to an antibody reaction concentration of 5 mg/mL by adding 50 mM sodium phosphate buffer (pH 7.5). 100 mmol/L sodium ethylenediaminetetraacetate salt was added in an amount of 5% of the total volume of the reaction solution, and the resulting mixture was well mixed by shaking. Then the antibody was reduced by adding 2 mg/mL TCEP in a TCEP-to-antibody molar ratio of 6.0:1, and the resulting mixture was well mixed by shaking and reacted on a thermomixer at 37 °C for 2 h. A 10 mg/mL solution of linker-payload X1 in dimethyl sulfoxide (manufacturer: Sinopharm) was prepared and slowly added according to the molar ratio of the drug to the antibody of 18.0:1. The resulting mixture was well mixed by shaking and reacted on a thermomixer at 4 °C for 1 h. The sample was exchanged into a storage buffer using an ultrafiltration tube (MWCO 30 KD, manufacturer: Millipore). First, ultrafiltration was performed 3 times using a 30 mM histidine acetate buffer (pH 5.5) containing 10% dimethyl sulfoxide, then ultrafiltration was performed 3 times using a 30 mM histidine acetate buffer (pH 5.5) without DMSO, and then ultrafiltration concentration was performed to give ADC-1-X1 (at a concentration of 26.8 mg/mL, 73% yield). The purity and DAR value were determined using size exclusion chromatography and hydrophobic chromatography.

Tests showed that the purity of the antibody-drug conjugate ADC-1-X1 was 98.68% and the DAR value p was 7.12.

### Preparation of antibody-drug conjugate ADC-2-X1 (hu3F8-3-X1)

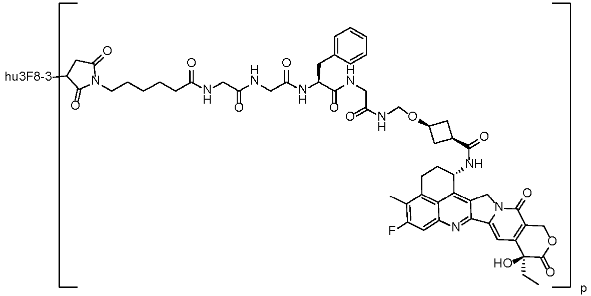

A reducing agent and a protective agent were formulated using ultrapure water: a 2 mg/mL TCEP (tris-2-carboxyethyl-phosphine, manufacturer: Thermo) and a 100 mmol/L sodium ethylenediaminetetraacetate salt (manufacturer: Sigma). 150 mg of 7.5 mg/mL anti-Her3 antibody (hu3F8-3) was placed into a 50 mL centrifuge tube and diluted to an antibody reaction concentration of 5 mg/mL by adding 50 mM sodium phosphate buffer (pH 7.5). 100 mmol/L sodium ethylenediaminetetraacetate salt was added in an amount of 5% of the total volume of the reaction solution, and the resulting mixture was well mixed by shaking. Then the antibody was reduced by adding 2 mg/mL TCEP in a TCEP-to-antibody molar ratio of 6.0:1, and the resulting mixture was well mixed by shaking and reacted on a thermomixer at 37 °C for 2 h. A 10 mg/mL solution of linker-payload X1 in dimethyl sulfoxide (manufacturer: Sinopharm) was prepared and slowly added according to the molar ratio of the drug to the antibody of 18.0: 1. The resulting mixture was well mixed by shaking and reacted on a thermomixer at 4 °C for 1 h. The sample was exchanged into a storage buffer using an ultrafiltration tube (MWCO 30 KD, manufacturer: Millipore). First, ultrafiltration was performed 3 times using a 30 mM histidine acetate buffer (pH 5.5) containing 10% dimethyl sulfoxide, then ultrafiltration was performed 3 times using a 30 mM histidine acetate buffer (pH 5.5) without DMSO, and then ultrafiltration concentration was performed to give an antibody-drug conjugate ADC-2-X1 (at a concentration of 21.15 mg/mL, 67.3% yield). The purity and DAR value were determined using size exclusion chromatography and hydrophobic chromatography. Tests showed that the purity of the antibody-drug conjugate ADC-2-X1 was 99.08% and the DAR value p was 7.01.

### Preparation of antibody-drug conjugate ADC-1-X2 (hu3F8-2-X2)

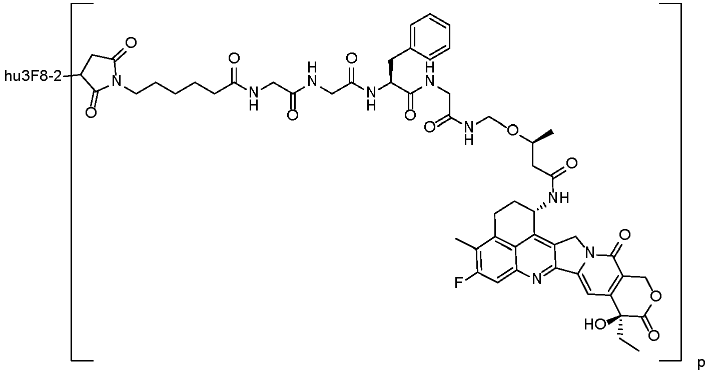

A reducing agent and a protective agent were formulated using ultrapure water: a 2 mg/mL TCEP (tris-2-carboxyethyl-phosphine, manufacturer: Thermo) and a 100 mmol/L sodium ethylenediaminetetraacetate salt (manufacturer: Sigma). 150 mg of 7.4 mg/mL anti-Her3 antibody (hu3F8-2) was placed into a 50 mL centrifuge tube and diluted to an antibody reaction concentration of 5 mg/mL by adding 50 mM sodium phosphate buffer (pH 7.5). 100 mmol/L sodium ethylenediaminetetraacetate salt was added in an amount of 5% of the total volume of the reaction solution, and the resulting mixture was well mixed by shaking. Then the antibody was reduced by adding 2 mg/mL TCEP in a TCEP-to-antibody molar ratio of 6.0:1, and the resulting mixture was well mixed by shaking and reacted on a thermomixer at 37 °C for 2 h. A 10 mg/mL solution of linker-payload X2 in dimethyl sulfoxide (manufacturer: Sinopharm) was prepared and slowly added according to the molar ratio of the drug to the antibody of 21: 1. The resulting mixture was well mixed by shaking and reacted on a thermomixer at 4 °C for 1 h. The sample was exchanged into a storage buffer using an ultrafiltration tube (MWCO 30 KD, manufacturer: Millipore). First, ultrafiltration was performed 3 times using a 30 mM histidine acetate buffer (pH 5.5) containing 10% dimethyl sulfoxide, then ultrafiltration was performed 3 times using a 30 mM histidine acetate buffer (pH 5.5) without DMSO, and then ultrafiltration concentration was performed to give an antibody-drug conjugate ADC-1-X2 (at a concentration of 20.56 mg/mL, 64% yield). The purity and DAR value were determined using size exclusion chromatography and hydrophobic chromatography.

Tests showed that the purity of the antibody-drug conjugate ADC-1-X2 was 99.02% and the DAR value p was 7.53.

### Preparation of antibody-drug conjugate ADC-2-X2 (hu3F8-3-X2)

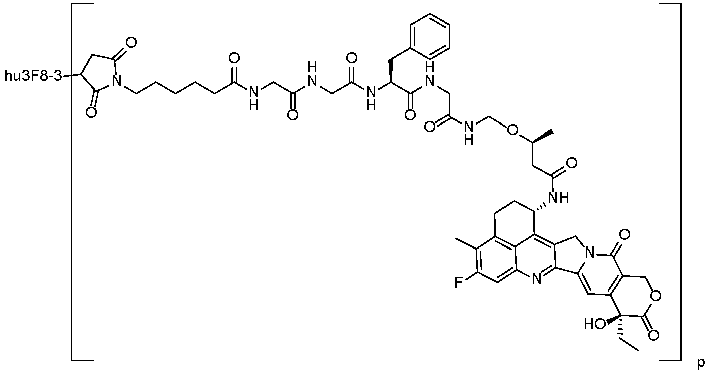

A reducing agent and a protective agent were formulated using ultrapure water: a 2 mg/mL TCEP (tris-2-carboxyethyl-phosphine, manufacturer: Thermo) and a 100 mmol/L sodium ethylenediaminetetraacetate salt (manufacturer: Sigma). 150 mg of 7.5 mg/mL anti-Her3 antibody (hu3F8-3) was placed into a 50 mL centrifuge tube and diluted to an antibody reaction concentration of 5 mg/mL by adding 50 mM sodium phosphate buffer (pH 7.5). 100 mmol/L sodium ethylenediaminetetraacetate salt was added in an amount of 5% of the total volume of the reaction solution, and the resulting mixture was well mixed by shaking. Then the antibody was reduced by adding 2 mg/mL TCEP in a TCEP-to-antibody molar ratio of 6.0:1, and the resulting mixture was well mixed by shaking and reacted on a thermomixer at 37 °C for 2 h. A 10 mg/mL solution of linker-payload X2 in dimethyl sulfoxide (manufacturer: Sinopharm) was prepared and slowly added according to the molar ratio of the drug to the antibody of 18.0: 1. The resulting mixture was well mixed by shaking and reacted on a thermomixer at 4 °C for 1 h. The sample was exchanged into a storage buffer using an ultrafiltration tube (MWCO 30 KD, manufacturer: Millipore). First, ultrafiltration was performed 3 times using a 30 mM histidine acetate buffer (pH 5.5) containing 10% dimethyl sulfoxide, then ultrafiltration was performed 3 times using a 30 mM histidine acetate buffer (pH 5.5) without DMSO, and then ultrafiltration concentration was performed to give an antibody-drug conjugate ADC-2-X2 (at a concentration of 21.63 mg/mL, 67.3% yield). The purity and DAR value were determined using size exclusion chromatography and hydrophobic chromatography. Tests showed that the purity of the antibody-drug conjugate ADC-2-X2 was 99.24% and the DAR value p was 7.11.

### Preparation of reference ADC-1 (U3-1402)

A reducing agent and a protective agent were formulated using ultrapure water: a 2 mg/mL TCEP (tris-2-carboxyethyl-phosphine, manufacturer: Thermo) and a 100 mmol/L sodium ethylenediaminetetraacetate salt (manufacturer: Sigma). 40 mg of 6.9 mg/mL patritumab monoclonal antibody (the antibody sequence comprises a heavy chain set forth in sequence No. 70 and a light chain set forth in sequence No. 72 in the patent "WO2007077028"; the antibody was produced by transfection of CHO cells followed by routine antibody expression and purification, with >95% purity) was placed into a 50 mL centrifuge tube and diluted to an antibody reaction concentration of 5 mg/mL by adding 50 mM sodium phosphate buffer (pH 7.5). 100 mmol/L sodium ethylenediaminetetraacetate salt was added in an amount of 5% of the total volume of the reaction solution, and the resulting mixture was well mixed by shaking. Then the antibody was reduced by adding 2 mg/mL TCEP in a TCEP-to-antibody molar ratio of 6.0:1, and the resulting mixture was well mixed by shaking and reacted on a thermomixer at 37 °C for 2 h. A 10 mg/mL solution of Deruxtecan (CAS 1599440-13-7, purchased from Shanghai Haoyuan Chemexpress Co. Ltd.) in dimethyl sulfoxide (manufacturer: Sinopharm) was prepared and slowly added according to the molar ratio of the drug to the antibody of 18.0: 1. The resulting mixture was well mixed by shaking and reacted on a thermomixer at 4 °C for 1 h. The sample was exchanged into a storage buffer using an ultrafiltration tube (MWCO 30 KD, manufacturer: Millipore). First, ultrafiltration was performed 3 times using a 30 mM histidine acetate buffer (pH 5.5) containing 10% dimethyl sulfoxide, and then ultrafiltration was performed 3 times using a 30 mM histidine acetate buffer (pH 5.5) without DMSO to give reference ADC-1 (at a concentration of 8.77 mg/mL, 65% yield). The purity and DAR value were determined using size exclusion chromatography and hydrophobic chromatography.

Tests showed that the purity of the reference ADC-1 was 99.42% and the DAR value p was 7.58.

### Example 3. Internalization Activity of Antibody-Drug Conjugate

### Objective

The up-taken of the antibody-drug conjugate of the present application against the HER3 target on MDA-MB-453 cells expressing HER3 was detected. The cell, the antibody-drug conjugate (ADC) with a fixed concentration, and an internalization indicating reagent pHrodo were co-incubated, and the endocytosis capacity of the antibody-drug conjugate was evaluated by observing the number of fluorescent signal bright dots generated by pHrodo which entered the cell along with the antibody-drug conjugate in the cell at different time points.

### Procedures

1. Cell culture: MDA-MB-453 cells were cultured using a 10% FBS Leibovitz's L-15 medium.
2. Cell preparation: MDA-MB-453 cells in logarithmic phase were taken, washed with PBS once, and digested with 2-3 mL of Versene for 2-3 min. After the cells were digested completely, 10-15 mL of cell culture was added to elute the digested cells. The eluate was centrifuged at 1000 rpm for 5 min, and the supernatant was discarded. The resulting cells were resuspended in cell culture to give a single cell suspension, which was adjusted to a viable cell density of 3 × 10⁵ cells/mL.
3. Cell plating: the cell suspension was added to a 96-well cell culture plate at 50 µL/well. The culture plate was incubated in an incubator for 18 h (37 °C, 5% CO₂).
4. Sample adding: the test antibody-drug conjugate was co-incubated with Fab-pHrodo to form a complex, adjusted to a concentration of 10 nM, and added to the cells at 50 µL/well.
5. Culturing of cells: the culture plate was incubated in an incubator for 2, 4, 6, 24, or 48 h (37 °C, 5% CO₂).
6. Plate reading: when the corresponding time point was reached, the 96-well cell culture plate was taken out, Hoechst33342 was added to perform cell nucleus staining, the plate was placed in operatta CLS for photographing, and the number of cells and the number of fluorescent bright dots in each cell were read. The average number of cell fluorescent dots was calculated using Harmony^{®} imaging and analysis software.

**Table 12. Fluorescent signal bright dots generated by internalization of the antibody-drug conjugate of the present application at different time points in MDA-MB-453 cells**

| Antibody-drug conjugate | Average number of fluorescence dots in cells | | | | |
|---|---|---|---|---|---|
| Assay time (h) | 2 | 4 | 6 | 24 | 48 |
| Reference ADC-1 | 1.0 | 1.5 | 1.8 | 2.7 | 2.8 |
| ADC-1-X1 | 1.1 | 2.0 | 2.5 | 2.7 | 4.0 |
| ADC-1-X2 | 2.1 | 2.3 | 2.5 | 3.4 | 3.7 |
| ADC-2-X1 | 1.4 | 2.1 | 2.5 | 3.3 | 3.5 |
| ADC-2-X2 | 1.4 | 2.1 | 2.6 | 2.6 | 3.9 |
| IgG1 | 0.6 | 0.8 | 0.8 | 0.8 | 0.7 |

Conclusion: according to the results of FIG. 10 and Table 12, the antibody-drug conjugate of the present application has an up-taken on MDA-MB-453 cells with high expression of HER3, and the endocytosis capacity is stronger than that of the reference ADC-1.

### Example 4. Proliferation Inhibtion of Tumor Cells by HER3 Targeting Antibody-Drug Conjugate In Vitro

### Objective

To test the proliferation inhibitory activity of the antibody-drug conjugate of the present application targeting HER3 to HCC1569 and MDA-MB-453 cells with high expression of Her3 and MDA-MB-231 tumor cells not expressing Her3 in vitro. The cells were treated with the antibody-drug conjugates at different concentrations in vitro, and after 6 days of culture, the proliferation of cells was detected using the CTG reagent, and the in vitro activity of the compounds was evaluated according to the 50% growth inhibitory concentration (IC₅₀) value.

### Procedures

1. Cell culture: HCC1569, MDA-MB-453, or MDA-MB-231 cells were cultured in RPMI-1640 medium containing 10% FBS.
2. Cell Preparation: HCC1569, MDA-MB-453, or MDA-MB-231 cells in logarithmic phase were taken, washed with PBS once, and digested with 2-3 mL of trypsin for 2-3 min. After the cells were digested completely, 10-15 mL of cell culture was added to elute the digested cells. The eluate was centrifuged at 1000 rpm for 5 min, and the supernatant was discarded. The resulting cells were resuspended in 10-20 mL of cell culture to give a single cell suspension. The cells were stained with trypan blue and viable cells were counted.
3. Cell plating: the HCC1569, MDA-MB-453, or MDA-MB-231 single cell suspension was mixed well and adjusted to a viable cell density of 6 × 10⁴ cells/mL with cell culture. The cell suspension with the adjusted density was mixed well and added to a 96-well cell culture plate at 50 µL/well. The culture plate was incubated in an incubator for 18 h (37 °C, 5% CO₂).
4. Antibody-drug conjugate concentration: the antibody-drug conjugates were diluted 3-fold starting from 100 nM to 0.0152 nM for a total of 9 concentrations.
5. Sample adding: the prepared samples to be detected at different concentrations were added to the culture plate, and two duplicate wells were set for each sample. The culture plate was incubated in an incubator for 6 days (37 °C, 5% CO₂).
6. Development: the 96-well cell culture plate was taken out and left to stand for 30 min so as to be equilibrated to room temperature. The CTG reagent was added at 50 µL/well, and the plate was shaken on an orbital shaker for 2 min and incubated in the dark at room temperature for 10 min.
7. Plate reading: the 96-well cell culture plate was taken out, placed in a 2104 EnVision microplate reader, and measured for the chemiluminescence using the microplate reader.

### Data analysis

The inhibition rates (IRs) of the detected compounds were calculated according to the following formula: IR (%) = (1- (RLU compound - RLU blank control) / (RLU vehicle control - RLU blank control)) × 100%. The inhibition rates of compounds at different concentrations were calculated using Microsoft Excel and data were processed and analyzed using Graphpad Prism 9.

**Table 13. IC₅₀ values for the inhibition of the in vitro proliferation of HCC1569 and MDA-MB-453 cells by the antibody-drug conjugates of the present application**

| Antibody-drug conjugate | HCC1569 | MDA-MB-453 | MDA-MB-231 |
|---|---|---|---|
| | IC50 (nM) | IC50 (nM) | IC50 (nM) |
| Reference ADC-1 | 40.7 | 9.619 | - |
| ADC-1-X1 | 0.115 | 0.042 | - |
| ADC-1-X2 | 5.167 | 0.094 | - |

| | | | |
|---|---|---|---|
| Note: "-" indicates no proliferation inhibitory activity. | | | |

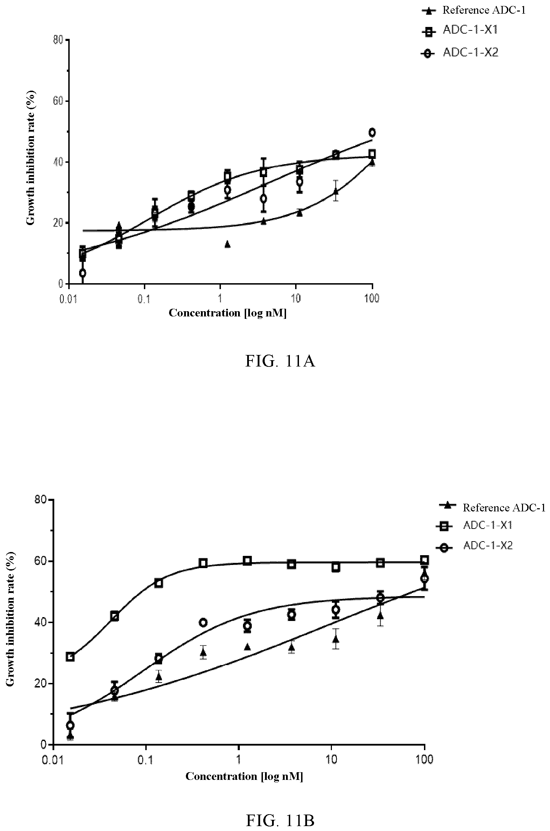

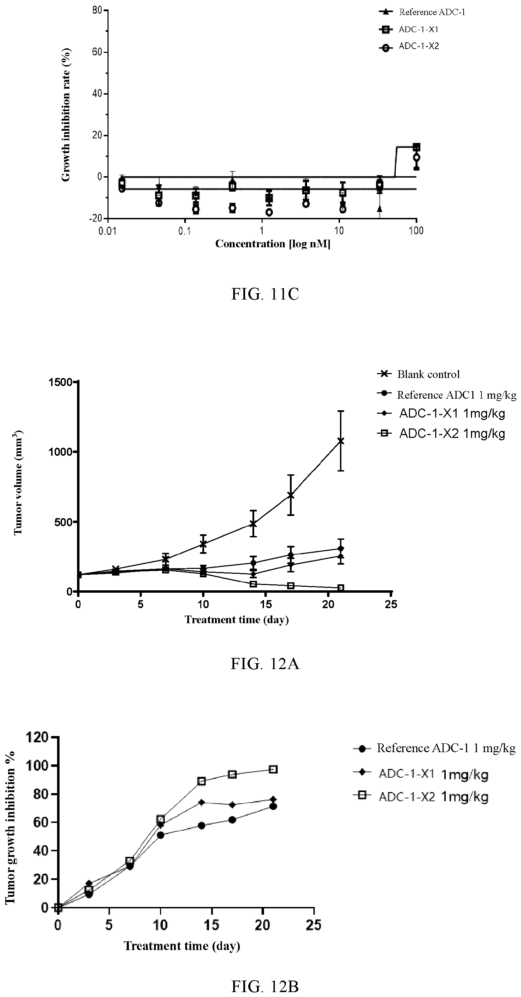

Conclusion: according to the results shown in Table 13 and FIG. 11 (11A/11B/11C), the antibody-drug conjugates of the present application have significant inhibitory activity on the proliferation of Her3-positive high expression cells HCC1569 and MDA-MB-453, and the inhibitory activities are stronger than that of the reference ADC-1, meanwhile, they have no inhibitory activity on the proliferation of MDA-MB-231 cells not expressing HER3, therefore, they have good selectivity. Example 5. Evaluation of Efficacy of Antibody-Drug Conjugate in HCC1569 Tumor-Bearing Mice Objective

To evaluate anti-tumor effect of the antibody-drug conjugates of the present application in a female NOD/SCID mouse model with subcutaneous xenograft of HCC1569 breast cancer cells.

### 1. Test compounds and materials

Blank control (negative control group): PBS
Reference ADC-1 (positive control group): 1 mg/kg
ADC-1-X1 (treatment group): 1 mg/kg
ADC-1-X2 (treatment group): 1 mg/kg

### 2. Preparation method: all samples were diluted with PBS.

### 3. Test animal

8- to 10-week-old NOD/SCID mice, purchased from Jiangsu GemPharmatech Co., Ltd.

### 4. Experiment process

The test mice were inoculated subcutaneously on the right anterior scapula with 2 × 10⁶ HCC1569 cells resuspended in a PBS/Matrigel (1:1) mixture (0.1 mL/mouse). The growth of the tumors was monitored. When the tumors grew to a mean volume of 100-150 mm³, the mice were randomly grouped for administration according to the tumor size and the mouse weight, 6 mice per group. The day of administration for each group was defined as day 0.

The administration was performed by tail vein injection 1 time per week, and the experiment ended 21 days after the administration. The tumor volume and weight were measured twice a week, and the data were recorded. At the end of the experiment, mice were euthanized and tumor growth inhibition TGI% was calculated as follows: (TGI = (1 - (Ti - T0) / (Vi - V0))). Ti: mean tumor volumes of the treatment groups and the positive control group on day i of administration; T0: mean tumor volumes of the treatment groups and the positive control group on day 0 of administration; Vi: mean tumor volume of the negative control group on day i of administration; V0: mean tumor volume of the negative control group on day 0 of administration.

### Data analysis

Data were analyzed using Excel 2016 statistical software, wherein the mean value was calculated as avg; the SD value was calculated as STDEV; and the P value of the difference between groups was calculated as TTEST.

The results of the experiment are shown in Table 14 and FIG. 12. At the end of the experiment, the blank control group had a mean tumor volume of 1078.32 mm³.

**Table 14. In vivo tumor-inhibiting effect of the antibody-drug conjugates of the present application in HCC1569 tumor-bearing mice**

| Antibody-drug conjugate | Mean tumor volume | Tumor growth inhibition % |
|---|---|---|
| Reference ADC-1 | 308.64 ±66.78mm³ | 71.38% |
| ADC-1-X1 | 254.63±57.6 mm³ | 76.39% |
| ADC-1-X2 | 25.84±14.38 mm³ | 97.60% |

Conclusion: the antibody-drug conjugates of the present application can significantly reduce the tumor volume. Moreover, the antibody-drug conjugates of the present application have a better in vivo tumor-inhibiting effect than that of the reference ADC-1.

### Example 6. Evaluation of Efficacy of Antibody-Drug Conjugate in Colo205 Tumor-Bearing Mice Objective

To evaluate the anti-tumor effect of the antibody-drug conjugates of the present application using BALB/c nude mice as test animals;
to evaluate the efficacy of ADC-III-28 and reference ADC-2 on Colo205 xenograft tumor-bearing nude mice after the intraperitoneal injection.

### 1. Test compounds and materials

Blank control (negative control group): PBS
Reference ADC-1 (positive control group): 1 mg/kg
ADC-1-X1 (treatment group): 1 mg/kg
ADC-1-X2 (treatment group): 1 mg/kg

### 2. Preparation method: all were diluted with PBS.

### 3. Test animal

8- to 10-week-old BALB/c-nude mice, purchased from Jiangsu GemPharmatech Co., Ltd.

### 4. Experiment process

Colo205 cells were subcutaneously inoculated into the right flank of the mice, 1 × 10' cells (100 µL/mouse). When the tumors grew to 80-120 mm³, the animals were randomly grouped, 5 animals per group. The day of administration for each group was defined as day 0. The administration was performed by tail vein injection 1 time every two weeks, and the experiment ended 22 days after the administration. The tumor volume and weight were measured twice a week, and the data were recorded. At the end of the experiment, mice were euthanized and tumor growth inhibition TGI% was calculated as follows: (TGI = (1 - (Ti - T0) / (Vi - V0))). Ti: mean tumor volumes of the treatment groups and the positive control group on day i of administration; T0: mean tumor volumes of the treatment groups and the positive control group on day 0 of administration; Vi: mean tumor volume of the negative control group on day i of administration; V0: mean tumor volume of the negative control group on day 0 of administration.

Data were analyzed using Excel 2016 statistical software, wherein the mean value was calculated as avg; the SD value was calculated as STDEV; and the P value of the difference between groups was calculated as TTEST.

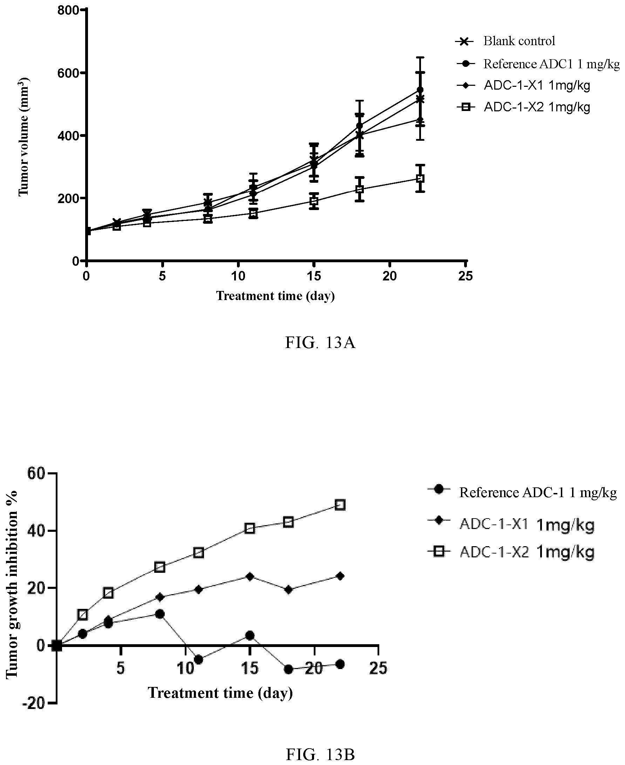

The results of the experiment are shown in Table 15 and FIG. 13. At the end of the experiment, the blank control group had a mean tumor volume of 515.92 mm³.

**Table 15. In vivo tumor-inhibiting effect of the antibody-drug conjugates of the present application in Colo205 tumor-bearing mice**

| Antibody-drug conjugate | Mean tumor volume | Tumor growth inhibition % |
|---|---|---|
| Reference ADC-1 | 545.75±252 mm³ | -6.38% |
| ADC-1-X1 | 385.11±111 mm³ | 24.33% |
| ADC-1-X2 | 263.20 ±105mm³ | 49.09% |

Conclusion: the antibody-drug conjugates of the present application can significantly reduce the tumor volume. Moreover, a tumor-inhibiting effect was observed in the antibody-drug conjugates of the present application at a lower administration concentration than that of the reference ADC-1. Example 7. Evaluation of Efficacy of Antibody-Drug Conjugate in NCI-H441 Lung Cancer Cell Tumor-Bearing MiceObjective

To evaluate the anti-tumor effect of the antibody-drug conjugates of the present application in NCI-H441 tumor-bearing mice using male NU/NU nude mice as test animals.
1. Test compounds and materials
   Blank control (negative control group): normal saline
   Reference ADC-1 (positive control group): 0.3 mg/kg or 1 mg/kg
   ADC-1-X2 (treatment group): 0.3 mg/kg or 1 mg/kg
2. Preparation method: all were diluted with normal saline.
3. Test animals: 6- to 8-week-old NU/NU nude mice, purchased from Vital River Laboratory Animal Technology Co., Ltd.
4. Experiment process

The test mice were inoculated subcutaneously on the right dorsum with 5 × 10⁶ NCI-H441 cells, and the cells were resuspended in 0.1 mL of PBS and Matrigel (1:1). The growth of the tumors was monitored periodically. When the tumors grew to a mean volume of 115.24 mm³, the mice were randomly grouped for administration according to the tumor size and the mouse weight, 6 animals per group. The day of administration for each group was defined as day 0. The administration was performed by tail vein injection for 1 time, and the experiment ended 28 days after the administration. The tumor volume and weight were measured twice a week, and the data were recorded. At the end of the experiment, mice were euthanized and tumor growth inhibition TGI% was calculated as follows: (TGI = (1 - (Ti - T0) / (Vi - V0))). Ti: mean tumor volumes of the treatment groups and the positive control group on day i of administration; T0: mean tumor volumes of the treatment groups and the positive control group on day 0 of administration; Vi: mean tumor volume of the negative control group on day i of administration; V0: mean tumor volume of the negative control group on day 0 of administration.

Data were analyzed using Excel 2016 statistical software, wherein the mean value was calculated as avg; the SD value was calculated as STDEV; and the P value of the difference between groups was calculated as TTEST.

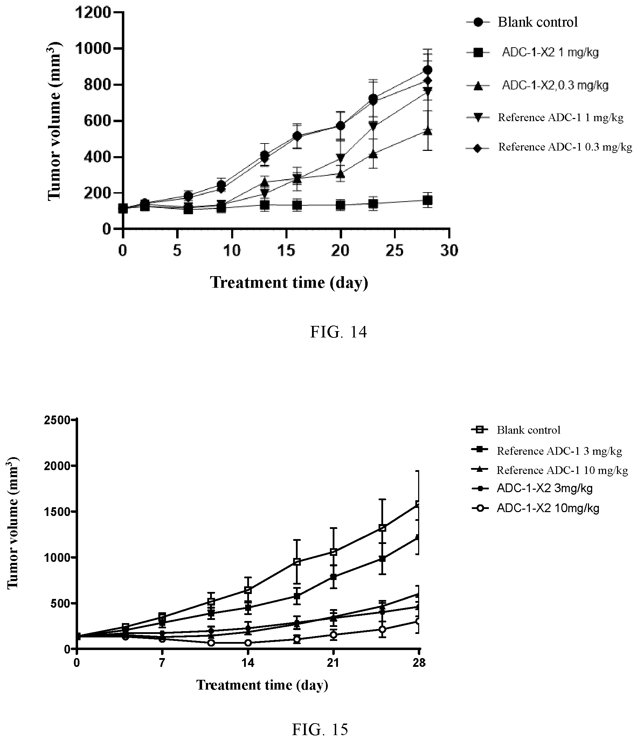

The results of the experiment are shown in Table 16 and FIG. 14. At the end of the experiment, the blank control group had a mean tumor volume of 1138.85 mm³.

**Table 16. In vivo tumor-inhibiting effect of the antibody-drug conjugates of the present application in NCI-H441 tumor-bearing mice**

| Group | Administration | Dose (mg/kg) | 28 days | | | |
|---|---|---|---|---|---|---|
| | | | Tumor volume (mean ± SEM) | T/C (%) | TGI (%) | p value (compared with blank control) |
| 1 | Blank control | - | 882.81 ± 114.09 | - | - | - |
| 2 | ADC-1-X2 | 1 | 162.00 ± 42.47 | 18.35 | 81.65 | 0.00787 |
| 3 | ADC-1-X2 | 0.3 | 546.52 ± 109.10 | 61.91 | 38.09 | 0.497 |
| 4 | Reference ADC-1 | 1 | 760.76 ± 208.92 | 86.17 | 13.83 | 0.987 |
| 5 | Reference ADC-1 | 0.3 | 823.01 ± 109.69 | 93.23 | 6.77 | 1 |

Conclusion: the antibody-drug conjugates of the present application can significantly reduce the tumor volume, and the antibody-drug conjugates of the present application have better tumor inhibition than that of the reference ADC-1 under the same concentration.

### Example 8. Evaluation of Efficacy of Antibody-Drug Conjugate in Human Lung Cancer LU1542 Subcutaneous Xenograft Tumor-Bearing Mice

### Objective

To evaluate the anti-tumor effect of the antibody-drug conjugates of the present application in human lung cancer LU1542 subcutaneous xenograft tumor-bearing mice using female BALB/c nude mice as test animals.
1. Test compounds and materials
   Blank control (negative control group): PBS
   Reference ADC-1 (positive control group): 3 mg/kg or 10 mg/kg
   ADC-1-X2 (treatment group): 3 mg/kg or 10 mg/kg
2. Preparation method: all were diluted with PBS.
3. Test animals: 6- to 8-week-old BALB/c nude mice, purchased from Beijing AniKeeper Biotech Co., Ltd.
4. Experiment process

Tumor tissues were collected from tumor-bearing mice of HuPrime^{®} lung cancer xenograft model LU1542, cut into tumor masses of a diameter of 2-3 mm and then subcutaneously inoculated into the right anterior scapula of the Balb/c nude mice. When the mean tumor volume of the tumor-bearing mice reached 136.8 mm³, the mice were randomly grouped, 6 animals per group. The day of administration for each group was defined as day 0. The administration was performed by tail vein injection for 1 time, and the experiment ended 28 days after the administration. The tumor volume and weight were measured twice a week, and the data were recorded. At the end of the experiment, mice were euthanized and tumor growth inhibition TGI% was calculated as follows: (TGI = (1 - (Ti - T0) / (Vi - V0))). Ti: mean tumor volumes of the treatment groups and the positive control group on day i of administration; T0: mean tumor volumes of the treatment groups and the positive control group on day 0 of administration; Vi: mean tumor volume of the negative control group on day i of administration; V0: mean tumor volume of the negative control group on day 0 of administration.

Data were analyzed using Excel 2016 statistical software, wherein the mean value was calculated as avg; the SD value was calculated as STDEV; and the P value of the difference between groups was calculated as TTEST.

The results of the experiment are shown in Table 17 and FIG. 15. At the end of the experiment, the blank control group had a mean tumor volume of 1580.09 mm³.

**Table 17. In vivo tumor-inhibiting effect of the antibody-drug conjugates of the present application in NCI-H441 tumor-bearing mice**

| Antibody-drug conjugate | Mean tumor volume | Tumor growth inhibition % |
|---|---|---|
| ADC-1-X2 3mg/kg | 461.39±106.26 mm³ | 77.50% |
| ADC-1-X2 10mg/kg | 302.78±129.69 mm³ | 88.49% |
| Reference ADC-1 3 mg/kg | 1219.23 ±187.13 mm³ | 24.98% |
| Reference ADC-1 10 mg/kg | 602.13± 898 mm³ | 67.75% |

Conclusion: the antibody-drug conjugates of the present application can significantly reduce the tumor volume. Moreover, a tumor-inhibiting effect was observed in the antibody-drug conjugates of the present application at a lower administration concentration than that of the reference ADC, and the antibody-drug conjugates of the present application have better tumor inhibition under the same concentration.

Sequence of the present application (SEQ ID NO.):

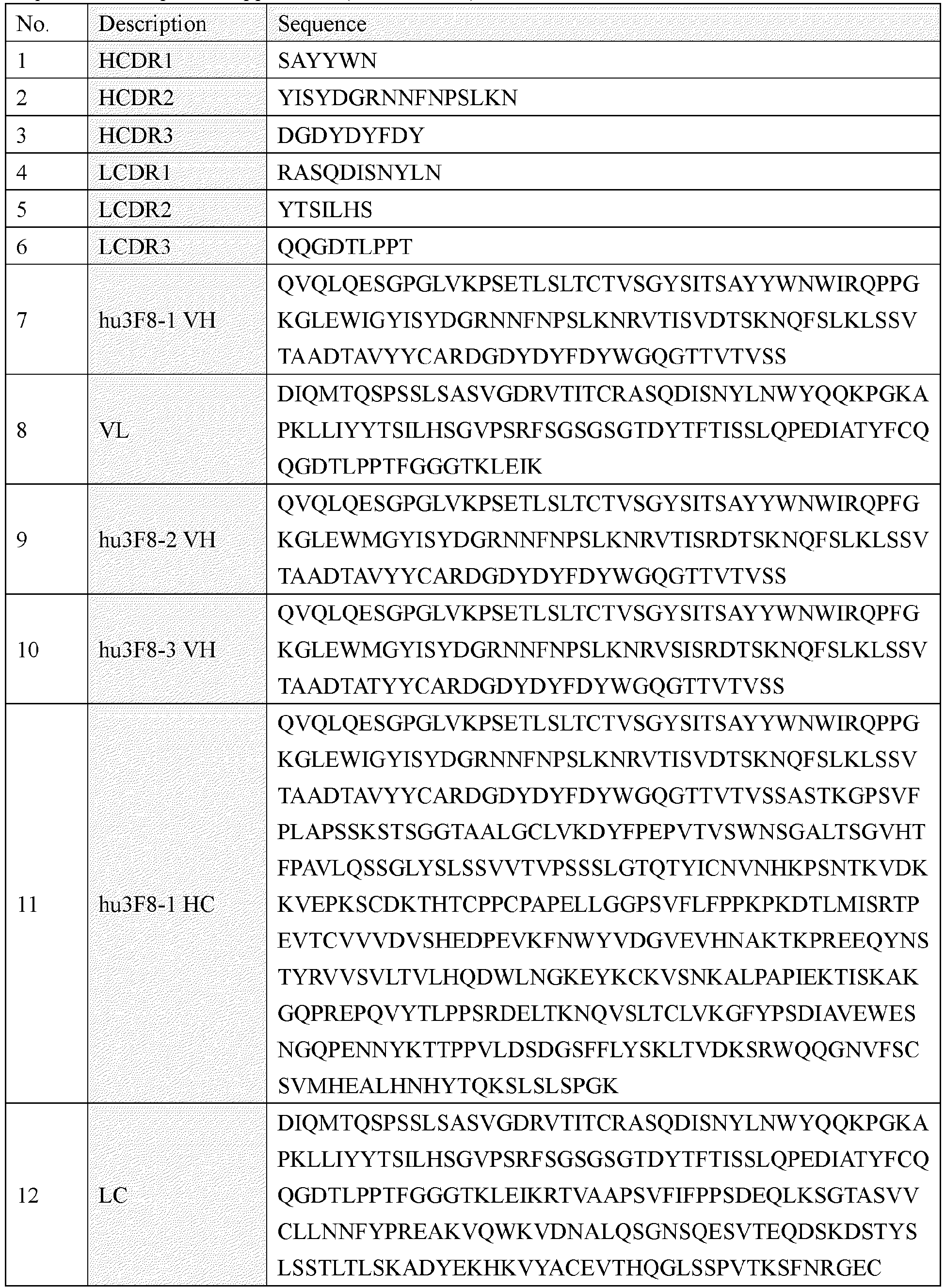

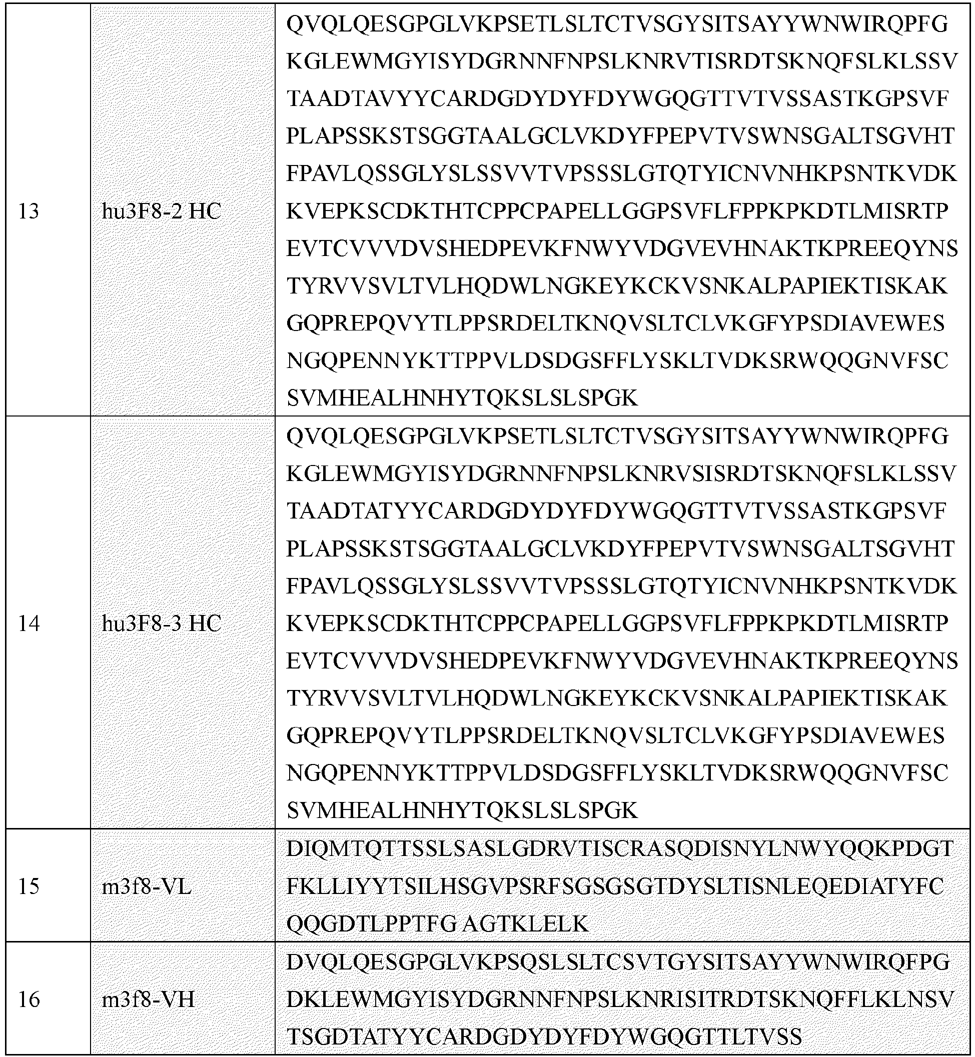

## Claims

1. An anti-Her3 antibody-drug conjugate, an isomer thereof, a pharmaceutically acceptable salt thereof, or a mixture thereof, wherein the anti-Her3 antibody-drug conjugate has a structure of formula (I):
Ab-(L-M-D)ₚ (I)
wherein,
L and M are linker units;
D is a cytotoxic drug;
p represents an average connection number, and p is selected from integers or decimals from 1 to 10, preferably integers or decimals from 3 to 8;
Ab is an anti-Her3 antibody or an antigen-binding fragment thereof, which comprises a heavy chain variable region and a light chain variable region, wherein the heavy chain variable region comprises HCDR1, HCDR2, and HCDR3 of the amino acid sequences set forth in SEQ ID NO: 1, SEQ ID NO: 2, and SEQ ID NO: 3, respectively, and the light chain variable region comprises LCDR1, LCDR2, and LCDR3 of the amino acid sequences set forth in SEQ ID NO: 4, SEQ ID NO: 5, and SEQ ID NO: 6, respectively.

2. The anti-Her3 antibody-drug conjugate, the isomer thereof, the pharmaceutically acceptable salt thereof, or the mixture thereof according to claim 1, wherein the anti-Her3 antibody-drug conjugate has a structure of formula (I-1): 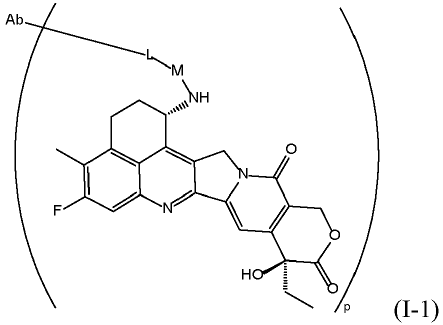 wherein,
L and M are linker units;
p represents an average connection number, and p is selected from integers or decimals from 1 to 10, preferably integers or decimals from 3 to 8;
Ab is an anti-Her3 antibody or an antigen-binding fragment thereof, which comprises a heavy chain variable region and a light chain variable region, wherein the heavy chain variable region comprises HCDR1, HCDR2, and HCDR3 of the amino acid sequences set forth in SEQ ID NO: 1, SEQ ID NO: 2, and SEQ ID NO: 3, respectively, and the light chain variable region comprises LCDR1, LCDR2, and LCDR3 of the amino acid sequences set forth in SEQ ID NO: 4, SEQ ID NO: 5, and SEQ ID NO: 6, respectively.

3. The anti-Her3 antibody-drug conjugate, the isomer thereof, the pharmaceutically acceptable salt thereof, or the mixture thereof according to claim 1 or 2, wherein the anti-Her3 antibody or the antigen-binding fragment thereof comprises:
(I) a heavy chain variable region of the amino acid sequence set forth in SEQ ID NO: 7 or having at least 95%, 96%, 97%, 98%, or 99% identity thereto, and a light chain variable region of the amino acid sequence set forth in SEQ ID NO: 8 or having at least 95%, 96%, 97%, 98%, or 99% identity thereto; or
(II) a heavy chain variable region of the amino acid sequence set forth in SEQ ID NO: 9 or having at least 95%, 96%, 97%, 98%, or 99% identity thereto, and a light chain variable region of the amino acid sequence set forth in SEQ ID NO: 8 or having at least 95%, 96%, 97%, 98%, or 99% identity thereto; or
(III) a heavy chain variable region of the amino acid sequence set forth in SEQ ID NO: 10 or having at least 95%, 96%, 97%, 98%, or 99% identity thereto, and a light chain variable region of the amino acid sequence set forth in SEQ ID NO: 8 or having at least 95%, 96%, 97%, 98%, or 99% identity thereto.

4. The anti-Her3 antibody-drug conjugate, the isomer thereof, the pharmaceutically acceptable salt thereof, or the mixture thereof according to claim 1 or 2, wherein the anti-Her3 antibody or the antigen-binding fragment thereof is a murine antibody or a fragment thereof, a chimeric antibody or a fragment thereof, or a humanized antibody or a fragment thereof.

5. The anti-Her3 antibody-drug conjugate, the isomer thereof, the pharmaceutically acceptable salt thereof, or the mixture thereof according to claim 1 or 2, wherein the anti-Her3 antibody or the antigen-binding fragment thereof is selected from a Fab, a Fab', a Fab'-SH, an Fv, an scFv, a F(ab')₂, an sdAb, a bispecific antibody, or a linear antibody.

6. The anti-Her3 antibody-drug conjugate, the isomer thereof, the pharmaceutically acceptable salt thereof, or the mixture thereof according to claim 1 or 2, wherein the antibody is of the type of IgG1, IgG2, IgG3, or IgG4.

7. The anti-Her3 antibody-drug conjugate, the isomer thereof, the pharmaceutically acceptable salt thereof, or the mixture thereof according to any one of claims 1-6, wherein the anti-Her3 antibody or the antigen-binding fragment thereof comprises:
(1) a heavy chain of the amino acid sequence set forth in SEQ ID NO: 11 or having at least 95%, 96%, 97%, 98%, or 99% identity thereto, and a light chain of the amino acid sequence set forth in SEQ ID NO: 12 or having at least 95%, 96%, 97%, 98%, or 99% identity thereto; or
(2) a heavy chain of the amino acid sequence set forth in SEQ ID NO: 13 or having at least 95%, 96%, 97%, 98%, or 99% identity thereto, and a light chain of the amino acid sequence set forth in SEQ ID NO: 12 or having at least 95%, 96%, 97%, 98%, or 99% identity thereto; or
(3) a heavy chain of the amino acid sequence set forth in SEQ ID NO: 14 or having at least 95%, 96%, 97%, 98%, or 99% identity thereto, and a light chain of the amino acid sequence set forth in SEQ ID NO: 12 or having at least 95%, 96%, 97%, 98%, or 99% identity thereto.

8. The anti-Her3 antibody-drug conjugate, the isomer thereof, the pharmaceutically acceptable salt thereof, or the mixture thereof according to any one of claims 1-7, wherein
M is -L²-L³-X-L¹-;
L² is -O- or -S-;
L³ is -(C(R^{1a})(R^{1b}))ₘ- , and m is selected from 0, 1, 2, or 3, wherein when L³ comprises a methylene unit, 0 or 1 methylene unit of L³ may be replaced by -C(O)- or -C(S)-;
L¹ is -(C(R^{2a})(R^{2b}))ₙ-, and n is selected from 1, 2, or 3, wherein when L¹ may comprise a methylene unit, 0 or 1 methylene unit of L¹ may be replaced by -C(O)- or -C(S)-;
X is selected from 3- 6 membered saturated carbocyclyl, 3- 6 membered saturated heterocyclyl, or a single bond, wherein the 3- 6 membered saturated carbocyclyl and the 3- 6 membered saturated heterocyclyl are optionally substituted with 0, 1, 2, or 3 R^{3a},
wherein each R^{1a}, each R^{1b}, each R^{2a}, each R^{2b}, and each R^{3a} may independently be hydrogen, halogen, or a C₁₋₆ aliphatic group which may be optionally substituted with R,
wherein each R may independently be hydrogen or halogen.

9. The anti-Her3 antibody-drug conjugate, the isomer thereof, the pharmaceutically acceptable salt thereof, or the mixture thereof according to claim 8, wherein L³ is selected from a single bond, - C(R^{1a})(R^{1b})-, or -C(R^{1a})(R^{b})C(R^{1a})(R^{1b})-,
wherein each R^{1a} and each R^{1b} may independently be hydrogen, halogen, or a C₁₋₆ aliphatic group which may be optionally substituted with R,
wherein each R may independently be hydrogen or halogen.

10. The anti-Her3 antibody-drug conjugate, the isomer thereof, the pharmaceutically acceptable salt thereof, or the mixture thereof according to claim 9, wherein L³ is selected from a single bond, -CH₂-, -CH(CH₃)-, -C(CH₃)₂-, -CH₂CH₂-, -CH(CH₃)CH₂-, or -C(CH₃)₂CH₂-.

11. The anti-Her3 antibody-drug conjugate, the isomer thereof, the pharmaceutically acceptable salt thereof, or the mixture thereof according to claim 8, wherein L¹ is selected from -C(R^{2a})(R^{2b})-, - C(R^{2a})(R^{2b})C(O)-, or -C(O)-,
wherein each R^{2a} and each R^{2b} may independently be hydrogen, halogen, or a C₁₋₆ aliphatic group which may be optionally substituted with R,
wherein each R may independently be hydrogen or halogen.

12. The anti-Her3 antibody-drug conjugate, the isomer thereof, the pharmaceutically acceptable salt thereof, or the mixture thereof according to claim 11, wherein L¹ is selected from -CH₂-, -CH₂C(O)-, -CH(CH₃)C(O)-, or -C(O)-.

13. The anti-Her3 antibody-drug conjugate, the isomer thereof, the pharmaceutically acceptable salt thereof, or the mixture thereof according to claim 8, wherein X is 3- 6 membered saturated carbocyclyl optionally substituted with 0, 1, 2, or 3 R^{3a} or a single bond,
wherein each R^{3a} may independently be hydrogen, halogen, or a C₁₋₆ aliphatic group which may be optionally substituted with R,
wherein each R may independently be hydrogen or halogen.

14. The anti-Her3 antibody-drug conjugate, the isomer thereof, the pharmaceutically acceptable salt thereof, or the mixture thereof according to claim 13, wherein X is 3- 6 membered saturated carbocyclyl or a single bond.

15. The anti-Her3 antibody-drug conjugate, the isomer thereof, the pharmaceutically acceptable salt thereof, or the mixture thereof according to any one of claims 1-14, wherein -M- is selected from: 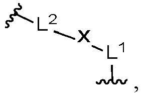 wherein
L² is -O- or -S-;
X is selected from 3- 6 membered saturated carbocyclyl optionally substituted with 0, 1, 2, or 3 R^{3a}; L¹ is selected from -C(R^{2a})(R^{2b})-, -C(R^{2a})(R^{2b})C(O)-, or -C(O)-,
wherein each R^{2a}, each R^{2b}, or each R^{3a} may independently be hydrogen, halogen, or a C₁₋₆ aliphatic group which may be optionally substituted with R,
wherein each R may independently be hydrogen or halogen.

16. The anti-Her3 antibody-drug conjugate, the isomer thereof, the pharmaceutically acceptable salt thereof, or the mixture thereof according to claim 15, wherein -M- is selected from: 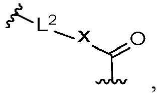 wherein
L² is -O- or -S-;
X is selected from 3- 6 membered saturated carbocyclyl.

17. The anti-Her3 antibody-drug conjugate, the isomer thereof, the pharmaceutically acceptable salt thereof, or the mixture thereof according to any one of claims 1-14, wherein -M- is selected from: 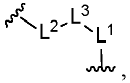 wherein
L² is -O- or -S-;
L³ is selected from -C(R^{1a})(R^{1b})- or -C(R^{1a})(R^{1b})C(R^{1a})(R^{1b})-;
L¹ is selected from -C(R^{2a})(R^{2b})-, -C(R^{2a})(R^{2b})C(O)-, or -C(O)-,
wherein each R^{1a}, each R^{1b}, each R^{2a}, or each ^{2b} may independently be hydrogen, halogen, or a C₁₋₆ aliphatic group which may be optionally substituted with R,
wherein each R may independently be hydrogen or halogen.

18. The anti-Her3 antibody-drug conjugate, the isomer thereof, the pharmaceutically acceptable salt thereof, or the mixture thereof according to claim 17, wherein -M- is selected from: 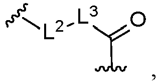 wherein
L² is -O- or -S-;
L³ is selected from -CH₂-, -CH(CH₃), -C(CH₃)₂-, -CH₂CH₂-, -CH(CH₃)CH₂-, or -C(CH₃)₂CH₂-.

19. The anti-Her3 antibody-drug conjugate, the isomer thereof, the pharmaceutically acceptable salt thereof, or the mixture thereof according to any one of claims 1-18, wherein -M- is selected from:

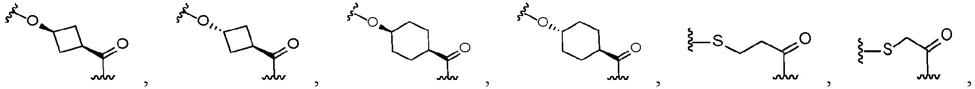

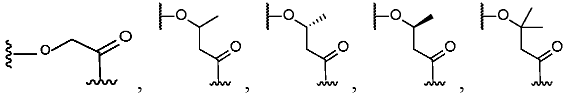

20. The anti-Her3 antibody-drug conjugate, the isomer thereof, the pharmaceutically acceptable salt thereof, or the mixture thereof according to any one of claims 1-19, wherein L is -Lₐ-L_{b}-L_{c}-, -La- is 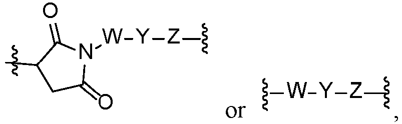
wherein W is -(C(R^{wa})(R^{wb}))_{wn}-, Y is -(OCH₂CH₂)_{yn}-O_{yp}-, and Z is -(C(R^{za})(R^{zb}))_{zn},
wherein wn is 1, 2, 3 or 6, and
1 methylene unit of W is each independently replaced by -Cyr-, -N(R^{wx})C(O)-, -C(O)N(R^{wx})- or - C(O)-;
wherein yn is 0, 4 or 8, and yp is 0 or 1;
wherein zn is 1, 2 or 3, and
1 methylene unit of Z is each independently replaced by -Cyr-, -N(R^{zx})C(O)-, -C(O)N(R^{xz})- or -C(O)-;
-Cyr- is 3- 10 membered saturated carbocyclylene, wherein -Cyr- is unsubstituted or independently substituted with 1 to 3 substituent R^{cx};
wherein each R^{wa}, each R^{wb}, each R^{za}, each R^{zb}, each R^{wx}, each R^{zx} and each R^{cx} are independently hydrogen, halogen, -OR^{r}, or a C₁₋₆ aliphatic group optionally substituted with R^{r},
wherein each R^{r} is independently hydrogen, halogen or a C₁₋₆ aliphatic group;
-L_{b}- is selected from the group consisting of: 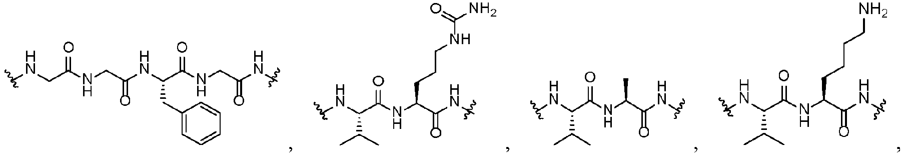 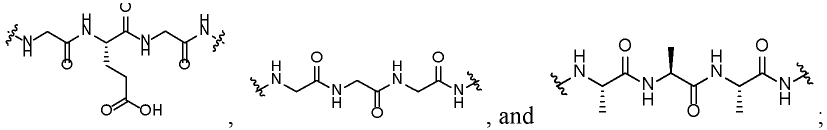
-L_{c}- is 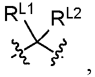 wherein R^{L1} and R^{L2} are each independently selected from the group consisting of: hydrogen, halogen, -OH and a C₁₋₆ aliphatic group.

21. The anti-Her3 antibody-drug conjugate, the isomer thereof, the pharmaceutically acceptable salt thereof, or the mixture thereof according to claim 20, wherein -Lₐ- is 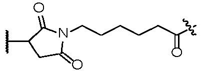 or 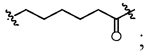 -Lb- is 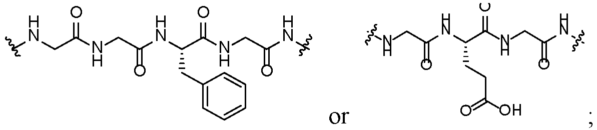 -L_{c}- is

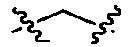

22. The anti-Her3 antibody-drug conjugate, the isomer thereof, the pharmaceutically acceptable salt thereof, or the mixture thereof according to any one of claims 1-21, wherein L is

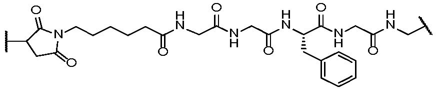

23. The anti-Her3 antibody-drug conjugate, the isomer thereof, the pharmaceutically acceptable salt thereof, or the mixture thereof according to any one of claims 1-21, wherein L is

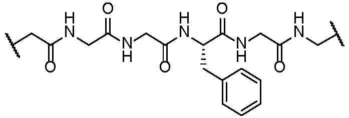

24. The anti-Her3 antibody-drug conjugate, the isomer thereof, the pharmaceutically acceptable salt thereof, or the mixture thereof according to any one of claims 1-23, wherein the anti-Her3 antibody-drug conjugate has a structure of formula (II-1) or (II-2): 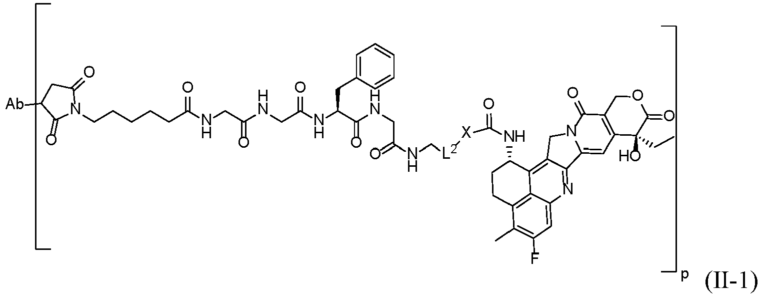 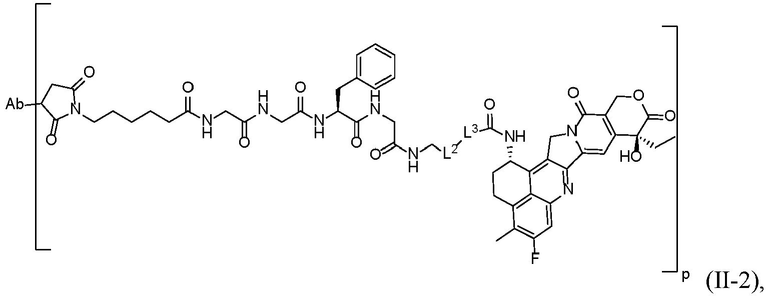 wherein,
p is as described in claim 1;
Ab is as described in any one of claims 1-7;
L² is -O- or -S-;
X is selected from 3- 6 membered saturated carbocyclyl optionally substituted with 0, 1, 2, or 3 R^{3a}, L³ is selected from -C(R^{1a})(R^{1b})- or -C(R^{1a})(R^{1b})C(R^{1a})(R^{1b})-,
wherein each R^{1a}, each R^{1b}, or each R^{3a} may independently be hydrogen, halogen, or a C₁₋₆ aliphatic group which may be optionally substituted with R,
wherein each R may independently be hydrogen or halogen.

25. The anti-Her3 antibody-drug conjugate, the isomer thereof, the pharmaceutically acceptable salt thereof, or the mixture thereof according to any one of claims 1-24, wherein the anti-Her3 antibody-drug conjugate is selected from the following structural formulas: 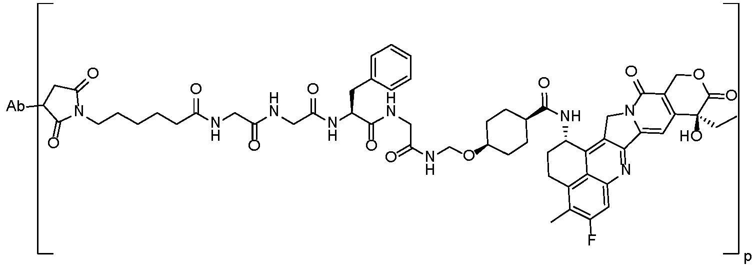 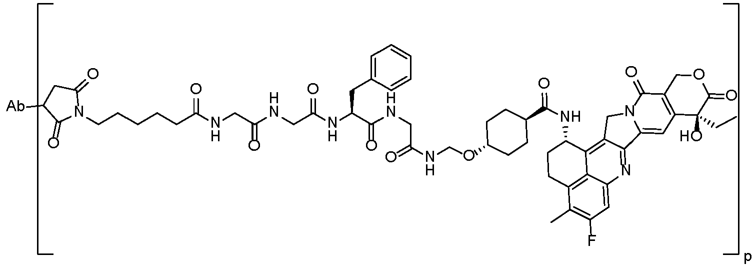 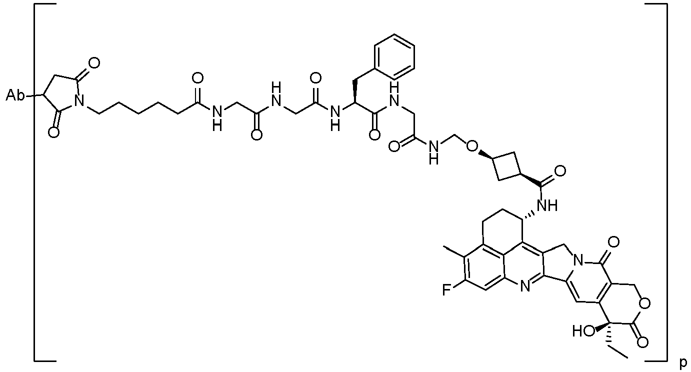 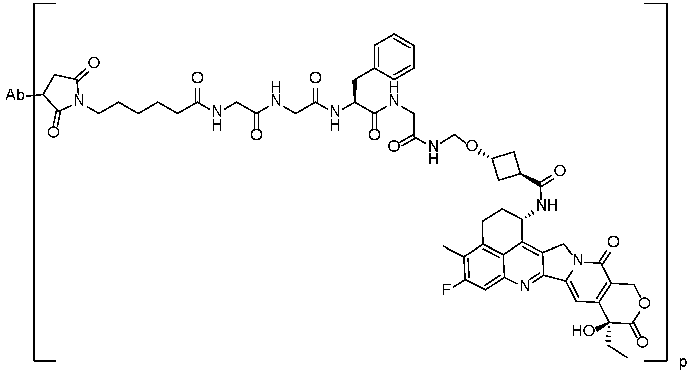 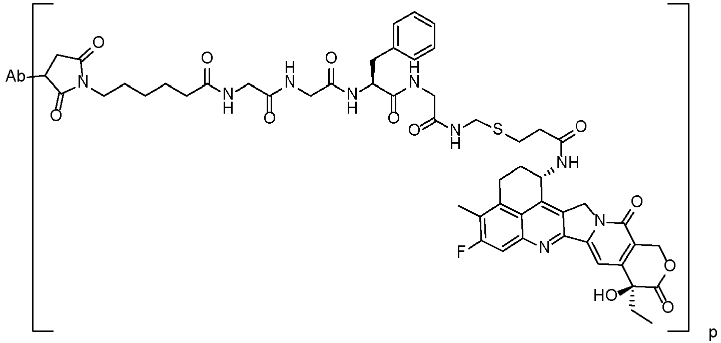 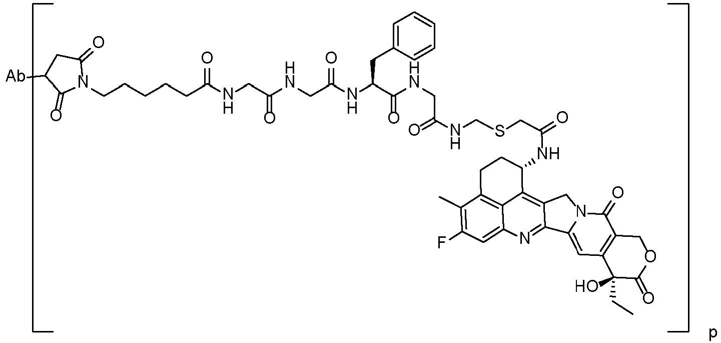 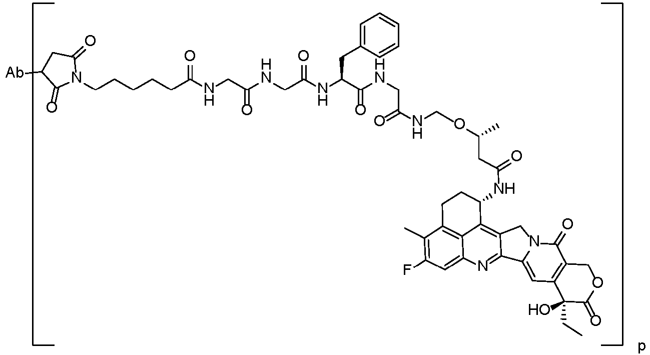 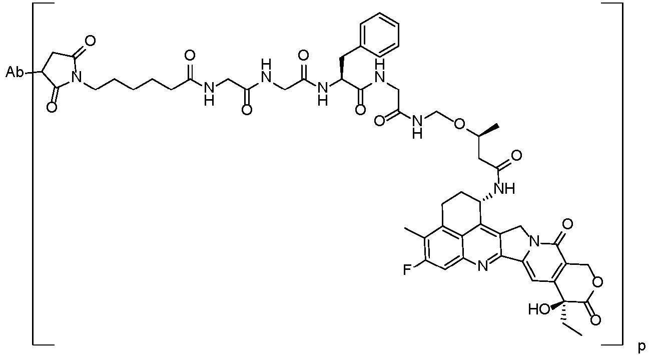 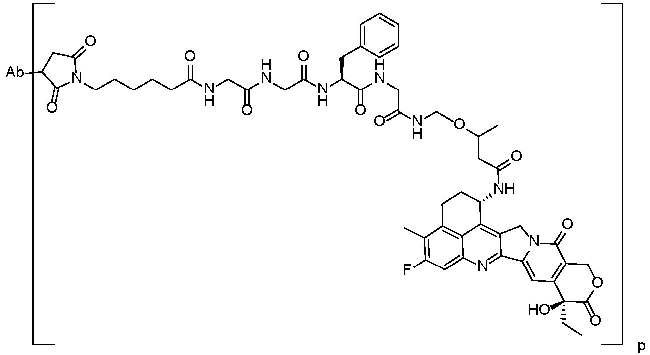 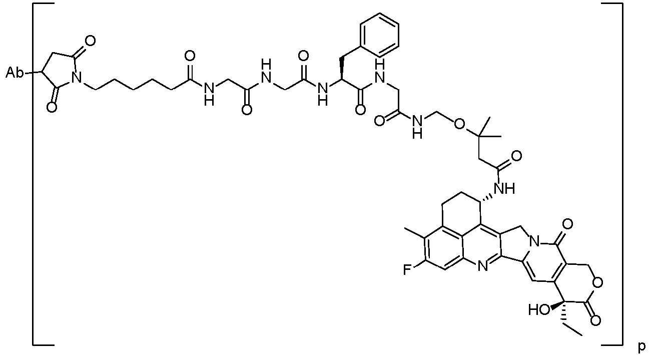 wherein,
p is as described in claim 1;
Ab is as described in any one of claims 1-7.

26. An anti-Her3 antibody-drug conjugate, an isomer thereof, a pharmaceutically acceptable salt thereof, or a mixture thereof, wherein the anti-Her3 antibody-drug conjugate is selected from: 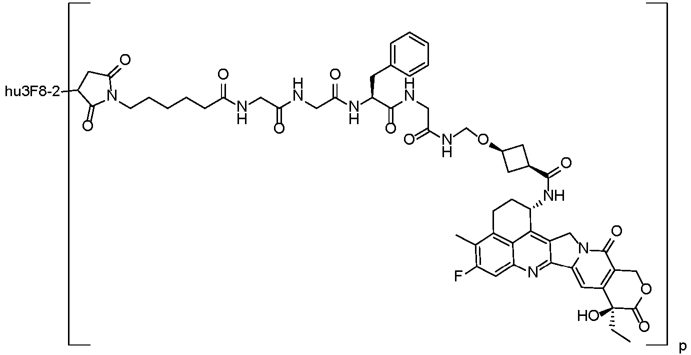 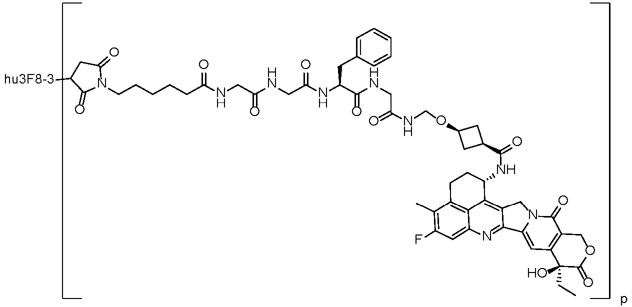 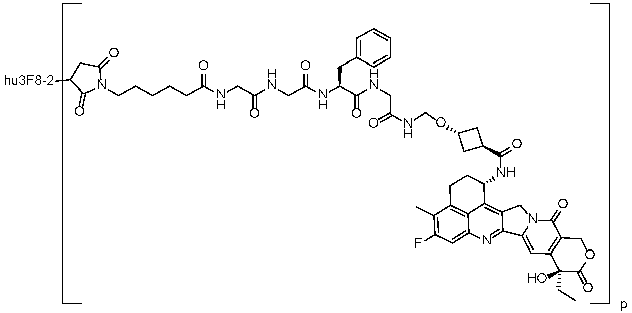 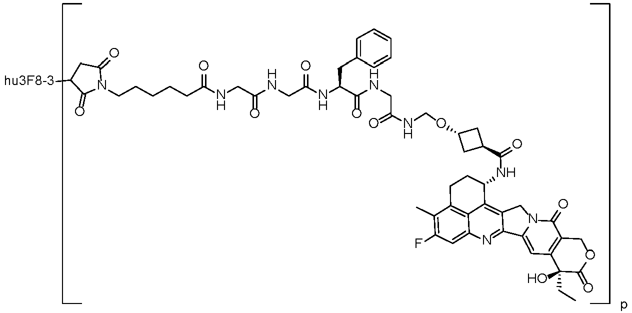 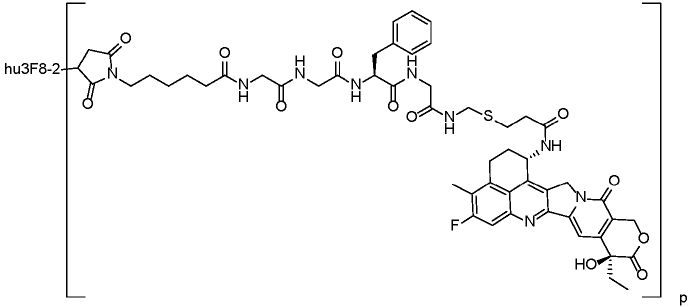 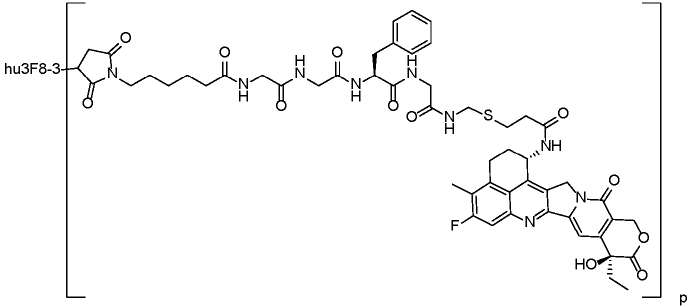 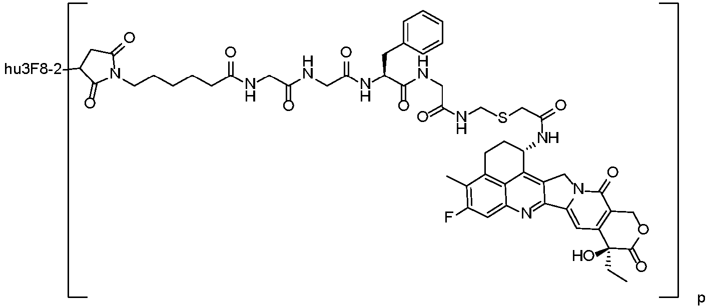 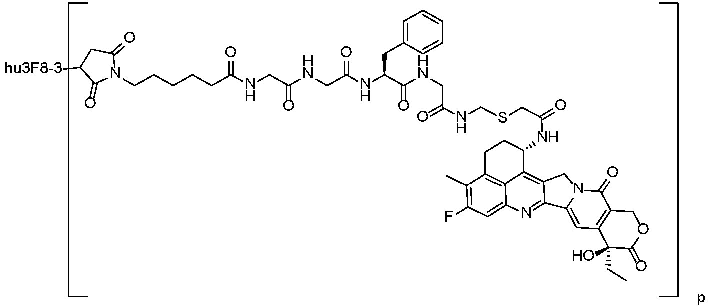 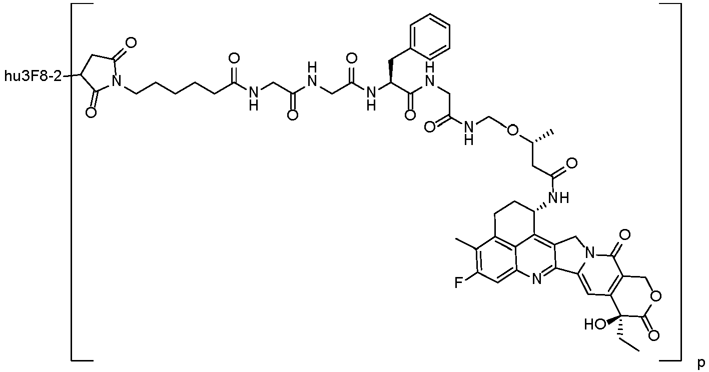 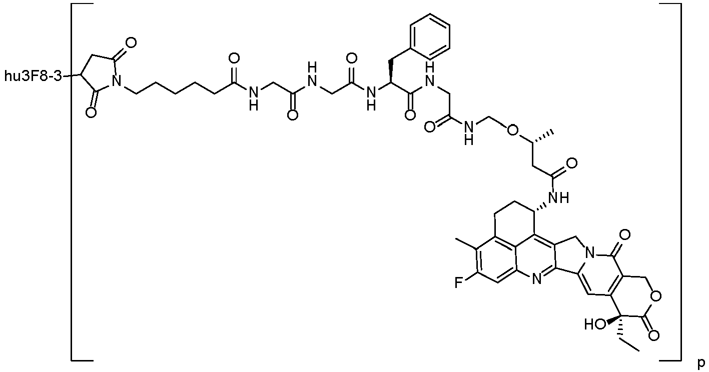 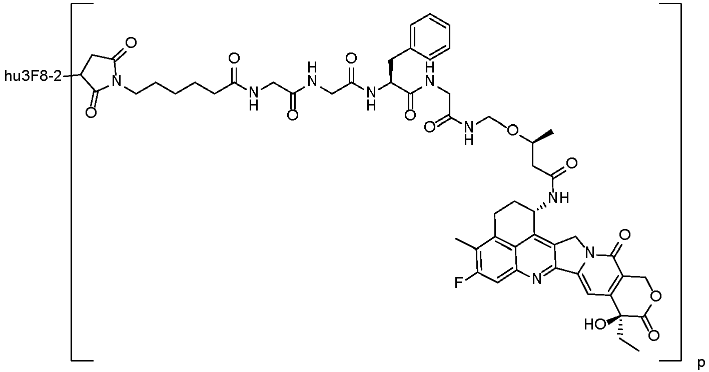 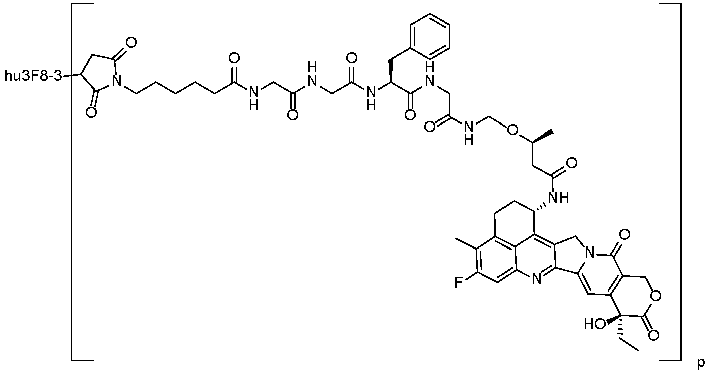 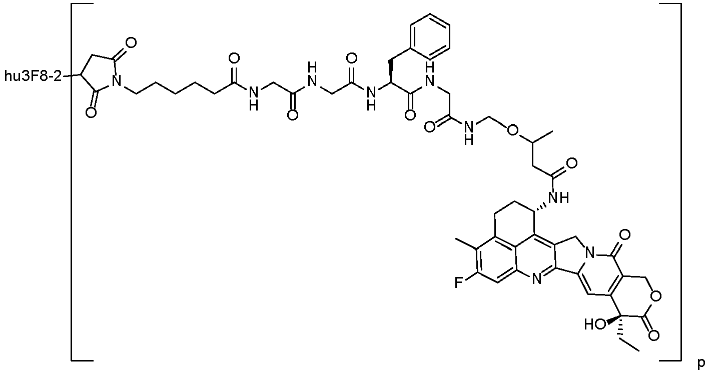 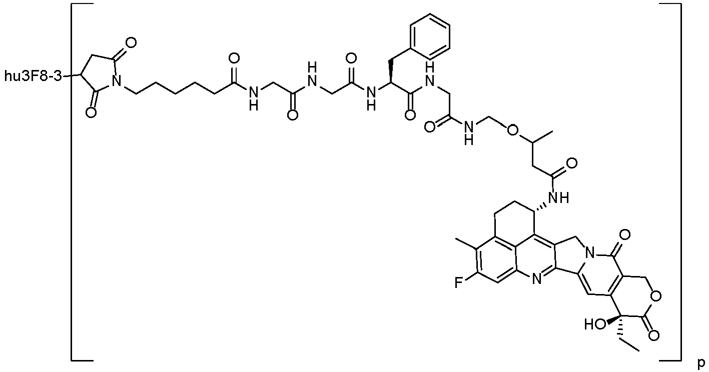 wherein,
p represents an average connection number, and p is selected from integers or decimals from 1 to 10, preferably integers or decimals from 3 to 8.

27. A pharmaceutical composition comprising the anti-Her3 antibody-drug conjugate, the isomer thereof, the pharmaceutically acceptable salt thereof, or the mixture thereof according to any one of claims 1-26, and a pharmaceutically acceptable carrier or excipient.

28. Use of the anti-Her3 antibody-drug conjugate, the isomer thereof, the pharmaceutically acceptable salt thereof, or the mixture thereof according to any one of claims 1-26 or the pharmaceutical composition according to claim 27 in preparing a medicament for treating and/or preventing a Her3-mediated disease or condition, wherein preferably, the disease or condition is cancer; more preferably, the cancer is selected from lung cancer, kidney cancer, urinary tract carcinoma, colorectal cancer, prostate cancer, glioblastoma multiforme, ovarian cancer, pancreatic cancer, breast cancer, melanoma, liver cancer, bladder cancer, gastric cancer, and esophageal cancer.

29. A method for treating and/or preventing a Her3-mediated disease or condition, comprising administering to a subject in need the anti-Her3 antibody-drug conjugate, the isomer thereof, the pharmaceutically acceptable salt thereof, or the mixture thereof according to any one of claims 1-26 or the pharmaceutical composition according to claim 27, wherein preferably, the disease or condition is cancer; more preferably, the cancer is selected from lung cancer, kidney cancer, urinary tract carcinoma, colorectal cancer, prostate cancer, glioblastoma multiforme, ovarian cancer, pancreatic cancer, breast cancer, melanoma, liver cancer, bladder cancer, gastric cancer, and esophageal cancer.
